(19) Europäisches Patentamt
European Patent Office
Office européen des brevets

(11) EP 2 947 064 B1

(12) **EUROPÄISCHE PATENTSCHRIFT**

(45) Veröffentlichungstag und Bekanntmachung des Hinweises auf die Patenterteilung:
**17.01.2018 Patentblatt 2018/03**

(51) Int Cl.:
***C07C 29/16** (2006.01)* ***C07C 2/24** (2006.01)*
***C07C 45/50** (2006.01)*

(21) Anmeldenummer: **15168100.4**

(22) Anmeldetag: **19.05.2015**

(54) **HERSTELLUNG QUALITATIV HOCHWERTIGER OXO-ALKOHOLE AUS UNSTETEN ROHSTOFFQUELLEN**

PRODUCTION OF HIGH QUALITY OXO ALCOHOLS FROM VOLATILE RAW MATERIAL SOURCES

PRODUCTION D'OXO-ALCOOLS QUALITATIFS DE GRANDE VALEUR À PARTIR DE SOURCES DE MATIÈRES PREMIÈRES DISCONTINUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.05.2014 DE 102014209536**

(43) Veröffentlichungstag der Anmeldung:
**25.11.2015 Patentblatt 2015/48**

(73) Patentinhaber: **Evonik Degussa GmbH**
**45128 Essen (DE)**

(72) Erfinder:
- **Geilen, Frank**
  **45721 Haltern am See (DE)**
- **Dyballa, Katrin Marie**
  **45657 Recklinghausen (DE)**
- **Hess, Dieter**
  **45770 Marl (DE)**
- **Peitz, Stephan**
  **45739 Oer-Erkenschwick (DE)**
- **Franke, Robert**
  **45772 Marl (DE)**
- **Fridag, Dirk**
  **45721 Haltern am See (DE)**
- **Maschmeyer, Dietrich**
  **45657 Recklinghausen (DE)**
- **Reeker, Helene**
  **44227 Dortmund (DE)**
- **Stochniol, Guido**
  **45721 Haltern am See (DE)**

(56) Entgegenhaltungen:
**EP-A1- 0 850 905** **WO-A1-99/25668**
**WO-A1-03/029180**

EP 2 947 064 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Verfahren zur kontinuierlichen Herstellung eines Alkoholgemisches, bei welchem ein Olefine enthaltendes Einsatzgemisch, dessen Zusammensetzung sich über die Zeit verändert, unter Erhalt eines Oligomerisats einer Oligomerisierung unterworfen und zumindest ein Teil der im Oligomerisat enthaltenden Olefinoligomere in einer Hydroformylierung in Gegenwart eines homogenen Katalysatorsystems mit Kohlenmonoxid und Wasserstoff in Aldehyde oxiert werden, von denen zumindest ein Teil durch anschließende Hydrierung in das Alkoholgemisch umgesetzt werden.

Die Hydroformylierung - auch Oxo-Reaktion genannt - ermöglicht es, Olefine (Alkene) mit Synthesegas (Gemisch aus Kohlenmonoxid und Wasserstoff) in Aldehyde umzusetzen. Die erhaltenen Aldehyde weisen dann entsprechend ein Kohlenstoff-Atom mehr auf als die eingesetzten Olefine. Durch anschließende Hydrierung der Aldehyde entstehen Alkohole, die aufgrund ihrer Genese auch "Oxo-Alkohole" genannt werden.

Grundsätzlich sind alle Olefine der Hydroformylierung zugänglich, in der Praxis werden jedoch meist solche Olefine als Substrat in der Hydroformylierung eingesetzt, die zwei bis 20 Kohlenstoffatome aufweisen. Da Oxo-Alkohole vielfältig eingesetzt werden können - etwa für die Herstellung von Weichmachern für PVC, als Detergentien in Waschmitteln und als Riechstoffe - wird die Hydroformylierung großindustriell praktiziert.

Eine gute Übersicht über den allgemeinen Stand der Hydroformylierung von Olefinen findet sich in

B. CORNILS, W. A. HERRMANN, "Applied Homogeneous Catalysis with Organometallic Compounds", Vol. 1 & 2, VCH, Weinheim, New York, 1996

und in

R. Franke, D. Selent, A. Börner, "Applied Hydroformylation", Chem. Rev., 2012 (112), S.5675-5732, DOI:10.1021/cr3001803.

Ein Beispiel für einen weltweit stark nachgefragten Oxo-Alkohol ist Isononanol, kurz INA. Isononanol ist ein Gemisch aus isomeren Nonylalkoholen wie zum Beispiel n-Nonanol und einfach und/oder mehrfach verzweigten Nonanolen wie insbesondere Methyloctanol. INA trägt die CAS-Nr. 27458-94-2 und wird im Wesentlichen in der Weichmacherherstellung eingesetzt.

Den $C_9$-Oxo-Alkohol INA erhält man durch Hydroformylierung von $C_8$-Olefinen wie zum Beispiel 1-Octen zu den entsprechenden $C_9$-Aldehyden und deren anschließender Hydrierung.

In der Patentliteratur finden sich detaillierte Verfahrensbeschreibungen zur Herstellung von INA: So offenbaren DE102008007080A1 und EP2220017B1 Co-basierte Verfahren zur Herstellung von INA. Aus EP1674441 B1 ist ein zweistufiges INA-Verfahren bekannt, bei dem auf eine Cokatalysierte Hydroformylierung eine Rh-katalysierte Oxo-Reaktion folgt.

Wichtige Kriterien zur Unterscheidung von technischen Hydroformylierungsverfahren sind neben dem eingesetzten Substrat das Katalysatorsystem, die Phasenaufteilung im Reaktor und die Technik zum Austrag der Reaktionsprodukte aus dem Reaktor. Ein weiterer technisch relevanter Aspekt ist die Anzahl der durchgeführten Reaktionsstufen.

Industriell kommen entweder Cobalt- oder Rhodium-basierte Katalysatorsysteme zum Einsatz, wobei letztere mit Organophosphor-Liganden wie Phosphin-, Phosphit-, Phosphoramidit- oder Phosphonit-Liganden mit jeweils dreiwertigem Phosphor komplexiert werden. Diese Katalysatorsysteme liegen homogen gelöst in der Reaktionsmischung vor.

Die Art des Katalysatorsystems und die optimalen Reaktionsbedingungen für die Hydroformylierung sind von der Reaktivität des eingesetzten Olefins abhängig. Die unterschiedliche Reaktivität von isomeren $C_8$-Olefinen ist beschrieben in:

B. L. Haymore, A. van Hasselt, R. Beck, Annals of the New York Acad. Sci., 415, 1983, S.159-175

Die Produkte der Hydroformylierung sind durch die Struktur der Einsatzolefine, das Katalysatorsystem und die Reaktionsbedingungen bestimmt. Sind als Folgeprodukte der Oxoaldehyde Alkohole für die Herstellung von Detergentien und Weichmachern angestrebt, so sollen durch die Oxo-Reaktion möglichst lineare Aldehyde hergestellt werden. Die hieraus synthetisierten Produkte weisen besonders vorteilhafte Eigenschaften auf, z.B. niedrige Viskositäten der daraus resultierenden Weichmacher.

Die Struktur der Einsatzolefine hängt stark von ihrer Herkunft ab. So kommen zur Herstellung von $C_9$-Aldehyden unterschiedliche $C_8$-Olefine in Betracht: Einfachstenfalls wird 1-Octen verwendet, das zum Beispiel durch Oligomerisierung von Ethylen (s.u.) oder neben anderen Olefinen durch einen Fischer-Tropsch-Prozess dargestellt werden kann. Auf andere Weise gewonnenes 1-Octen wird stets begleitet von einer großen Zahl strukturisomerer $C_8$-Olefine. So

gesehen wird nicht ein einziges Olefin hydroformyliert, sondern ein Einsatzgemisch verwendet, welches eine Vielzahl von isomeren Olefinen umfasst.

Ein Gemisch aus $C_8$-Olefinen mit einer definierten Anzahl von Isomeren lässt sich durch die Oligomerisierung von $C_2$ oder $C_4$-Olefinen erhalten:

Unter der Oligomerisierung versteht man die Reaktion von Kohlenwasserstoffen mit sich selbst, wobei entsprechend längerkettige Kohlenwasserstoffe entstehen. So lässt sich beispielsweise durch die Oligomerisierung von zwei Olefinen mit drei Kohlenstoffatomen (Dimerisierung) ein Olefin mit sechs Kohlenstoffatomen aufbauen. Verbinden sich hingegen drei Olefine mit drei Kohlenstoffatomen miteinander (Trimerisierung), entsteht ein Olefin mit neun Kohlenstoffatomen.

Werden Butene - das sind so genannte $C_4$-Olefine mit vier Kohlenstoffatomen - einer Oligomerisierung unterworfen, entstehen dabei im Wesentlichen Olefine mit acht Kohlenstoffatomen (oft "Dibutene" genannt), Olefine mit zwölf Kohlenstoffatomen ($C_{12}$-Olefine, "Tributene") sowie in einem geringeren Umfang Olefine mit mehr als zwölf Kohlenstoffatomen ($C_{12+}$-Olefine). Technisch unterscheidet man zwischen sog. Di-n-Butenen, also isomeren $C_8$-Olefinen, die aus Gemischen von 1-Buten und/oder 2-Butenen hergestellt werden und so genannten Diisobutenen, die durch Dimerisierung von Isobuten erhalten werden und einen höheren Verzweigungsgrad aufweisen.

Di-n-butene, also Gemische von $C_8$-Olefinen, die durch Oligomerisierung von linearen Butenen entstehen, sind für die Herstellung von hoch linearen Oxo-Alkoholen deutlich besser geeignet als Diisobutene, da ihr Verzweigungsgrad viel geringer ist.

Noch geringer verzweigt sind $C_8$-Olefingemische, die durch Oligomerisierung von dem $C_2$-Olefin Ethen erhalten werden. Ethen entsteht beim Cracken von Naphta, wird aber in großen Mengen zur Herstellung von Polyethylen benötigt und ist deswegen ein vergleichsweise teurer Rohstoff. Deswegen ist die Herstellung von $C_8$-Olefinen aus Ethen nicht immer wirtschaftlich, wenngleich sich daraus hochwertige Oxo-Alkohole produzieren lassen.

Deutlich preisgünstiger als Ethen sind die $C_4$-Olefingemische, die ebenfalls beim Cracken von Naphta entstehen (so genanntes Crack-$C_4$). Die Herstellung von $C_9$-Alkohlen aus diesen Rohstoffen im Wege der Oligomerisierung und der anschließenden Hydroformylierung und Hydrierung ist wirtschaftlich interessant, sofern es gelingt, aus einem preiswerten $C_4$-Gemisch von minderer Qualität hinreichend lineares Isononanol herzustellen.

Ein industriell praktiziertes Verfahren zur Oligomerisierung von $C_4$-Olefinen ist der sogenannte "OCTOL-Prozess". Die ausführliche Beschreibung desselben findet sich in der Nicht-Patentliteratur, beispielsweise unter:

- B. Scholz: The HÜLS OCTOL Process: Heterogeneously catalyzed dimerization of n-butenes and other olefins. DGMK-Tagung in Karlsruhe, veröffentlicht in Erdöl, Erdgas, Kohle, April 1989, Seiten 21 und 22.
- R.H. Friedlander, D.J. Ward, F. Obenaus, F. Nierlich, J. Neumeister: Make plasticizer olefins via n-butene dimerization. Hydrocarbon Processing, February 1986, Seiten 31 bis 33.
- F. Nierlich: Oligomerize for better gasoline. Hydrocarbon Processing, February 1992, Seiten 45 bis 46.

Innerhalb der Patentliteratur beschreibt beispielsweise DE102008007081A1 eine auf dem OCTOL-Prozess beruhende Oligomerisierung. EP1029839A1 befasst sich mit der Fraktionierung der in dem OCTOL-Prozess entstehenden $C_8$-Olefine.

Der OCTOL-Prozess wird in der Regel mehrstufig mit Hilfe einer Reaktorkaskade durchgeführt, die eine der Stufenanzahl entsprechenden Anzahl von hintereinandergeschalteten Reaktionszonen bzw. Reaktoren umfasst. Zwischen den einzelnen Reaktionszonen ist jeweils eine Destillationskolonne vorgesehen, welche die zuvor entstandenen Oligomere aus dem Oligomerisat von den nicht umgesetzten Butenen abtrennt und ausschleust. Die nicht umgesetzten Butene werden teilweise in die vorhergehende Oligomerisierung zurückgeführt und zum anderen Teil der nachfolgenden Oligomerisierung zugeführt.

Ein weiteres Mehrstufen-Verfahren zur Oligomerisierung von $C_4$-Olefinen ist aus WO99/25668 bzw. aus DE10015002A1 bekannt. Hier wird eine Verdünnung der bereitgestellten Olefin-Ströme durch rückgeführte Butane praktiziert, um die Wärmeabfuhr aus der exothermen Reaktion über den Reaktoraustrag zu vereinfachen.

Je nachdem, auf welche Art und Weise sich die einzelnen n-Buten-Moleküle im Zuge der Oligomerisierung verbinden, erhält man ein Oligomerisat mit unterschiedlichem Verzweigungsgrad. Der Verzweigungsgrad wird durch den Isoindex beschrieben, welcher die mittlere Anzahl der Methylgruppen pro $C_8$-Molekül in dem Isomerengemisch angibt.

Der Isoindex für Dibuten ist in Formel (1) definiert:

$$\text{Isoindex} = (\text{Gewichtsanteil Methylheptene} + 2^{*}\ \text{Gewichtsanteil Dimethylhexene}) / 100 \qquad (1)$$

So tragen n-Octene mit 0, Methylheptene mit 1 und Dimethylhexene mit 2 zum Isoindex eines Produktgemisches aus $C_8$-Olefinen bei. Je niedriger der Isoindex, umso weniger verzweigt sind die Moleküle innerhalb des Gemisches aufgebaut.

Für die Eigenschaften des Weichmachers spielt nun der Verzweigungsgrad des olefinischen Ausgangsgemisches, welches für die Herstellung des Weichmacheralkohols verwendet wird, eine maßgebliche Rolle: Je höher die Linearität des $C_8$-Olefingemisches, desto besser sind die Eigenschaften des daraus hergestellten $C_9$-Weichmacheralkohols. Bei der Herstellung von Dibuten als Ausgangsprodukt für Weichmacheralkohole wird somit angestrebt, die Oligomerisierung so zu fahren, dass ein $C_8$-Produktgemisch erhalten wird, dessen Isoindex möglichst klein ist.

So wird beispielsweise in EP1029839A1 die Fraktionierung der Oligomerisate so eingerichtet, dass das abgetrennte $C_8$-Produktgemisch einen möglichst geringen Isoindex aufweist.

In WO99/25668A1 wird indes ein geringer Isoindex dadurch erreicht, dass man solche Mengen des vom Oligomerisat abgetrennten Butans und nicht umgesetzten Butens in die Oligomerisierung zurückführt, dass der maximale Gehalt an Oligomeren im umgesetzten Reaktionsgemisch 25 Gew.-% an keiner Stelle der Reaktorkaskade übersteigt.

Beide Verfahren nutzten als Ausgangsgemisch für die Oligomerisierung jeweils ein "Raffinat II", welches einen hohen Anteil an 1-Buten aufweist. Unter "Raffinat II" wird gemeinhin ein Butan/Buten-Gemisch verstanden, welches aus $C_4$-Schnitten erhalten wird, die aus Steam-Crackern stammen und aus denen bereits Butadien und Isobuten abgetrennt wurden. So enthält typisches Raffinat II um die 50 Gew.-% 1-Buten.

Es lässt sich zeigen, dass ein hoher Anteil an 1-Buten im bereitgestellten Kohlenwasserstoffgemisch sich günstig auf die Linearität des Oligomerisats auswirkt. Deshalb verwundert es nicht, dass die WO99/25668A1 ausgehend von dem Rohstoff Raffinat II C8-Produktgemische herstellt, deren Isoindex kleiner ist als 1.

Auch Nierlich hebt im seinem oben zitierten Aufsatz "Oligomerize for better gasoline", hervor, dass sich Raffinat II besser als Ausgangsstoff für eine Oligomerisierung eigne als Raffinat III. "Raffinat III" erhält man durch Entfernung von 1-Buten aus Raffinat II, weswegen sein Gehalt an 1-Buten deutlich geringer ist als der von Raffinat II. Da 1-Buten ebenfalls ein wichtiges Zielprodukt der C4-Chemie darstellt, das unter anderem bei der Herstellung bestimmter Polyethylen-basierter Kunststoffe mit geringer Dichte (sog. Linear Low Density Polyethylene, LLDPE) Verwendung findet, besteht ein großes industrielles Interesse an der Isolierung von 1-Buten aus Raffinat-II-Strömen. Dies führt zunehmend dazu, dass für andere chemische Verwendungen, wie zum Beispiel die Oligomerisierung zunehmend nur noch Raffinat-III-Ströme zur Verfügung stehen.

Auch aufgrund der inzwischen eingetretenen Rohstoffverknappung ist das petrochemisch hergestellte Raffinat II nicht mehr überall in großen Mengen und zu günstigen Konditionen verfügbar. So liefern alternative Rohstoffquellen $C_4$-Olefingemische, die teilweise fast gar kein 1-Buten mehr enthalten, sondern überwiegend 2-Buten.

Zu nennen ist hier vor allem das aus fluidkatalytischen Crackern stammende FCC-$C_4$. Andere 1-Buten-arme $C_4$-Quellen sind chemische Prozesse, wie die Dehydrierung von Butanen, sowie die fermentative oder die pyrolytische Umwandlung von nachwachsenden Rohstoffen.

Im Gegensatz zu klassischem Crack-$C_4$ weisen diese alternativen $C_4$-Gemische nicht nur einen geringen Anteil an 1-Buten auf, sondern unterliegen auch hinsichtlich ihrer Zusammensetzung zeitlichen Schwankungen:

So lässt sich der $C_4$-Strom einer unsteten Quelle allgemein als eine Summe von einzelnen Massenströmen reiner Substanzen auffassen, wobei sich die jeweiligen Substanzmassenströme innerhalb bestimmter Substanzmassenstrombereiche mit einer bestimmten Veränderungsgeschwindigkeit verändern. Tabelle 1 zeigt die dynamische Spezifikation eines sich mit der Zeit hinsichtlich seiner Zusammensetzung verändernden $C_4$-Stroms.

Tabelle 1: Dynamische Spezifikation eines unsteten $C_4$-Stroms

| Substanz | Substanzmassenstrom | Veränderungsgeschwindigkeit |
|---|---|---|
| Isobuten: | 0 kg/s bis 1 kg/s | - 0.05 $g/s^2$ bis 0.05 $g/s^2$ |
| 1-Buten: | 0 kg/s bis 6 kg/s | - 0.30 $g/s^2$ bis 0.30 $g/s^2$ |
| 2-Buten (cis + trans): | 1 kg/s bis 13 kg/s | - 0.30 $g/s^2$ bis 0.30 $g/s^2$ |
| Isobutan: | 0 kg/s bis 3 kg/s | - 0.15 $g/s^2$ bis 0.15 $g/s^2$ |
| n-Butan: | 1 kg/s bis 7 kg/s | - 0.30 $g/s^2$ bis 0.30 $g/s^2$ |
| Sonstige Stoffe: | 0 kg/s bis 1 kg/s | - 0.05 $g/s^2$ bis 0.05 $g/s^2$ |

So könnte ein spezifikationsgemäßer Strom pro Sekunde 1 kg 1-Buten liefern, wobei sich dieser Wert mit einer Geschwindigkeit von 0.25 $g/s^2$ verändern dürfte. Dies bedeutet, dass innerhalb von 100000 Sekunden (=28 Stunden) der Teilmassenstrom des 1-Buten auf bis zu 3.5 kg/s anschwillt. Umgekehrt ist auch eine Verringerung des 1-Buten-Gehalt mit einer Veränderungsgeschwindigkeit von -0.1 $g/s^2$ spezifikationsgerecht, sodass ein 1 kg/s 1-Buten-Strom innerhalb von weniger als drei Stunden (nämlich in 10 000 Sekunden) vollständig versiegt, sodass der $C_4$-Strom plötzlich frei von 1-Buten ist. Da sämtliche Komponenten zeitlichen Schwankungen unterliegen, kann es auch zu Schwankungen des Gesamtmassenstromes kommen. Diese Schwankungen werden limitiert durch die Grenzen des Betriebsbereichs der jeweiligen Anlage.

Die Herstellung qualitativ hochwertiger Oxo-Alkohole aus solch unsteten Rohstoffquellen ist der technisch anspruchsvolle Hintergrund dieser Erfindung. Trotz der technischen Schwierigkeiten besteht an einem entsprechenden Prozess ein großes wirtschaftliches Interesse, da durch die zunehmende Verknappung $C_4$-haltiger Rohstoffströme zukünftig kleinere Ströme aus vielen unterschiedlichen Quellen vereinigt eingesetzt werden müssen, um World-Scale-Anlagen der $C_4$-Aufarbeitung hinreichend versorgen zu können. Diese Ströme haben mitunter sehr unterschiedliche Zusammensetzungen, die zum Teil auch jahreszeitlich schwanken können. Dies führt zu einer deutlich unsteteren Rohstoffversorgung als die bislang übliche Versorgung mit Crack-$C_4$ aus einem oder wenigen Naphtha-Crackern.

Sowohl auf dem Gebiet der Oligomerisierung, als auch auf dem der Hydroformylierung gibt es erste Versuche, mit schwankender Eduktqualität umzugehen:

In WO 99/25668 A1 wird beschrieben, dass die Skelettisomerenverteilung des $C_8$-Produktes beeinflusst werden kann durch Rückführung eines Teils des Reaktoraustrages in die Reaktion.

Die zum Anmeldezeitpunkt noch unveröffentlichte deutsche Patentanmeldung DE 102013212481.3 zeigt, dass auch aus minderwertigen, an 1-Buten-armen $C_4$-Strömen noch ein Produkt mit vergleichsweise guter Isomerenverteilung hergestellt werden kann. Beide Literaturquellen stellen übereinstimmend dar, dass für die Erzeugung eines den Anwendererwartungen entsprechenden Weichmachers nur Dibutenströme mit geringem Verzweigungsgrad als Ausgangsprodukt für die Hydroformylierung verwendet werden können. Die Bewertung des Verzweigungsgrades erfolgt anhand des Isoindexes gemäß Formel (1). Je niedriger der Isoindex, umso weniger verzweigt sind die Moleküle innerhalb des Gemisches aufgebaut. DE 102013212481.3 stellt dar, dass vorzugsweise nur $C_8$-Olefingemische mit einem Iso-Index kleiner 1,10 für die Weiterverarbeitung zu Weichmacheralkoholen verwendbar sind. Besonders bevorzugt werden sogar nur Gemische mit einem Iso-Index kleiner 1,05 eingesetzt.

Bei der Verwendung von Rhodiumkomplexen als Katalysator für die Hydroformylierung ist der Ligand für die Produktzusammensetzung der Aldehyde mitbestimmend. Nicht-modifizierte Rhodiumcarbonylkomplexe katalysieren die Hydroformylierung von Olefinen mit end- und innenständigen Doppelbindungen, wobei die Olefine auch verzweigt sein können, zu Aldehyden mit einem hohen Verzweigungsgrad. Der Anteil an terminal hydroformyliertem Olefin ist im Vergleich zum Cobalt-katalysierten Produkt meist geringer.

Mit einem ligandmodifizierten Katalysatorsystem, bestehend aus Rhodium und Triorganophosphin, z.B. Triphenylphosphin werden mit hoher Selektivität nur Olefine mit terminaler Doppelbindung hydroformyliert. Eine Isomerisierung der Doppelbindung und die Hydroformylierung der innenständigen Doppelbindungen treten kaum auf.

Die Hydroformylierung von Olefinen mit innenständigen Doppelbindungen an Katalysatorsystemen, die sterisch anspruchsvolle Bisphosphitliganden enthalten, erfolgt bei langkettigen Olefinen zwar mit guter Selektivität, jedoch nicht mit zufriedenstellender Aktivität. Siehe hierzu:

P. W. N. M. van Leeuwen, in Rhodium Catalyzed Hydroformylation, P. W. N. M. van Leeuwen, C. Claver (Hrsg.), Kluwer, Dordrecht, 2000.

Rhodium-Monophosphit-Komplexe in katalytisch aktiven Zusammensetzungen hingegen sind geeignet für die Hydroformylierung von verzweigten langkettigen Olefinen mit innenständigen Doppelbindungen.

Seit den 1970er Jahren ist die Verwendung von so genannten "bulky phosphites" in der Hydroformylierung beschrieben:

H. Tricas, O. Diebolt, P. W .N .M .van Leeuwen, Journal of Catalysis, 2013 (298), S. 198-205

Diese zeichnen sich durch eine gute Aktivität aus, jedoch ist die n/i-Selektivität für endständig hydroformylierte Verbindungen verbesserungswürdig.

Neben dem Einsatz von reinen Liganden ist auch der Einsatz von Ligandenmischungen in der Literatur beschrieben.

In US 20120253080 wird der Einsatz von Monophosphiten mit Bisphosphiten beschrieben. Der Einsatz erfolgt als so genannter "Monitoring Ligand". Bisphosphite haben eine deutlich höhere Komplexbildungskonstante als Monophosphite und bilden so stabilere aber weniger aktive Katalysatorkomplexe. Jedoch hat diese Kombination den Nachteil, dass die Bisphosphite zwar eine ausgezeichnete Selektivität besitzen, im Fall von langkettigen Olefinen ihre Aktivität jedoch verbesserungswürdig ist, da in einem großtechnischen Prozess neben der Selektivität zum gewünschten Produkt auch die Raum-Zeit-Ausbeute bzw. die Aktivität des Katalysatorsystems eine elementare Rolle spielt. Außerdem sind die Bisphosphite in ihrer Herstellung meist deutlich teurer als beispielsweise Monophosphite.

In EP1099678B1 wird der kombinierte Einsatz von Phosphoniten mit Bisphosphiten beschrieben. Hierbei ist jedoch nachteilig, dass beide Ligandentypen in ihrer Herstellung sehr teuer sind und ein großtechnischer Prozess somit kaum wirtschaftlich sein kann. Außerdem beeinflusst der Zusatz des Bisphosphitliganden die Ausbeute der Reaktion

merklich, da diese Liganden bei der Verwendung von z.B. Dibuten als Substrat weniger aktiv sind. Ein weiteres Problem ist, dass Phosphonite auch sehr viel instabiler sind als andere Organophosphor-Liganden.

Der Austrag aus der Dibutenhydroformylierung enthält je nach gewähltem Verfahren eine Mischung der isomeren $C_9$-Aldehyde (sog. Isononylaldehyde, kurz INAL) und der isomeren C9-Alkohole (sog. Isononylalkohole, kurz INA). Die Mischung kommt dadurch zustande, dass Hydroformylierungskatalysatoren zu einem gewissen Grad auch hydrieraktiv sind, d.h. sie katalysieren die Reaktion der Aldehyde mit dem im Synthesegas enthaltenen Wasserstoff zu Alkoholen. Da für die avisierte Anwendung nur die Isononylalkohole Verwendung finden, wird der Austrag der Hydroformylierung einer Hydrierung unterzogen, um die enthaltenen Aldehyde zu Alkoholen umzuwandeln. Die katalytische Hydrierung von Aldehyden zu Alkoholen ist in der Literatur breit beschrieben, sie kann sowohl heterogen, wie auch homogen katalysiert ablaufen. Einen Überblick liefern

J. Falbe, H. Bahrmann, W. Lipps, D. Mayer, G. D. Frey, Alcohols Aliphatic in Ullmann's Encyclopedia of Industrial Chemistry, 2013

und

D. Sanfilippo, P. N. Rylander, Hydrogenation and Dehydrogenation in Ullmann's Encyclopedia of Industrial Chemistry, 2009.

Der Hydrierprozess hat keinen messbaren Einfluss auf die Isomerenverteilung, d.h. die Skelettisomeren des Aldehyds finden sich in gleichem Verhältnis im Alkohol wieder.

Aus EP1430014B1 ist bekannt, dass die Isomerenverteilung des Isononylalkohols direkt die Eigenschaften des durch Veresterung daraus hergestellten Weichmachers beeinflusst. Die Veresterung mit Carbonsäuren, insbesondere mit Phthalsäure ist ebenfalls wohlbekannt. Technisch wird die Alkoholmischung mit Phthalsäure oder Phthalsäureanhydrid verestert. Das als Weichmacher eingesetzte Produkt ist dann das Diisononylphthalat (DINP). Dieses kann prinzipiell auch durch Umesterung eines Dialkylphthalats mit Isononylalkohol hergestellt werden, Verwendung findet in dem Zusammenhang vor allem Dimethylphthalat. EP1430014B1 zeigt, dass der Prozessschritt der Estererzeugung keinen Einfluss auf die Qualität des Weichmachers hat, diese wird ausschließlich von den Eigenschaften des $C_9$-Alkohols bestimmt.

Für die Abhängigkeit der anwendungstechnischen Eigenschaften von der Struktur gibt es qualitative Regeln. Wilson beispielsweise diskutiert die Eigenschaften von Phthalaten in Abhängigkeit von der Gesamt-Kohlenstoff-Zahl, das heißt von der Molmasse, und dem Verzweigungsgrad, also unter anderem von der Isomerenzusammensetzung:

Alan S. Wilson, Plasticizers, The Institute of Materials, 1995, ISBN 0 901716 76 6, Seiten 135-136.

Bei gleicher Molmasse bewirkt zum Beispiel wachsende Verzweigung unter anderem die negativen Effekte einer wachsenden Viskosität, eines ansteigenden Dampfdrucks, was mit einer höheren Flüchtigkeit einhergeht, eine geringere weichmachende Wirkung und eine geringere Thermo- und Lichtstabilität.

Anders herum bewirkt eine hohe Verzweigung auch positive Effekte, nämlich eine bessere PVC-Verträglichkeit, eine geringere Migration, eine größere Hydrolysebeständigkeit, einen geringerer biologischen Abbau (während der Gebrauchsphase) und einen höheren elektrischen Widerstand.

Es ist sofort ersichtlich, dass es den besten Weichmacher nicht gibt, man muss je nach Anwendung einen Kompromiss schließen. So wird man, wenn die weichmachende Wirkung im Vordergrund steht, einen Weichmacher mit möglichst geringer Verzweigung bevorzugen. Will man hingegen mit PVC Kabelummantelungen herstellen, wird man eher zu Produkten mit etwas höherer Verzweigung greifen wegen der besseren elektrischen Isolierwirkung.

Wadey et al. zeigen an Hand von einigen synthetisch hergestellten Einzelisomeren an DINP sehr deutlich, wie wichtige Eigenschaften von der Struktur der Ester abhängen:

Brian L. Wadey, Lucien Thil, Mo A. Khuddus, Hans Reich; The Nonyl Phthalate Ester and It's Use in flexible PVC, Journal of Vinyl Technology, 1990,12, S. 208-211

Zusammenfassend kann festgestellt werden, dass die anwendungstechnischen Eigenschaften von der Struktur des Phthalats, diese grundlegend von der Struktur des Alkohols und diese wiederum beträchtlich von der Struktur des zugrundeliegenden Olefins abhängen. Zusätzlich komplizierend wirkt, dass diese Verbindungen als Isomerengemische produziert werden.

Wenn einmal eine Rezeptur entwickelt ist, muss nun in der Produktion sichergestellt werden, dass ein Produkt mit gleichbleibenden Eigenschaften hergestellt wird. Beispielsweise darf bei Schmiermitteln die Viskosität nur in engen Grenzen variieren, um die gewünschte Viskositätsklasse einzuhalten. Bei Weichmachern muss beispielsweise die weichmachende Wirkung konstant bleiben, um die Verarbeiter des Weichmachers nicht zu ständiger Rezepturanpassung zu zwingen, oder es darf ein Grenzwert für den elektrischen Widerstand nicht unterschritten werden. Eine laufende Produktionskontrolle durch anwendungstechnische Prüfungen ist aber zeitlich gar nicht möglich, ganz abgesehen von den Kosten, die dies verursachen würde.

Ein Konstanthalten der Isomerenzusammensetzung des Einsatzgemisches könnte das Problem theoretisch zwar lösen, dies ist aber in der Praxis nicht durchzuhalten. Ein Cracker wird nicht betrieben, um einen $C_4$-Strom konstanter Zusammensetzung zu liefern, sondern um Ethylen, Propylen, Benzin oder andere Massenprodukte herzustellen. In der Praxis wird die Zusammensetzung des $C_4$-Stromes, der aus dem Cracker zur Verfügung steht, immer je nach Zusammensetzung seines Rohstoffes und nach Fahrweise schwanken. Das Problem verschärft sich weiter, wenn $C_4$-Ströme aus unterschiedlichen Crackern zugekauft werden, wie dies bei einer Großproduktion schon heute die Regel ist und zukünftig noch stärker der Fall sein wird.

Allein schon durch den am Anfang stehenden Rohstoff kommt es somit zwangsweise zu Änderungen in der Zusammensetzung des Produktes, somit zu Änderungen in der Isomerenverteilung und damit zu Änderungen in den anwendungstechnischen Eigenschaften. Wie schon vorher diskutiert, kommen weitere Änderungen in der Produktzusammensetzung in den nachfolgenden Schritten Oligomerisierung und Hydroformylierung, zum Beispiel durch Änderung der Betriebsbedingungen oder Alterung des Oligomerisierungskatalysators, zum Tragen.

Nach Alledem lässt sich festhalten, dass das Problem der konstanten Produktqualität bislang nicht ganzheitlich beleuchtet wurde. Die einzelnen Prozessschritte wurden immer als unabhängige Einheiten betrachtet. Die Qualität des nach klassischen Verfahren produzierten Weichmachers schwankt innerhalb definierter Grenzen, wobei diese Schwankung zufällig und von zahlreichen äußeren Einflüssen abhängig ist.

Die der Erfindung im Wesentlichen zugrunde liegende Aufgabe ist ein Verfahren zur Herstellung von Oxo-Alkoholen aus schwankenden Rohstoffquellen anzugeben, das es erlaubt, über einen langen Produktionszeitraum eine konstante Weichmacherqualität zu erzeugen. Zudem soll das Verfahren die Nachfrage des Marktes berücksichtigen und bei Bedarf unter Beibehaltung der Produktqualität einen höheren Durchsatz ermöglichen.

Gelöst werden diese Aufgaben

- durch eine Steuerung der Temperatur und/oder des Umsatzes der Oligomerisierung in Abhängigkeit von der gegenwärtigen Zusammensetzung des Oligomerisats;
- und durch eine Steuerung der Zusammensetzung des Katalysatorsystems und/oder des Drucks der Hydroformylierung in Abhängigkeit von der gegenwärtigen Zusammensetzung der Aldehyde.

Die beiden Steuerungen setzen denknotwendig voraus, dass die Zusammensetzung des Oligomerisats und die Zusammensetzung der Aldehyde bestimmt werden.

Angewendet werden diese Maßnahmen auf ein herkömmliches Verfahren zur kontinuierlichen Herstellung eines Alkoholgemisches der eingangs genannten Gattung.

Gegenstand der Erfindung ist mithin ein Verfahren gemäß Anspruch 1 zur kontinuierlichen Herstellung eines Alkoholgemisches, bei welchem ein Olefine enthaltendes Einsatzgemisch, dessen Zusammensetzung sich über die Zeit verändert, unter Erhalt eines Oligomerisats einer Oligomerisierung unterworfen und zumindest ein Teil der im Oligomerisat enthaltenden Olefinoligomere in einer Hydroformylierung in Gegenwart eines homogenen Katalysatorsystems mit Kohlenmonoxid und Wasserstoff in Aldehyde oxiert werden, von denen zumindest ein Teil durch anschließende Hydrierung in das Alkoholgemisch umgesetzt werden,
bei welchem die Zusammensetzung des Oligomerisats und die Zusammensetzung der Aldehyde bestimmt werden,
bei welchem die Temperatur und/oder der Umsatz der Oligomerisierung in Abhängigkeit von der gegenwärtigen Zusammensetzung des Oligomerisats gesteuert werden,
und bei welchem die Zusammensetzung des Katalysatorsystems und/oder der Druck der Hydroformylierung in Abhängigkeit von der gegenwärtigen Zusammensetzung der Aldehyde gesteuert werden.

Eine Grundidee der vorliegenden Erfindung besteht darin, sowohl in der Oligomerisierung, als auch in der Hydroformylierung die Schwankungen der Einsatzstoffe ausgleichend zu steuern und dabei bestehende Rückkopplungseffekte zu berücksichtigen. Mithin wird ein systemischer Steuerungsansatz verfolgt, welcher die beiden für die Produktqualität der Oxo-Alkohole und der daraus hergestellten Weichmacher maßgeblichen Reaktionen erfasst.

Da die erfindungsgemäße Steuerung unter Berücksichtigung von Rückkopplung geschieht, liegt im engeren Sinne des deutschen Fachsprachgebrauchs vielmehr eine Regelung als eine Steuerung vor. Da aber nicht jede Fachsprache zwischen den Begriffen "Steuerung" und "Regelung" differenziert, wird im hier der Begriff der "Steuerung" verwendet, um Übersetzungsprobleme zu vermeiden.

Um die Steuerung der einzelnen Prozessschritte "Oligomerisierung" und "Hydroformylierung" zu ermöglichen, wird der Gesamtprozess über wenige, aussagekräftige Messgrößen beschrieben. Als Indikatoren dienen die Zusammensetzung des Oligomerisats und die Zusammensetzung der aus der Hydroformylierung abgezogenen Aldehyde.

Auf Basis dieser zwei Messgrößen können die Teilprozesse Oligomerisierung und Hydroformylierung so gesteuert werden, dass entweder Schwankungen in der Rohstoffqualität und/oder die Alterung von Katalysatoren ausgeglichen werden kann, oder dass bei Bedarf eine höhere Produktionsmenge bei gleichem Rohstoffeinsatz erzielt werden kann.

Die Steuerung der beiden Reaktionen erfolgt erfindungsgemäß nicht nur über die üblichen Prozessparameter Druck und Temperatur, sondern im Falle der Oligomerisierung auch über den Umsatz der Reaktion und im Falle der Hydroformylierung zusätzlich über die Zusammensetzung des Katalysatorsystems. Das erfindungsgemäße Verfahren bedient sich insoweit bisher ungenutzter Stellgrößen.

Unter Umsatz der Oligomerisierung versteht sich der prozentuale Massenanteil der in die Oligomerisierung eingebrachten Olefine, die zu Olefinoligomeren umgesetzt werden. Der Umsatz lässt sich durch Druck, Temperatur, Verweilzeit und spezifische Katalysatorbeladung beeinflussen. Bevorzugt wird der Umsatz der Oligomerisierung über die Produktrückführung gesteuert. Dazu später mehr.

Unter Veränderung der Zusammensetzung des Katalysatorsystems der Hydroformylierung ist zu verstehen, dass nicht durchgängig dasselbe homogene Katalysatorsystem genutzt wird, sondern dass die stoffliche Zusammensetzung des Katalysatorsystems verändert wird. Konkret bedeutet dies, dass das Mischungsverhältnis der Liganden, die sich an das Zentralmetall anlagern und so eine katalytisch aktive Komplexverbindung bilden, im laufenden Betrieb variiert wird. Auch dazu später mehr.

Als industrielles Verfahren wird die erfindungsgemäße Herstellung der Oxo-Alkohole kontinuierlich betrieben. Dies bedeutet, dass rund um die Uhr an allen Tagen in der Woche Einsatzgemisch in den Prozess eingebracht wird und daraus Oxo-Alkohole produziert werden. Unter Berücksichtigung der üblichen Abschalt- und Revisionszeiten rechnet man mit mindestens 8000 Betriebsstunden im Jahr.

In Hinblick auf die kontinuierliche Betriebsweise sollten die Messwerte Zusammensetzung des Oligomerisats und Zusammensetzung der Aldehyde ebenfalls kontinuierlich bestimmt werden, sodass die Oligomerisierung und/oder die Hydroformylierung auch kontinuierlich gesteuert werden. Die Maschenweite der Messungen wird in Abhängigkeit der Veränderungsgeschwindigkeit des Einsatzgemisches gewählt. Erste Betriebserfahrungen zeigen, dass eine Messung der Zusammensetzungen alle paar Stunden ausreichend ist, zumal die gesteuerten chemischen Reaktionen nicht instantan auf Veränderung ihrer Stellgrößen reagieren. Falls wie im oben beschriebenen Szenario ein Totalverlust des 1-Butens innerhalb von weniger als 3 Stunden zu erwarten wäre, sind selbstverständlich engere Messintervalle erforderlich. Wenn die Schwankungen moderater ausfallen, kann es aber auch genügen die Messungen alle 12 oder 24 Stunden durchzuführen. Unter einer "kontinuierlichen Bestimmung" ist in diesem Zusammenhang also eine Anpassung der Messintervalle an die erwartete Veränderungsgeschwindigkeit der jeweiligen Messgröße zu verstehen. Soweit von einer gegenwärtigen Zusammensetzung die Rede ist, ist damit die zu Beginn des laufenden Messintervalls analysierte Zusammensetzung gemeint.

Die Steuerung der Stellgrößen in Hinblick auf die Messwerte der Analytik kann grundsätzlich von Hand durch die Besatzung der Produktionsanlage erfolgen. Im Interesse eines hohen Automatisierungsgrades wird die Steuerung aber in einer an sich bekannten Prozessleittechnik implementiert.

Die Analyse der Zusammensetzung der Aldehyde ergibt einen mehrdimensionalen Datensatz. Dieser macht die die Steuerung der Hydroformylierung sehr komplex. Einfacher und damit effektiver steuern lässt sich die Oxo-Reaktion, indem aus der Zusammensetzung der Aldehyde zunächst eine skalare Regelgröße errechnet wird und die Steuerung darauf eingerichtet wird, diese Regelgröße konstant zu halten. Gemäß diesem Konzept wird aus dem mehrdimensionalen Datensatz, der die Zusammensetzung der aus der Hydroformylierung abgezogenen Aldehyde wiedergibt, zunächst nach einer festen mathematischen Berechnungsvorschrift eine eindimensionale (skalare) Größe errechnet. Diese dient dann als Regelgröße, zu deren Konstanthaltung der Druck und/oder die Katalysatorzusammensetzung der Hydroformylierung angepasst werden/wird, sobald die Regelgröße von einem vorgegebenen Sollwert abweicht. Die Regelung von Druck und/oder Katalysatorzusammensetzung erfolgt dann mit einem herkömmlichen Regler mit Proportional-, Integral-, Differenzial- oder PID-Charakteristik.

Im Übrigen ist unter Konstanthaltung in diesem Zusammenhang nicht zwangsläufig eine mathematische Konstanz zu verstehen sondern vielmehr, dass die Regelung der Regelgröße gewisse Abweichungen von der Sollgröße erlaubt, wobei die Toleranz des Reglers geeignet eingestellt wird. Unter einem konstanten Regelwert ist also die Einhaltung eines Toleranzwertbereiches um die eigentliche Sollgröße gemeint (regelungstechnische Konstanz).

Zweckmäßigerweise wird nicht nur die Hydroformylierung, sondern auch die Oligomerisierung in dieser Weise geregelt. Dementsprechend werden/wird die Temperatur und/oder der Umsatz der Oligomerisierung ebenfalls so gesteuert, dass die aus der Zusammensetzung der Aldehyde errechnete, erste skalare Regelgröße konstant gehalten wird. Die Regelung kann auch hier mit P-, I-, D-, oder PID-Charakteristik erfolgen.

Die kombinierte Steuerung von Oligomerisierung und Hydroformylierung in Hinblick auf die Konstanthaltung der die Aldehyd-Zusammensetzung repräsentierenden, ersten skalaren Regelgröße verbessert die Regelgüte des Gesamtprozesses und verstetigt damit die Produktqualität der hergestellten Oxo-Alkohole.

Insbesondere dann, wenn die produzierten Oxo-Alkohole dafür bestimmt sind, zu Weichmachern weiterverarbeitet zu werden, wird als erste skalare Regelgröße eine Näherung der Viskosität des Estergemisches herangezogen,

welches durch Veresterung des Alkoholgemisches oder durch Umesterung mit Hilfe des Alkoholgemisches erhältlich ist.

Oxo-Alkohole sind nur bedingt unmittelbar als Weichmacher für Kunststoffe einsetzbar. Insbesondere PVC lässt sich besser mit einem Estergemisch weich machen, welches aus dem Oxo-Alkohol hergestellt wird. Dies geschieht entweder durch Umsetzung des Alkohols mit einer Säure (Veresterung) oder durch Umesterung eines bestehenden Esters in Gegenwart des Alkohols. Für die Produktqualität des hergestellten Weichmachers ist die Viskosität des Estergemisches maßgeblich. Es wurde gefunden, dass sich die Viskosität des Weichmachers aus der Zusammensetzung der Aldehyde mit guter Näherung vorherbestimmen lässt, da weder die Hydrierung der Aldehyde zu den Alkoholen noch ihre Umsetzung zu den Ziel-Estern Einfluss auf deren Viskosität hat.

Es ist zwar aus EP1430014B1 bekannt, dass sich beispielsweise die Viskosität $\eta$ eines Phthalsäureestergemisches, welches durch Veresterung von Phthalsäure oder Phthalsäureanhydrid mit einem Gemisch aus isomeren Alkylalkoholen erhältlich ist, mit guter Näherung gemäß der Formel (2) berechnen lässt:

$$\ln(\eta) = \Sigma\, x_i \ln(\eta_i) \qquad (2)$$

wobei $x_i$ den Molenbruch des isomerenreinen Alkohols und $\eta_i$ die Viskositätskennzahl des isomerenreinen Alkohols bedeutet.

Gemäß der Erfindung wird aber die als erste skalare Regelgröße herangezogene Näherung der Viskosität $\eta$ des Estergemisches nicht aus den Alkoholen errechnet, sondern bereits aus den Aldehyden. Hierfür wird ebenfalls Formel (2) verwendet, jedoch steht dann $x_i$ für den Molenbruch des jeweiligen isomerenreinen Aldehyds und $\eta_i$ die Viskositätsbeitrag des isomerenreinen Aldehyds.

Der Vorteil der Berechnung der genäherten Viskosität aus den Aldehyden statt aus den Alkoholen besteht darin, dass die zu regelnde Größe unmittelbar hinter dem zu regelnden Prozess ermittelt wird und nicht erst mit weiter Zeitverzögerung stromabwärts hinter der Hydrierung. Damit ist das Ansprechverhalten des Regelkreises deutlich besser.

Die für die Berechnung der ersten skalaren Regelgröße $\eta$ gemäß Formel (2) benötigten Parameter $x_i$, also die Molenbrüche der einzelnen isomeren Aldehyde des Aldehydgemisches, werden durch die Analyse der Zusammensetzung des Aldehydgemisches erhalten. Dies kann in bekannter Weise durch Gaschromatographie geschehen. Die ebenfalls benötigten Koeffizienten $\eta_i$ stehen für die Viskositätsbeiträge des jeweiligen isomeren Aldehyds und sind damit Naturkonstanten. Sie werden für die in dem Prozess vorkommenden Aldehyde einmalig experimentell bestimmt. Die Viskositätsbeiträge $\eta_i$ der Aldehyde, die bei der Hydroformylierung von Dibuten zu erwarten sind, sind in Tabelle 2 dargestellt.

Tabelle 2: Viskositätsbeiträge von isomerenreinen $C_9$-Aldehyden

| Isononylisomere | Viskositätsbeitrag $\eta_i$ der Isomeren |
|---|---|
| n-Nonanal | 45,5 |
| 2-Methyloctanal | 72,2 |
| 2-Ethylheptanal | 98,7 |
| 2-Propylhexanal | 98,7 |
| 4-Methyloctanal | 67,2 |
| 2,3-Dimethylheptanal | 108,4 |
| 3-Ethylheptanal | 50,5 |
| 2-Propyl-3-methyl-pentanal | 229,5 |
| 2-Ethyl-4-methylhexanal | 158,4 |
| 2,5-Dimethylheptanal | 120,2 |
| 6-Methyloctanal | 61,1 |
| 4,5-Dimethylheptanal | 94,4 |
| 2,3,4-Trimethylhexanal | 574,3 |
| 3-Ethyl-4-methyl -hexanal | 120,6 |
| 3,5,5-Trimethylhexanal | 111,8 |
| Rest | (173,1) |

Die Viskosität ist stark abhängig von der Messtemperatur und auch von der Messmethode. Für die einzelnen Viskositätenbeiträge $\eta_i$ gilt, wie für die Gesamtviskosität des Estergemisches, dass sie unter Normalbedingungenbedingungen (293,15 K und 1013 mbar absolut) angegeben werden.

Auch für die Steuerung der Oligomerisierung fällt ein mehrdimensionaler Messwert an, nämlich die Zusammensetzung des Oligomerisats. Auch hier ist es im Interesse der Effektivität der Regelung opportun, diesen mehrdimensionalen Messwert in eine zweite skalare Regelgröße umzurechnen und diesen ebenso konstant zu halten, wie es oben analog für die Hydroformylierung beschrieben wurde.

Als für die Steuerung der Oligomerisierung benötigte, zweite skalare Regelgröße wird eine Näherung des Isoindex der Olefinoligomere herangezogen. Der Isoindex ist ein Maß für den Verzweigungsgrad des Oligomerisats. Er gibt im Falle des Dibutens die mittlere Anzahl der Methylgruppen pro $C_8$-Molekül in dem Isomerengemisch an. Der Isoindex für Dibuten ist gemäß Formel (1) definiert.

Zur Berechnung des Isoindex als zweite skalare Regelgröße werden lediglich der Gewichtsanteil der Methylheptene und der Gewichtsanteil der Dimethylhexene am Oligomerisat benötigt. Diese lassen sich in bekannter Weise beispielsweise durch hydrierende Gaschromatographie bestimmen.

Wird nicht das gesamte Oligomerisat der Hydroformylierung zugeführt, sondern nur ein Teil der gebildeten Olefinoligomere, wird vorzugsweise nur der Isoindex der zu hydroformylierenden Olefinoligomere bestimmt.

Grundsätzlich lässt sich der hier beschriebenen Maßnahmen auf jedweden Prozess zur Herstellung von Oxo-Alkoholen aus Olefinen anwenden, der über die Route Oligomerisierung, Hydroformylierung, Hydrierung läuft. Auf die Anzahl der Kohlenstoffatome der eingesetzten Olefine und auf die daraus resultierende Anzahl der Kohlenstoffatome der Oxo-Alkohole kommt es nicht an. Es können Einsatzgemische auf Basis von Ethen, Propen, Buten oder Penten verwendet werden und Oxo-Alkohole mit 3, 5, 7, 9, 11 und 13 Kohlenstoffatomen produziert werden.

Die Beispiele befassen sich mit einem $C_4$-basierten Prozess zur Herstellung von INA. Für den Fachmann sind die gezeigten Erkenntnisse jedoch ohne Weiteres auf $C_2$, $C_3$ oder $C_5$ basierte Prozesse übertragbar.

Für den Fall des Einsatzes von Buten als Rohstoff ist zu beachten, dass in der Oligomerisierung neben den gewünschten $C_8$-Olefinen auch solche mit zwölf, sechszehn und mehr Kohlenstoffatomen entstehen. Für die Herstellung von Isononanol ist es daher erforderlich, die Dibutene aus dem Oligomerisat abzutrennen. Somit besteht eine Weiterbildung der Erfindung darin, dass als Einsatzgemisch Olefine mit vier Kohlenstoffatomen eingesetzt werden, welche im Zuge der Oligomerisierung zu Olefinoligomeren mit acht, zwölf und sechzehn Kohlenstoffatomen oligomerisiert werden, und dass die Olefinoligomere mit acht Kohlenstoffatomen aus dem Oligomerisat abgetrennt und zu Aldehyden mit neun Kohlenstoffatomen hydroformyliert werden.

Das erfindungsgemäße Verfahren eignet sich hervorragend dafür, Oxo-Alkohole konstanter Qualität aus schwankenden Rohstoffströmen zu erzeugen. Sofern ein $C_4$-basierter Prozess betrieben wird, können durchaus Einsatzgemische mit Spezifikation gemäß Tabelle 1 verarbeitet werden.

Ein so stark schwankender $C_4$-Strom kann aus unterschiedlichen und vor allem preiswerten Quellen stammen. Es kann auch ein aus unterschiedlichen Quellen vereinter $C_4$-Schnitt verwendet werden.

Die Oligomerisierung erfolgt in an sich bekannter und bewährter Weise an einem heterogenen Nickel-Katalysator. Insoweit wird auf die Literatur zum OCTOL-Prozess verwiesen.

Die Oligomerisierung kann mehrstufig durchgeführt werden.

Die Oligomerisierung wird vorzugsweise in Kreislauffahrweise betrieben, dergestalt, dass ein Anteil der in dem aus der Oligomerisierung abgezogenen Oligomerisat enthaltenen unumgesetzten Butene in die Oligomerisierung zurückgeführt wird. Die Kreislauffahrweise eröffnet nämlich die Möglichkeit, den Umsatz der Oligomerisierung durch Veränderung des Anteils der zurückgeführten Butene zu steuern. Die Steuerbarkeit des Umsatzes über den Anteil der zurückgeführten Butene ist ausführlich beschreiben in der WO 99/25668 A1 sowie in der zum Anmeldezeitpunkt noch unveröffentlichten deutschen Patentanmeldung DE 102013212481.3.

Die Steuerung der Zusammensetzung des homogenen Katalysatorsystems der Hydroformylierung erfolgt dadurch, dass ein Katalysatorsystem verwendet wird, welches Rhodium sowie potentiell zumindest zwei unterschiedliche Monophosphit-Liganden umfasst, deren Mischungsverhältnis aktiv durch Zudosierung eines der beiden Monophosphit-Liganden beeinflusst wird. Wird einer der beiden Liganden zudosiert, verschiebt sich das Mischungsverhältnis zu seinen Gunsten. An den Grenzen des Mischungsbereichs kann es vorkommen, dass ein Ligand in der Mischung nur noch in Spuren enthalten ist.

Die Formulierung, dass das Katalysatorsystem "potentiell" Liganden umfasst, ist hier so zu verstehen, dass im Reaktionsgemisch die zur Rede stehenden Liganden anwesend sind und damit der Bildung des Katalysatorsystems grundsätzlich zur Verfügung stehen. Dies heißt aber nicht, dass alle im Reaktionssystem anwesenden Liganden katalytisch aktiv sind: In der Praxis wird nämlich stets ein Überschuss an Ligand dem Reaktionssystem zugeführt, sodass nicht die vollständige Menge Ligand an Rhodium komplexieren kann und mithin nicht katalytisch aktiv ist. Letztendlich lässt sich nicht genau sagen, welcher Ligand komplexiert und damit katalytisch aktiv wird. Im Sinne der Erfindung ist es aber entscheidend, den zugegebenen Liganden grundsätzlich die Möglichkeit zu geben, aktiver Teil des Katalysatorsystems zu werden. Eine im Reaktionsgemisch anwesende Substanz, welche dazu befähigt ist, mit Rhodium einen

katalytisch aktiven Komplex zu bilden, wird mithin als potentieller Ligand bezeichnet.

Die Verschiebung des Mischungsverhältnisses der potentiell im Katalystorkomplex aktiven Liganden geschieht in einer Weise, dass das molare Verhältnis der Summe aller Monophosphit-Liganden zu Rhodium unter Berücksichtigung von Ligandenverlusten und im Rahmen der technischen Möglichkeiten und Messgenauigkeiten konstant bleibt. Hintergrund dieser Maßgabe ist, dass in jeder Rh-katalysierten Hydroformylierung mit der Zeit Ligand verloren geht, sei es durch Desaktivierung, Zerfall oder Verlust über den Produktaustrag. Um die Konzentration des aktiven Katalysatorsystems im Reaktionsgemisch konstant zu halten, muss also ständig Ligand nachdosiert werden. Eine bevorzugte Weiterbildung der Erfindung sieht nun vor, dass bei Einsatz eines Katalysatorsystems mit mehreren Liganden nicht immer alle Liganden im gleichen (ursprünglichen) Mischungsverhältnis nachdosiert werden, sondern dass einer der Liganden bevorzugt nachdosiert wird, um die Zusammensetzung des Katalysatorsystems und damit die gesamte Hydroformylierung gezielt zu beeinflussen. Es wird dann praktisch der Verlust des einen Liganden weniger stark ausgeglichen als der des anderen.

Bevorzugt wird ein Katalysatorsystem eingesetzt, welches potentiell genau zwei Liganden umfasst. Dabei wird ein erster Ligand als "Basisligand" verwendet, der in einer großen Konzentration vorliegt und die Katalyse maßgeblich bewerkstelligt. Als Basisligand wird beispielsweise ein preiswertes Monophosphit verwendet. Der zweite Ligand wird indes als "Steuerligand" verwendet, um die Selektivität der Hydroformylierung durch Zudosierung zu steuern. Der Steuerligand sollte eine deutlich größere Selektivität als der Basisligand aufweisen, um die Selektivität der Oxo-Reaktion gezielt beeinflussen zu können. Mithin wird der Steuerligand deutlich teurer sein als der Basisligand, weswegen er in der Praxis verglichen mit dem Basisligand nur in geringer Konzentration eingesetzt wird. Anders als bei den im Stand der Technik üblichen Bisphosphit-"Monitoring"-Liganden sollte der erfindungsgemäße Steuerligand eine dem Basisliganden zumindest annähernd vergleichbare Aktivität besitzen.

Konkret bietet es sich an, die zu erwartenden Verluste des Basisliganden stets vollständig auszugleichen und die Verluste des Steuerliganden wahlweise vollständig, teilweise oder über zu kompensieren, je nachdem, in welche Richtung die Selektivität der Hydroformylierung gesteuert werden soll.

Sofern ein Katalysatorsystem mit genau zwei Liganden genutzt wird, bietet sich als erster Monophosphit-Ligand, also als Basisligand, eine Verbindung gemäß Strukturformel II an. Als zweiter Monophosphit-Ligand (Steuerligand) kann dann eine Verbindung gemäß einer der folgenden Strukturformeln Ia, Ib, oder III gewählt werden:

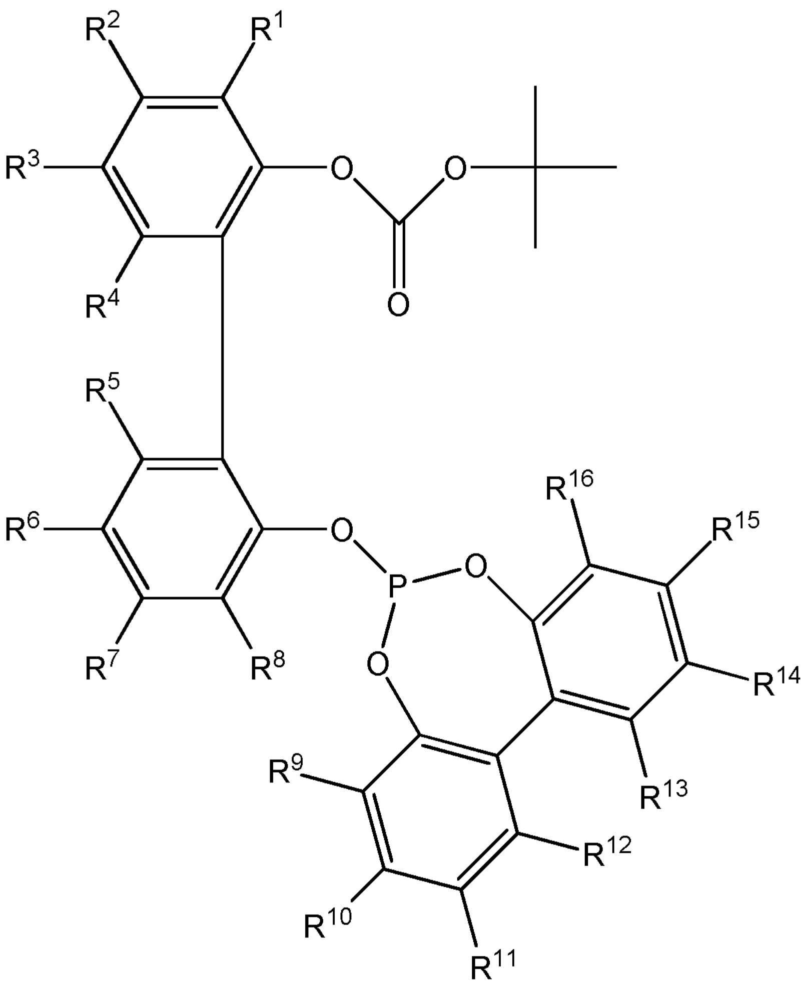

**Ia**

wobei in Ia

$R^1$, $R^2$, $R^3$, $R^4$, $R^5$, $R^6$, $R^7$, $R^8$ jeweils unabhängig voneinander ausgewählt sind aus:

-H, $-(C_1-C_{12})$-Alkyl, $-O-(C_1-C_{12})$-Alkyl, $-O-(C_6-C_{20})$-Aryl, $-(C_6-C_{20})$-Aryl, Halogen, COO-$(C_1-C_{12})$-Alkyl, CONH-$(C_1-C_{12})$-Alkyl, $-(C_6-C_{20})$-Aryl-CON[$(C_1-C_{12})$-Alkyl]$_2$, -CO-$(C_1-C_{12})$-Alkyl, -CO-$(C_6-C_{20})$-Aryl,-COOH, -OH, $-SO_3H$ $-SO_3Na$, $-NO_2$, -CN, $-NH_2$, -N[$(C_1-C_{12})$-Alkyl]$_2$;

wobei $R^9$, $R^{10}$, $R^{11}$, $R^{12}$, $R^{13}$, $R^{14}$, $R^{15}$, $R^{16}$ jeweils unabhängig voneinander ausgewählt sind aus:

-H, $-(C_1-C_{12})$-Alkyl, $-O-(C_1-C_{12})$-Alkyl, $-O-(C_6-C_{20})$-Aryl, $-(C_6-C_{20})$-Aryl, -Halogen, -COO-$(C_1-C_{12})$-Alkyl, -CONH-$(C_1-C_{12})$-Alkyl, $-(C_6-C_{20})$-Aryl-CON[$(C_1-C_{12})$-Alkyl]$_2$, -CO-$(C_1-C_{12})$-Alkyl, -CO-$(C_6-C_{20})$-Aryl, -COOH, -OH, $-SO_3H$ $-SO_3Na$, $-NO_2$, -CN, $-NH_2$, -N[$(C_1-C_{12})$-Alkyl]$_2$.

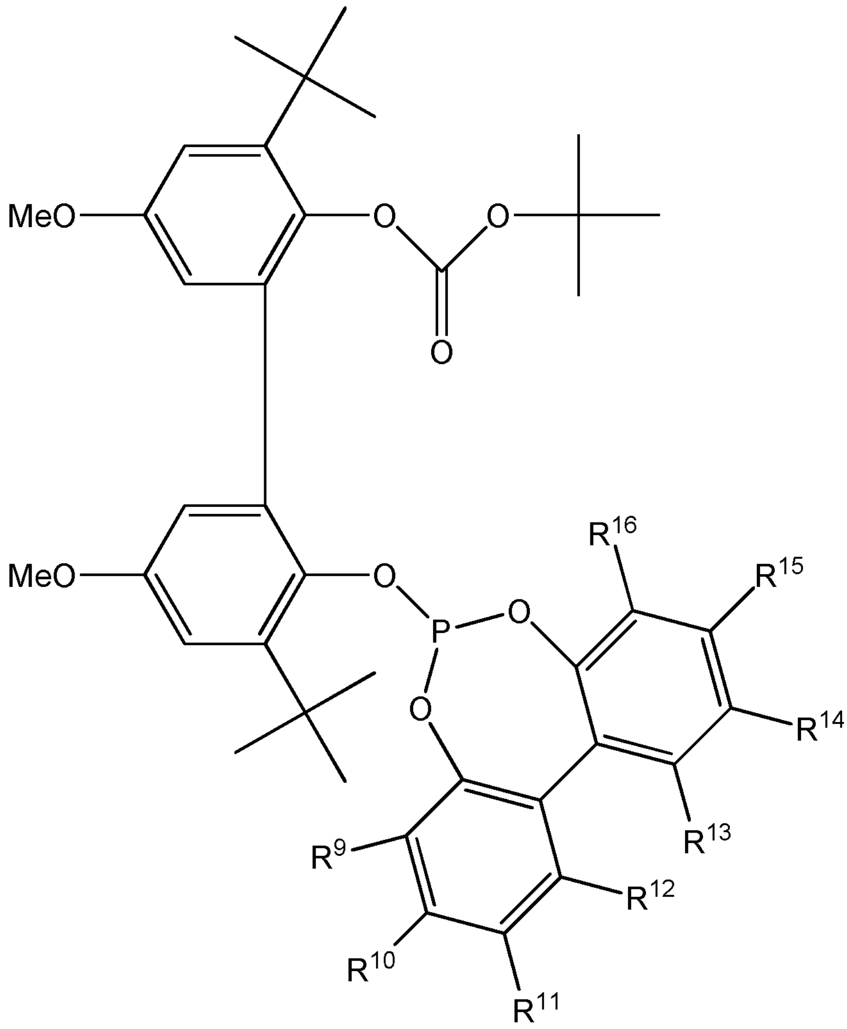

**Ib**

wobei in Ib

$R^9$, $R^{10}$, $R^{11}$, $R^{12}$, $R^{13}$, $R^{14}$, $R^{15}$, $R^{16}$ jeweils unabhängig voneinander ausgewählt sind aus: -H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, - OH, -$SO_3H$ -$SO_3Na$, -$NO_2$, -CN, -$NH_2$, -N[($C_1$-$C_{12}$)-Alkyl]$_2$.

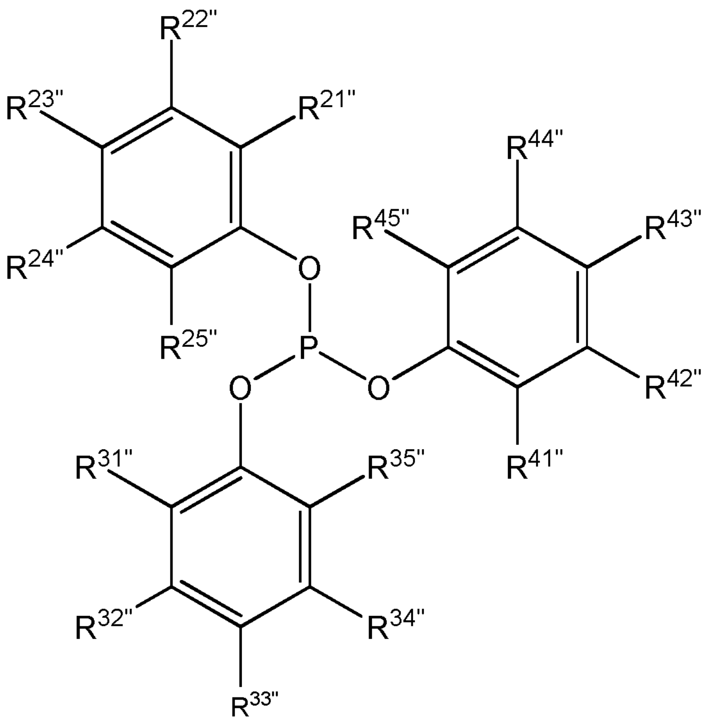

II

wobei in II
$R^{21"}$, $R^{22"}$, $R^{23"}$, $R^{24"}$, $R^{25"}$, $R^{31"}$, $R^{32"}$, $R^{33"}$, $R^{34"}$, $R^{35"}$, $R^{41"}$, $R^{42"}$, $R^{43"}$, $R^{44"}$, $R^{45"}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -COOH, - OH, -$SO_3H$ -$SO_3Na$, -$NO_2$, -CN, -$NH_2$, -N[($C_1$-$C_{12}$)-Alkyl]$_2$.

Bevorzugt sind in II $R^{21"}$, $R^{22"}$, $R^{23"}$ , $R^{24"}$, $R^{25"}$, $R^{31"}$, $R^{32"}$, $R^{33"}$, $R^{34"}$, $R^{35"}$, $R^{41"}$, $R^{42"}$, $R^{43"}$, $R^{44"}$, $R^{45"}$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.

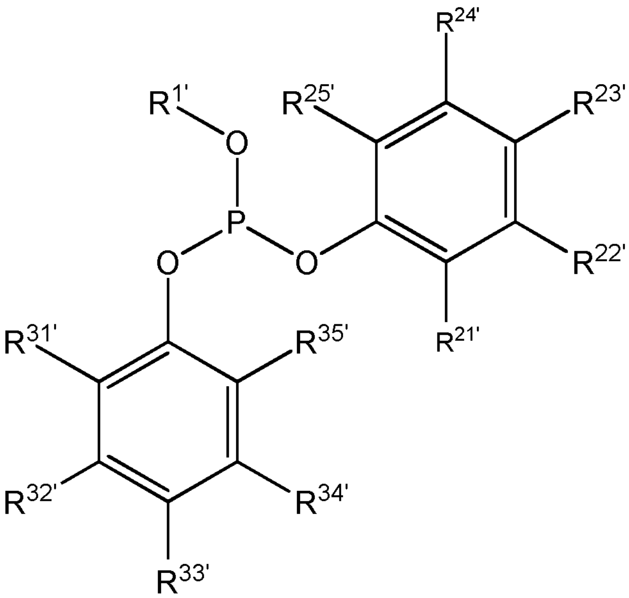

III

wobei in III

$R^{1'}$ ausgewählt ist aus:

-($C_1$-$C_{12}$)-Alkyl, -($C_3$-$C_{12}$)-Cycloalkyl, und

$R^{21'}$, $R^{22'}$, $R^{23'}$, $R^{24'}$, $R^{25'}$, $R^{31'}$, $R^{32'}$, $R^{33'}$, $R^{34'}$, $R^{35'}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH.

Bevorzugt sind in III $R^{21'}$, $R^{22'}$, $R^{23'}$, $R^{24'}$, $R^{25'}$, $R^{31'}$, $R^{32'}$, $R^{33'}$, $R^{34'}$, $R^{35'}$ jeweils unabhängig voneinander ausgewählt aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl.

Alle den Strukturformeln Ia, Ib, II und III unterfallenden Substanzen scheinen sich in Hinblick auf ihr katalytisches Verhalten ähnlich zu verhalten, sodass sie sich als Liganden im Sinne der Erfindung eignen.

Versuche belegen, dass die folgenden Kombinationen von konkreten Substanzen sich als Liganden für das steuerbare Katalysatorsystem der Hydroformylierung hervorragend eignen:

a) Substanz L2 als erster Monophosphit-Ligand (Basisligand) und Substanz L3 als zweiter Monophosphit-Ligand (Steuerligand);
b) Substanz L2 als erster Monophosphit-Ligand (Basisligand) und Substanz L1 als zweiter Monophosphit-Ligand (Steuerligand);
c) Substanz L2 als erster Monophosphit-Ligand (Basisligand) und Substanz L4 als zweiter Monophosphit-Ligand (Steuerligand);

Wobei L1, L2, L3 und L4 wie folgt definiert sind:

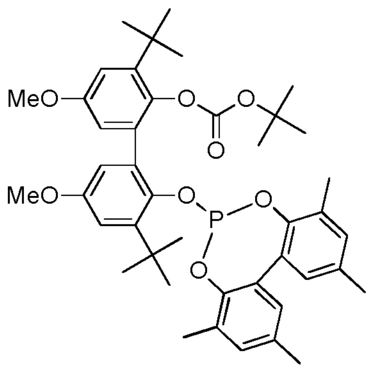

L1

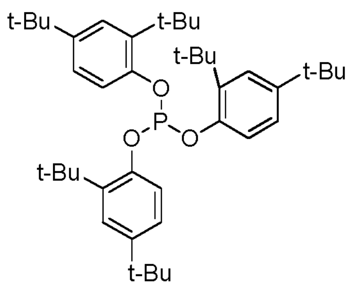

L2

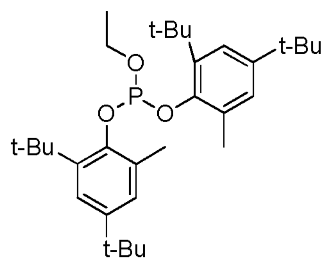

L3

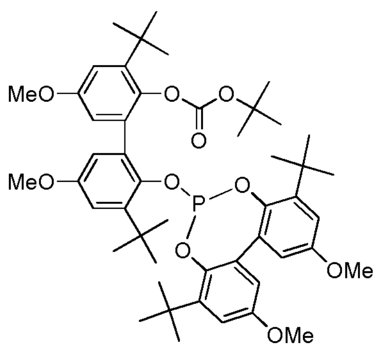

L4

(L2) Tris-(2,4-di-*tert*-butylphenyl)phosphit;

(L1) *tert*-Butyl-(3,3'-di-*tert*-butyl-5,5'-dimethoxy-2'-((2,4,8,10-tetramethyldibenzo[d,f][1,3,2]dioxaphosphepin-6-yl)oxy)-[1,1'-biphenyl]-2-yl)carbonat;

(L3) Bis-(4,6-di-*tert*-butyl-2-methylphenyl)ethylphosphit;

(L4) *tert*-Butyl (3,3'-di-*tert*-butyl-2'-((4,8-di-*tert*-butyl-2,10-dimethoxydi-benzo[d,f][1,3,2]dioxaphosphepin-6-yl)oxy)-5,5'-dimethoxy-[1,1'-biphenyl]-2-yl)carbonat

Wie die Experimente zeigen, vermögen die Steuerliganden L1, L3 und L4 die von dem Basisligand L2 ausgehende n/i-Selektivität stark zu beeinflussen.

Als besonders gut zur Steuerung geeignet hat sich hervorgetan eine Mischung aus Basisligand L2 und Steuerligand L1 oder L4.

Das erfindungsgemäße Verfahren zielt auf die Produktion von Oxo-Alkoholen ab, die für die Weiterverarbeitung zu Weichmacher-Estern bestimmt sind. Da die Qualität dieser Endprodukte in hohem Maße von der Qualität der Oxo-Alkohole abhängig ist, profitieren die Hersteller von Weichmachern stark von den Vorzügen dieses Verfahrens.

Dargestellt wird deswegen auch ein Verfahren zur Herstellung eines Estergemisches, insbesondere zu dessen Verwendung als Weichmacher für Polyvinylchlorid (PVC), bei welchem ein erfindungsgemäß hergestelltes Alkoholgemisch eingesetzt wird.

Der prinzipielle Prozessablauf und dessen Regelung werden nun anhand der Figur 1 erläutert.
Es zeigt

Figur 1: Vereinfachte schematische Darstellung eines Produktionsstranges zur Durchführung eines erfindungsgemäßen Verfahrens unter besonderer Berücksichtigung der Stoffströme und Regelkreise.

Von links wird ein Einsatzgemisch C4 aus einer nicht dargestellten unsteten Quelle angeliefert. Es enthält im Wesentlichen Kohlenwasserstoffe mit vier Kohlenstoffatomen, nämlich gesättigte Butane und ungesättigte Butene. Die Zusammensetzung des Einsatzgemisches C4 schwankt stark, etwa so, wie in Tabelle 1 niedergelegt.

In einer Oligomerisierung [1] werden die Butene oligomerisiert, sodass daraus im Wesentlichen $C_8$, $C_{12}$ und $C_{16}$ - Olefine entstehen. Aus diesem Oligomerisat werden die Dibutene $C_8$ abgetrennt (nicht dargestellt) und in eine Hydroformylierung [2] gefahren.

Dort wird das Dibuten $C_8$ mit Kohlenmonoxid CO und Wasserstoff $H_2$ zu einem Gemisch aus Aldehyden mit neun Kohlenstoffatomen INAL hydroformyliert.

Die Aldehyde INAL werden anschließend in einer Hydrierung [3] mit Wasserstoff $H_2$ zu den entsprechenden Alkoholen INA hydriert.

In der Praxis läuft ein Teil der Hydrierung als Folgereaktion bereits in dem Hydroformylierungsreaktor ab, da die in der Hydroformylierung [2] frisch gebildeten Aldehyde gleich mit dem vorhanden Wasserstoff $H_2$ zu den entsprechenden Alkoholen weiterregieren. Die Tatsache, dass das aus der Hydroformylierung [2] abgezogene Aldehydgemisch INAL bereits etwas INA enthält, ist aber für die Steuerung des Prozesses unerheblich.

Das INA wird anschließend in einer Veresterung [4] mit Phthalsäure bzw. Phthalsäureanhydrid PSA zu dem Estergemisch Diisononylphthalat DINP umgesetzt, welches als Weichmacher für Polyvinylchlorid verwendet wird. Die Veresterung [4] erfolgt in einem Batch-Prozess und ist nicht Teil des kontinuierlichen Verfahrens zur Herstellung der Oxo-Alkohole.

Um die schwankende Zusammensetzung des Einsatzgemisches $C_4$ auszugleichen und zu einem Alkoholgemisch INA mit einer wenig schwankenden Produktqualität zu gelangen, ist erfindungsgemäß eine Steuerung des Herstellprozesses vorgesehen. Ziel ist es insbesondere ein Estergemisch DINP mit einer Viskosität zu produzieren, die in einem engen Spezifikationsfenster schwankt. Da die Viskosität des Estergemisches DINP durch die Viskosität des Aldehydgemisches INAL determiniert wird, zielt das Regelkonzept darauf ab, die Viskosität der Aldehyde INAL möglichst konstant an einem vorgegebenen Wert zu halten.

Hierfür ist sowohl für die Oligomerisierung [1] als auch für die Hydroformylierung [2] jeweils ein Regler R1 bzw. R2 vorgesehen.

Für die Regelung R2 der Hydroformylierung [2] wird die Zusammensetzung comp des Aldehydgemisches INAL kontinuierlich analysiert. Dies geschieht durch Gaschromatographie. Der Gaschromatograph liefert die Molenbrüche der einzelnen isomeren Aldehyde in dem INAL an einen Rechner calc2, der daraus mit Hilfe der Formel (2) und der im Rechner hinterlegten Viskositätsbeiträge eine erste skalare Regelgröße visc errechnet, die einer Näherung der Viskosität des Estergemisches DINP bei Normalbedingungen entspricht. Gelingt es, visc in einem definierten Wertebereich konstant zu halten, wird auch das Estergemisch DINP eine nahezu konstante Viskosität aufweisen. Der Sollwert wird anhand

der Viskositätsspezifikation des gewünschten DINP-Weichmacherproduktes festgelegt. Dieser Wert wird dem Regler R2 als Sollwert vorgegeben. Es sei angemerkt, dass es sich bei dem Sollwert genauer gesagt um einen Sollbereich handelt, innerhalb dessen die Viskosität schwanken darf, ohne dass regelnd eingegriffen werden muss. Dieser Sollbereich kann beispielsweise bei 90 bis 95 mPas liegen, kann aber abhängig von den Kundenanforderungen an das Weichmacherprodukt auch vollkommen andere Werte annehmen.

Falls der Istwert der ersten skalaren Regelgröße visc von dem Sollwert abweicht respektive den Sollbereich verlässt, greift Regler R2 in die Hydroformylierung [2] ein, um die Sollviskosität wieder herzustellen bzw. die Viskosität in den Spezifikationsbereich zurückzuführen.

Der Eingriff erfolgt über Abänderung des Druckes p der Hydroformylierung [2], oder über Veränderung des Katalysatorsystems cat der Hydroformylierung [2] oder über beides gleichzeitig.

Der Druck der Hydroformylierung wird über das Synthesegas aufgeprägt. Die Druckregelung ist mithin eine Regelung des Druckes des Synthesegases.

Steigt die Viskosität über den Sollwert an so kann dem regelnd entgegengewirkt werden, indem der Synthesegasdruck gesenkt wird und/oder Steuerligand zudosiert wird.

Die Anpassung der Regelgröße hängt dabei von dem verwendeten Steuerliganden ab. Für den möglichen Steuerliganden tert-Butyl-(3,3'-di-tert-butyl-5,5'-dimethoxy-2'-((2,4,8,10-tetramethyldibenzo[d,f][1,3,2]dioxaphosphepin-6-yl)oxy)-[1,1'-biphenyl]-2-yl)carbonat (L1) in Mischungen mit Basisligand Tris-(2,4-di-tert-butylphenyl)phosphit(L2) wurden experimentell bestimmte Abhängigkeiten ermittelt und in Beispiel 1 dargestellt.

Zur Verdeutlichung seien hier die beispielhaft bestimmten Regelparameter vorweggenommen:

Bei Verwendung dieser beiden potentiellen Liganden führt eine Erhöhung des Anteils des Steuerliganden L1 um 10 Prozentpunkte zu einer Verringerung der berechneten Viskosität um 0,87 mPas bei konstantem Einsatzstoff und konstanten Reaktionsbedingungen in der Hydroformylierung.

Die Abhängigkeit der Viskositätskenngröße visc von dem Mischungsverhältnis der eingesetzten Liganden muss für jede Ligandenmischung einmalig experimentell bestimmt werden.

So wird in Beispiel 2 eine analoge Ableitung für den Einsatz einer Mischung aus Bis-(4,6-di-tert-butyl-2-methylphenyl)ethylphosphit (L3) und L2 offenbart. Dort muss der Anteil an L3 in der Mischung beispielsweise um etwa 20 Prozentpunkte angehoben werden, um eine Erhöhung der berechneten Viskosität um 1 mPas zu erzielen. Die Werte gelten entsprechend, wenn die Viskosität unter den Sollwert sinken sollte.

Gemäß der Erfindung kann die Regelung der Hydroformylierung auch über den (absoluten) Reaktionsdruck erfolgen. In Beispiel 3 wird offenbart, wie die Abhängigkeit der Regelgröße visc vom Druck herzuleiten ist.

Auch hier ist zu berücksichtigen, dass die Druckabhängigkeit vom verwendeten Katalysatorsystem (respektive Ligandensystem) abhängen kann, weshalb sie im jeweiligen Einzelfall einmalig experimentell zu bestimmen ist. Für den Liganden L1 ergibt sich, dass eine Erhöhung des Druckes um etwa 6 bar die Regelgröße visc um 0,5 mPas steigert. Für eine Absenkung gilt entsprechendes.

Durch die prozesstechnische Auslegung der Anlagen ist die mögliche Variation des Druckes natürlich begrenzt, weshalb die Steuerung bevorzugt über die Ligandenmischung erfolgt.

Die Steuerung des Hydroformylierungssystems wird in Beispiel 4 veranschaulicht.

Die Regelung der zudosierten Menge Steuerligand bzw. der Druckänderung erfolgt mit PID-Charakteristik.

Die Regelung der Oligomerisierung [1] erfolgt über einen Regler R1 ebenfalls mit PID-Charakteristik. Dieser dient zur Konstanthaltung einer zweiten skalaren Regelgröße iso im Bereich eines voreingestellten Sollwertes.

Bei der zweiten skalaren Regelgröße iso handelt es sich um den mit der Zahl 100 multiplizierten Isoindex des aus dem Oligomerisat abgetrennten Dibutens $C_8$. Der Faktor 100 hat auf die Regelung keinen Einfluss sondern erleichtert das Ablesen und Einstellen. Zur Bestimmung des Isoindex wird das Dibuten $C_8$ hinsichtlich seiner Zusammensetzung mittels hydrierender Gaschromatographie kontinuierlich analysiert. Aus dem Gewichtsanteil der Methylheptene und dem Gewichtsanteil der Dimethylhexene errechnet ein Rechner calc1 mit Formel (1) den Isoindex des Dibutens $C_8$ und durch Multiplikation dieses Wertes mit 100 die skalare Regelgröße iso.

Um den Isoindex des produzierten Dibutens konstant in einem für die Hydroformylierung optimalen Bereich zu halten, greift Regler R1 auf die Oligomerisierung [1] ein, indem er deren Temperatur T und/oder deren Umsatz conv anpasst. Das Heben und Senken der Reaktionstemperatur T kann erfolgen durch Einstellen der Kühlwassermenge des Oligomerisierungsreaktors. Ebenso kann das Heben und Senken der Temperatur T durch Einstellen der Temperatur des eingespeisten Wärmeträgers erfolgen. Das Steuern des Umsatzes conv erfolgt durch Verändern der Rückführmenge unumgesetzter Butene in die in Kreislauffahrweise betriebene Oligomerisierung.

Die Bestimmung der Regelabhängigkeiten wurde hier ebenfalls experimentell durchgeführt und das Vorgehen in Beispiel 5 offenbart. Dort wird ersichtlich, dass die Abhängigkeiten zwischen dem Isoindex, Umsatz und den Steuerparametern Temperatur und Rücklaufverhältnis etwas komplexer sind. Die aus dem Beispiel abgeleiteten Abhängigkeiten sind in den beiden Formeln (3) und (4) wiedergegeben:

$$iso = 113{,}814 - 0{,}360474 \cdot T - 4{,}36236 \cdot R - 0{,}0618174 \cdot w(\text{1-B}) + 0{,}00518875 \cdot T^2 + 0{,}0364209 \cdot w^2(\text{1-B}) - 0{,}00545420 \cdot T \cdot w(\text{1-B}) + 0{,}174800 \cdot R^2 \quad (3)$$

$$conv = -181{,}465 + 7{,}52134 \cdot T + 237{,}263 \cdot R - 1{,}70002 \cdot w(\text{1-B}) - 0{,}0510611 \cdot T^2 - 4{,}39695 \cdot T \cdot R + 0{,}0272597 \cdot T \cdot w(\text{1-B}) + 1{,}08140 \cdot R \cdot w(\text{1-B}) \quad (4)$$

wobei iso und conv die in Figur 1 angegebenen Regelgrößen sind. T steht für die Temperatur (in Grad Celsius) des Wärmeträgers mit dem der Oligomerisierungsreaktor temperiert wird, R gibt die Rückführmenge an unumgesetzten Butenen (in den vorliegenden experimentellen Beispielen angegeben in kg/h) an und w(1-B) den Massenanteil an 1-Buten in dem eingesetzten C4-Kohlenwasserstoffstrom.

Aus diesen Abhängigkeiten kann mit Hilfe eines Rechners eine Regelung implementiert werden, die auf Änderungen der jeweiligen Größen reagiert:

Ist der Isoindex zu hoch, sollte in dieser Ausführungsform der Erfindung die Temperatur der Oligomerisierungsreaktion abgesenkt werden. Der damit einhergehende Umsatzverlust kann durch eine geeignete Erhöhung der Rückführmenge ausgeglichen werden.

Ist der Isoindex indes so gering, dass ohne weiteres eine Erhöhung der Temperatur im Sinne der einzuhaltenden Spezifikation vertretbar ist, kann die Temperatur der Oligomerisierung erhöht werden. Dies hat im Regelfall eine Steigerung des Umsatzes zur Folge, die üblicherweise auch gewünscht ist. Durch Reduzierung des Rückführungsverhältnisses kann der Umsatz aber auch wieder reduziert werden um eine unerwünschte Überproduktion zu verhindern. Ein Beispiel für eine solche Regelung wird in Beispiel 6 angeführt.

In der in Figur 1 dargestellten Ausführungsform der Erfindung berücksichtigt Regler R1 bei der Einstellung von Temperatur und/oder Umsatz der Oligomerisierung [1] neben dem zweiten skalaren Regelwert iso auch den ersten skalaren Regelwert visc, der hinter der Hydroformylierung [2] gewonnen wird:

Da der Isoindex indirekt maßgeblichen Einfluss auf die Viskosität des Weichmachers hat, gelten zur Steuerung der Viskosität im Reaktionssystem der Oligomerisierung die gleichen Reglungsweisen wie für die Regelung des Isoindexes selbst. Allerdings ist für die Steuerung der Oligomerisierung mittels der Viskosität eine einmalige experimentelle Bestimmung der Abhängigkeiten nötig. Hierfür werden die Ergebnisse aus den Beispielen 1 und 5 kombiniert. Hier sei beispielhaft die Abhängigkeit für eine Kombination aus Oligomerisierung und Hydroformylierung gezeigt, in der das Ligandensystem der Hydroformylierung gebildet wird aus L1 und L2:

$$visc = \{-4{,}1003453 \cdot \ln[x(\text{L1})] + 65{,}201179\} \cdot \{[113{,}814 - 0{,}360474 \cdot T - 4{,}36236 \cdot R - 0{,}0618174 \cdot w(\text{1-B}) + 0{,}00518875 \cdot T^2 + 0{,}0364209 \cdot w^2(\text{1-B}) - 0{,}00545420 \cdot T \cdot w(\text{1-B}) + 0{,}174800 \cdot R^2]/100\} + \{3{,}4837009 \cdot \ln[x(\text{L1})] + 21{,}154949\} \quad (5)$$

wobei visc die in Figur 1 angegebene Regelgröße ist. T steht für die Temperatur (in Grad Celsius) des Wärmeträgers, mit dem der Oligomerisierungsreaktor temperiert wird, R gibt die Rückführmenge an unumgesetzten Butenen in die Oligomerisierung (hier in kg/h) an und w(1-B) den Massenanteil an 1-Buten in dem für die Oligomerisierung eingesetzten C4-Kohlenwasserstoffstrom. x(L1) steht schließlich für den Stoffmengenanteil des Steuerliganden L1 in der Ligandenmischung von L1 und L2 (in mol-%). In steht für den Logarithmus naturalis.

Ist die Viskosität des Weichmachers über Soll, sollte in der vorliegenden Ausführungsform der Erfindung die Temperatur der Oligomerisierungsreaktion abgesenkt werden. Der mit der Absenkung der Temperatur einhergehende Umsatzverlust kann durch eine geeignete Erhöhung der Rückführmenge ausgeglichen werden.

Ist die Viskosität des Weichmachers indes so gering, dass ohne weiteres eine Erhöhung der Temperatur im Sinne der einzuhaltenden Spezifikation vertretbar ist, kann die Temperatur der Oligomerisierung erhöht werden. Dies hat im Regelfall eine Steigerung des Umsatzes zur Folge, die üblicherweise auch gewünscht ist.

Ein Beispiel für die Steuerung der Oligomerisierung mit Hilfe der Regelgröße visc und die gemeinschaftliche Steuerung der Hydroformylierung und Oligomerisierung als Gesamtprozess wird als Beispiel 7 beschrieben.

**Beispiele**

Arbeitsvorschrift für die Beispiele 1 bis 3

In einem 100 ml-Autoklaven der Fa. Parr Instruments wurde bei 130 - 140 °C, 30 oder 50 bar Synthesegasdruck ($CO/H_2$ = 1:1 (Vol-%)) 6 g der Octengemische aus Tabelle 1 hydroformyliert. Als Katalysatorvorstufe wurden bei einer Katalysatorkonzentration von 40 ppm Rh bezogen auf das gesamte Reaktionsgemisch 0,005 g $Rh(acac)(CO)_2$ vorgelegt, bei einer Konzentration von 100 ppm Rh entsprechend 0,0123 g $Rh(acac)(CO)_2$. Als Lösemittel wurden jeweils 40 bis 46 g Toluol verwendet. Der Ligand L1 bzw. der Ligand L2 bzw. der Ligand L3 bzw. der Ligand L4 bzw. die Ligandenmischung bestehend aus den Liganden L1 und L2 bzw. aus den Liganden L2 und L3 bzw. aus den Liganden L2 und L4 wurde in 20-fachem molaren Überschuss relativ zum Rhodium verwendet (Verhältnis Gesamtphosphor zu Rhodium). Zusätzlich wurden als GC-Standard ca. 0.5 g Tetraisopropylbenzol (TIPB) zugefügt. Ca. 6 g Edukt wurden nach Erreichen der vorgesehenen Reaktionstemperatur zudosiert.

Während der Reaktion wurde der Druck über eine Synthesegasregelung mit Massedurchflussmesser konstant gehalten. Die Rührerdrehzahl betrug 1200 $min^{-1}$. Proben wurden aus der Reaktionsmischung nach 180 Minuten gezogen.

In den Katalyseversuchen werden für die Liganden die Abkürzungen L1 bis L4 verwendet:

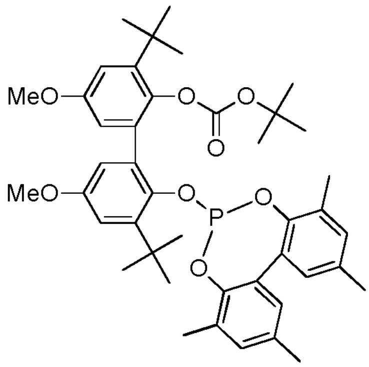

L1

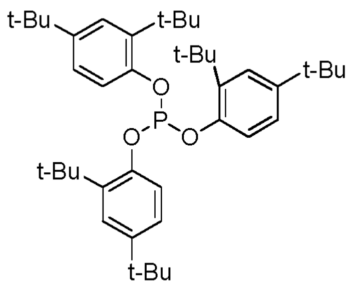

L2

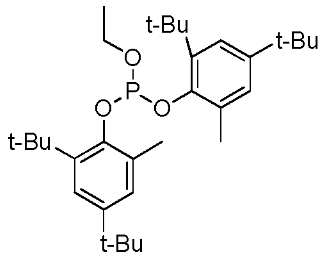

L3

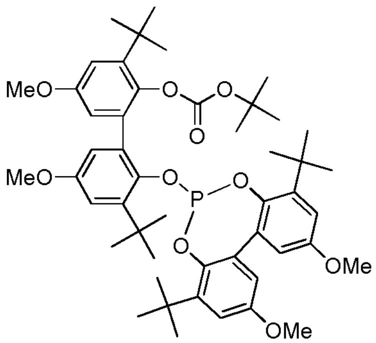

L4

Die Herstellung der Liganden L1, L3 und L4 wird im nachfolgenden experimentellen Teil beschrieben. Der Ligand L2 (TDTBPP bzw. Alkanox 240) ist kommerziell erhältlich.

Allgemeine Arbeitsvorschriften zur Herstellung der Liganden

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego, Christina Chai, Butterworth Heinemann (Elsevier), 6. Auflage, Oxford 2009).

Alle präparativen Arbeiten erfolgten in ausgeheizten Gefäßen. Die Charakterisierung der Produkte erfolgte mittels NMR-Spektroskopie. Chemische Verschiebungen ($\delta$) werden in ppm angegeben. Die Referenzierung der $^{31}P$-NMR-Signale erfolgte gemäß: $SR_{31P} = SR_{1H} * (BF_{31P} / BF_{1H}) = SR_{1H} * 0{,}4048$. (Robin K. Harris, Edwin D. Becker, Sonia M. Cabral de Menezes, Robin Goodfellow, and Pierre Granger, Pure Appl. Chem., 2001, 73, 1795-1818; Robin K. Harris,

Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte mittels eines Bruker Avance 300 bzw. Bruker Avance 400, die gaschromatographische Analyse an Agilent GC 7890A.

Reaktionsschema:

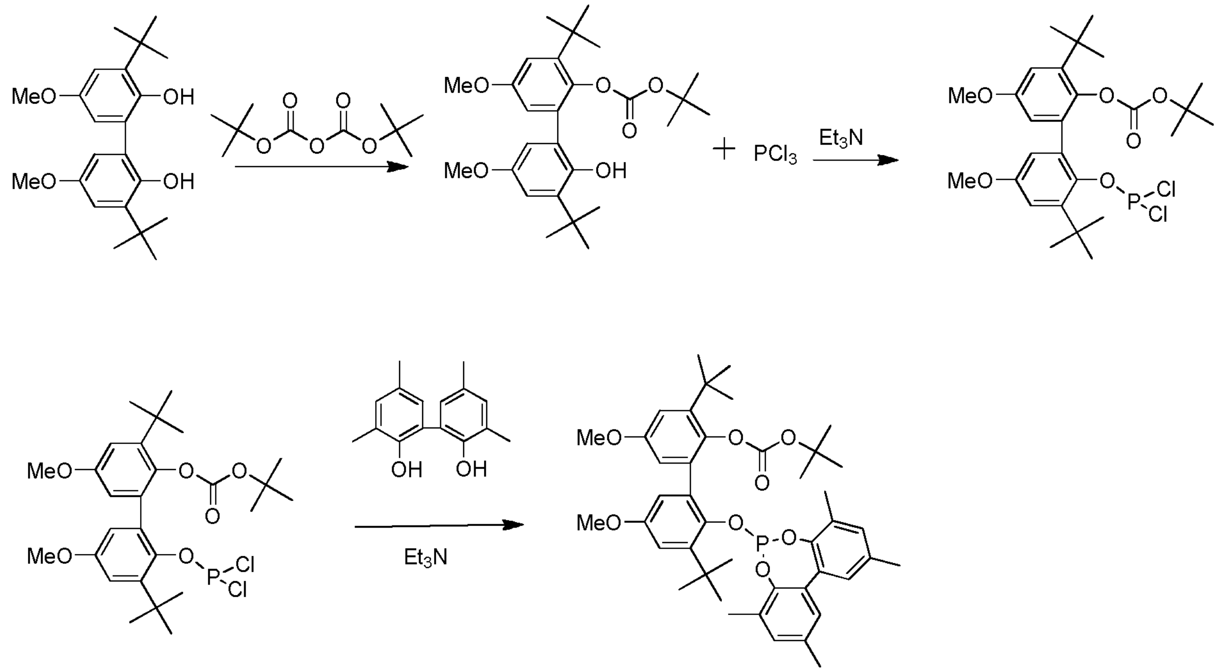


Einführung der BOC-Gruppe:

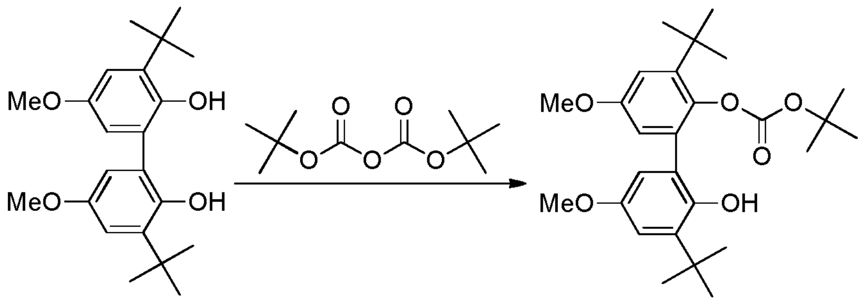

In einem 2L Schlenkkolben werden 400 mmol (143,8 g) des 3,3'-Di-tert.-butyl-5,5'-dimethoxy-[1,1'-biphenyl]-2,2'diol und 40 mmol (4,8 g) N,N-Dimethylaminopyridin (DMAP) in 900 mL $CH_2Cl_2$ gelöst. Anschließend wurden bei Raumtemperatur 400 mmol (88 g) Di-tert-butyldicarbonat in 280 ml $CH_2Cl_2$ gelöst, in einen 500 ml Tropftrichter überführt und innerhalb einer Stunde bei 32 °C zu der Biphenol/DMAP Lösung getropft. Die Lösung wurde über Nacht bei Raumtemperatur gerührt. Am nächsten Morgen wird das Lösungsmittel unter vermindertem Druck entfernt. Der leicht wachsartige, rötliche Rückstand wurde mit 800 ml n-Heptan versetzt und über Nacht gerührt. Dabei erhielt man einen weißen Rückstand, der abfiltriert, zweimal mit 50 ml n-Heptan nachgewaschen und dann getrocknet wurde. Das Zielprodukt konnte als weißer Feststoff (161,6 g, 84%) erhalten werden. $^1H$-NMR (Tolul-$d_8$): 95% und weitere Verunreinigungen.

Umsetzung von tert-Butyl-(3,3'-di-tert-butyl-2'-hydroxy-5,5'-dimethoxy-[1,1'-biphenyl]2-yl)-carbonat mit Phosphortrichlorid:

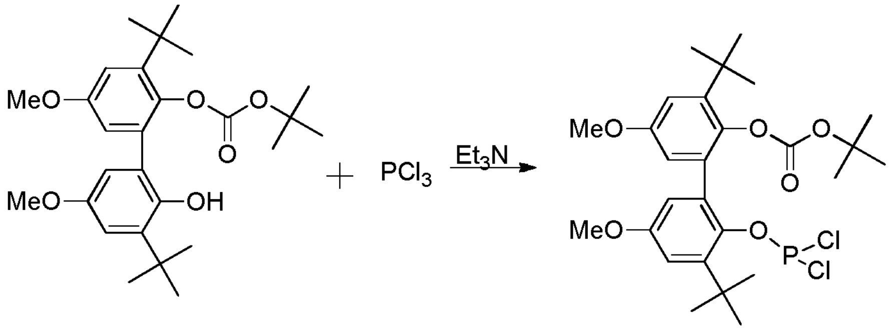


In einem sekurierten 250 ml Schlenkkolben wurden 12 g (0,026 mol) tert-Butyl-(3,3'-di-tert-butyl-2'-hydroxy-5,5'-dimethoxy-[1,1'-biphenyl]-2-yl)-carbonat unter Rühren in 120 ml getrocknetem Toluol und 12,8 ml (0,091 mol) Triethylamin gelöst.

In einem zweiten 500 ml Schlenkkolben wurden zunächst 100 ml getrocknetes Toluol mit 8,1 ml (0,091 mol) Phosphortrichlorid verrührt. Anschließend wurde die Phosphortrichlorid-Toluol-Lösung zu der zuvor hergestellten Carbonat-Amin-Toluol-Lösung innerhalb von 30 Minuten bei Raumtemperatur zugetropft. Nach vollständiger Zugabe wurde für 30 Minuten auf 80 °C erwärmt und über Nacht auf Raumtemperatur abgekühlt.

Am nächsten Morgen wurde die Mischung filtriert, mit 50 ml getrocknetem Toluol nachgewaschen und das Filtrat bis zur Trockne eingeengt. Das Zielprodukt konnte als Feststoff (13,1 g, 89%) erhalten werden. $^{31}$P-NMR (202,4 MHz, Toluol-$d_8$): 203,2 und 203,3 ppm (100%).

Umsetzung von tert-Butyl-(3,3'-di-tert-butyl-2'-((dichlorophosphino)oxy)-5,5'-dimethoxy-[1,1'-biphenyl]-2-yl)-carbonat mit 3,3',5,5'-Tetramethyl-(1,1'-biphenyl)-2,2'-diol (L1)

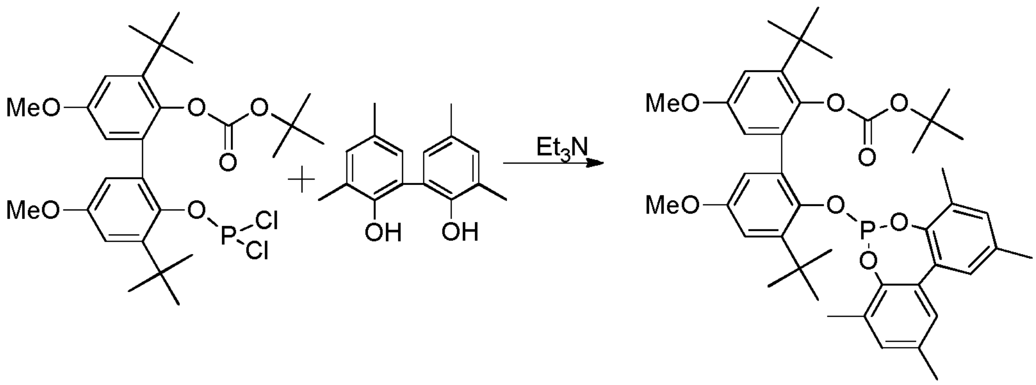


In einem sekurierten 1 L Schlenkkolben wurden 24,7 g (0,044 mol) tert-Butyl-(3,3'-di-tert-butyl-2'-((dichlorophosphino)oxy)-5,5'-dimethoxy-[1,1'-biphenyl]-2-yl)-carbonat in 400 ml Acetonitril gelöst.

In einem zweiten sekurierten Schlenkkolben (1 L) wurden 10,8 g (0,044 mol) 3,3',5,5'-Tetramethyl-(1,1'-biphenyl)-2,2'-diol in 200 ml Acetonitril und 13,1 ml (0,011 mol) getrocknetem Triethylamin unter Rühren gelöst. Anschließend wurde zu der Biphenol-Triethylamin-Lösung langsam die Chlorophosphit-Lösung hinzugetropft und über Nacht gerührt.

Der Ansatz wurde daraufhin filtriert und der Rückstand zweimal mit 15 ml Acetonitril gewaschen.

Das Filtrat wurde unter vermindertem Druck eingeengt bis ein Niederschlag ausfiel. Dieser wurde filtriert und getrocknet. Das Zielprodukt konnte als weißer Feststoff (28,5 g, 87%) erhalten werden. $^{31}$P-NMR (202,4 MHz,Toluol-$d_8$): 139,4 ppm (98,5%).

Umsetzung von tert-Butyl-(3,3'-di-tert-butyl-2'-((dichlorophosphino)oxy)-5,5'-dimethox-[1,1'-biphenyl]-2-yl)-carbonat mit 3,3-Di-tert.butyl-5.5-dimethoxy-biphenol (L4)

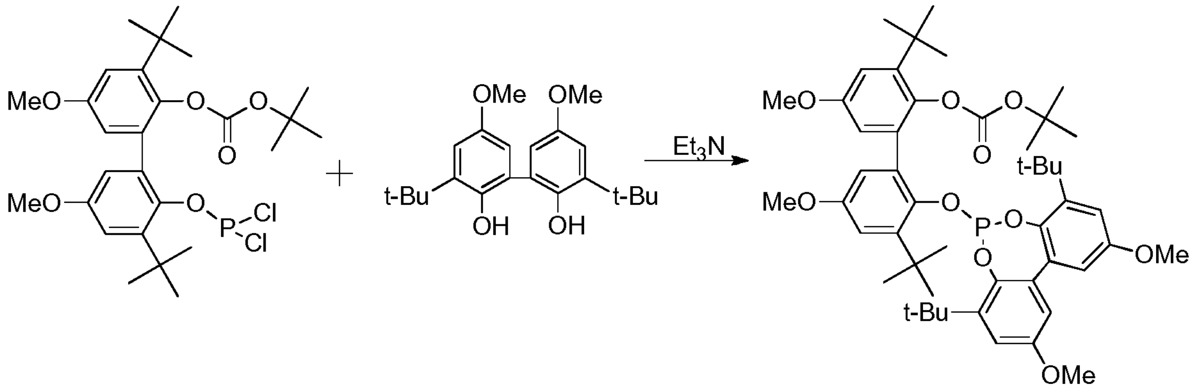


In einem sekurierten 250 ml Schlenkkolben wurden 7 g (0,0125 mol) tert-Butyl-(3,3'-di-tert-butyl-2'-((dichlorophosphino)oxy)-5,5'-dimethoxy-[1,1'-biphenyl]-2-yl)-carbonat in 100 ml getrocknetem Acetonitril gelöst.

In einem zweiten sekurierten Schlenkkolben (100 ml) wurden 4.5 g (0,0125 mol) 3,3-Di-tert.-butyl-5,5-dimethoxy-biphenol in 60 ml getrocknetem Acetonitril und 4.2 ml (0,03 mol) entgastem Triethylamin gelöst. Im Anschluss wurde die Biphenol-Triethylamin-Lösung langsam bei Raumtemperatur zu der Chlorophosphit-Lösung getropft und über Nacht bei Raumtemperatur gerührt.

Ein Teil des Lösungsmittels wurde unter vermindertem Druck entfernt. Der ausgefallene Feststoff wurde filtriert und getrocknet. Das Zielprodukt konnte als weißer Feststoff (10,5 g, 96%) erhalten werden. $^{31}$P-NMR (202,4 MHz, Toluol-$d_8$): 140,9 (95,2%) und weitere Verunreinigungen (weitere Verunreinigungen = P-H-Verbindungen, Oxidverbindungen, noch nicht vollständig umgesetztes Chlorophosphit).

Herstellung von Bis(2,4-di-tert.-butyl-6-methylphenyl)ethylphosphit (L3)

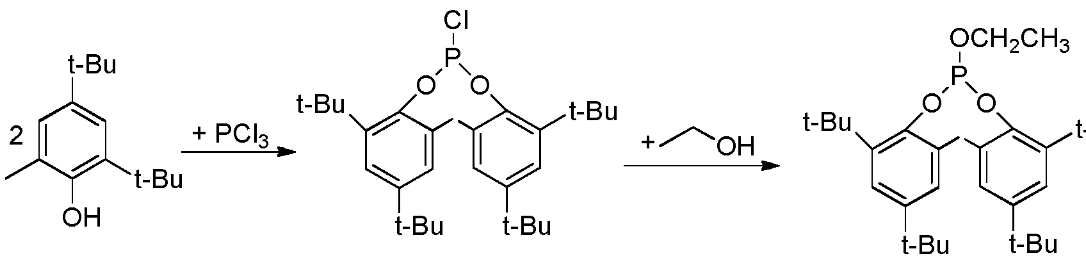


In einem 250 ml Schlenkkolben mit Magnetrührer, Aufsatz, Tropftrichter und Rückflusskühler wurden 22.5 g (0,1 mol) 2,4-Di-tert.-butyl-6-methylphenol (4,6-Di-tert-butyl-ortho-cresol) vorgelegt und zum Schmelzen des Phenols auf 55 °C erwärmt. Zur Schmelze wurden 0,13 ml (0,0015 mol) getrocknetes, entgastes Dimethylformamid zugegeben. Anschließend wurde innerhalb von 2 Stunden 5,7 ml (0,065 mol) Phosphortrichlorid zugetropft. Nach beendeter Zugabe wurde die Reaktionsmischung innerhalb 3 Stunden auf 140 °C erhitzt und 1 Stunden bei dieser Temperatur gerührt. Dann wurde unter Vakuum 1 Stunden bei 130 °C gerührt. Danach wurde die erhaltene klare gelb-orange Schmelze (=Bis(2,4-di-tert-butyl-6-methyl)-phosphorchloridit) über Nacht auf 80 °C abgekühlt und mit 75 ml entgastem Benzin (80-110 °C) verdünnt. Nach dem Abkühlen der Lösung auf - 5 °C wurden innerhalb von 15 Minuten 9.1 ml (0,0665 mol) entgastes Triethylamin zugegeben. Anschließend wurde innerhalb von 2 Stunden 4.4 mL (0,075 mol) getrocknetes und entgastes Ethanol zu getropft, wobei die Temperatur nicht über 5 °C anstieg. Dieses Gemisch wurde unter Rühren langsam über Nacht auf Raumtemperatur erwärmt.

Am nächsten Morgen wurde das ausgefallene Triethylaminhydrochlorid abfiltriert und das Filtrat unter vermindertem Druck eingeengt. Es entstand ein weißer Rückstand, der in 60 mL entgastem Ethanol umkristallisiert wurde. Das Produkt konnte so in 73,9%iger (19,03g) Ausbeute als weißer Feststoff in 98%iger Reinheit laut LC-MS erhalten

werden.

**Beispiel 1**

Dieses Beispiel offenbart die Bestimmung der Abhängigkeit der Regelgröße visc vom Mischungsverhältnis der Ligandenmischung. Diese wird analog EP 1430014 B1 aus der Isomerenverteilung der Produktaldehyde der $C_8$-Hydroformylierung berechnet und spiegelt eine angenäherte Viskosität einer Di-iso-nonylphthalat-Mischung wieder, die aus diesen Aldehyden durch Hydrierung und anschließende Veresterung der so erhaltenen Alkohole mit Phthalsäure hergestellt werden können.

Für die Bestimmung der Abhängigkeit wurden mehrere Hydroformylierungsexperimente nach der oben angegebenen Arbeitsvorschrift durchgeführt, wobei Di-n-Buten (DnB) mit unterschiedlichem Verzweigungsgrad verwendet wurde. Druck 50 bar, Temperatur 140 °C, Reaktionszeit 180 Minuten. Die Ergebnisse zeigt Tabelle 3.

Tabelle 3: Berechnungen zu Beispiel 1

| Nr | Isoindex DnB | P/Rh Ligand L1 | P/Rh Ligand L2 | Anteil L1 [mol-%] | visc [mPas] |
|---|---|---|---|---|---|
| 1 | 1,038 | 19,9 | 0 | 100 | 82,6 |
| 2 | 1,038 | 4,9 | 14,9 | 25 | 88,6 |
| 3 | 1,038 | 0 | 19,8 | 0 | 89,5 |
| 4 | 1,110 | 20,0 | 0 | 100 | 86,6 |
| 5 | 1,110 | 1,9 | 17,1 | 10 | 93,2 |
| 6 | 1,110 | 0 | 19,9 | 0 | 95,6 |
| 7 | 1,151 | 20,3 | 0 | 100 | 87,4 |
| 8 | 1,151 | 5,1 | 15,3 | 25 | 93,0 |
| 9 | 1,151 | 2,0 | 18,1 | 10 | 94,3 |

Die Ergebnisse des Versuchs sind in Figur 2 graphisch dargestellt:

Figur 2: Aus der Aldhehydverteilung von Hydroformylierungsausträgen berechnete Viskosität eines DINP als Regelgröße visc, aufgetragen über den Stoffmengenanteil des Liganden L1 in einer Mischung aus L1 und L2 für verschiedene Olefinqualitäten spezifiziert über den Isoindex.

Aus der Steigung der in der Auftragung dargestellten Regressionsgeraden ergibt sich die Regelvorschrift für die Regelung der Hydroformylierung mit Hilfe der Ligandenmischung. Wie Figur 2 zu entnehmen ist, unterscheiden sich die Geradensteigungen bei der Oxierung von Din-Buten-Gemischen mit unterschiedlichem Isoindex nur wenig, weshalb hier in guter Näherung der Mittelwert der Geradensteigungen für die Regelung verwendet werden kann. Sie beträgt $a_1$ = -0,087 mPas/%. Das heißt, bei Verwendung dieser beiden Liganden für die Mischung führt eine Erhöhung des Anteils des Steuerliganden L1 um 10 Prozentpunkte eine Verringerung der berechneten Viskosität um 0,87 mPas bei konstantem Einsatzstoff und konstanten Reaktionsbedingungen in der Hydroformylierung. Reicht diese Näherung für die Regelung nicht aus, muss die Abhängigkeit zwischen visc, der Ligandenmischung und dem Isoindex des verwendeten Di-n-Butens mathematisch beschrieben werden. Hierfür wurden die Daten in Figur 3 erneut aufgetragen.

Figur 3: Aus der Aldehydverteilung von Hydroformylierungsausträgen berechnete Viskosität eines DINP als Regelgröße visc, aufgetragen über den Isoindex des eingesetzten Di-n-Butens bei verschiedenen Mischungsverhältnissen.

Für die dargestellten Regressionsgeraden kann man Steigungen und Achsenabschnitte berechnen, die Tabelle 4 zusammengefasst wurden:

Tabelle 4: Steigungen aus der Grafik in Figur 3

| Anteil L1 [mol-%] | Natürlicher Logarithmus des Anteils L1 | Steigung der Regressionsgeraden | Achsenabschnitt der Regressionsgeraden |
|---|---|---|---|
| 100 | 4,6051702 | 43,964551 | 37,186982 |

(fortgesetzt)

| Anteil L1 [mol-%] | Natürlicher Logarithmus des Anteils L1 | Steigung der Regressionsgeraden | Achsenabschnitt der Regressionsgeraden |
|---|---|---|---|
| 25 | 3,2188758 | 55,003774 | 31,124070 |
| 10 | 2,3025851 | 55,692318 | 30,685065 |
| 0,1 | -2,3025851 | 74,062781 | 12,880316 |

Trägt man die Steigungen und die Achsenabschnitte über den natürlichen Logarithmus des L1-Anteils auf, kann man erneut eine lineare Regression durchführen, wie sie in Figur 4 dargestellt ist.

Figur 4: Auftragung der Steigungen und Achsenabschnitte der Regressionsgeraden aus Figur 3 über den natürlichen Logarithmus des L1-Anteils in der Ligandenmischung.

Für die beiden Auftragungen erhält man erneut je eine Steigung und einen Achsenabschnitt gemäß Tabelle 5:

Tabelle 5: Steigungen und Achsenabschnitte aus der Grafik in Figur 4

| | Steigung der Regressionsgeraden | Achsenabschnitt der Regressionsgeraden |
|---|---|---|
| Auftragung der Steigungen aus Fig. 3 | -4,1003453 | 3,4837009 |
| Auftragung der Achsenabschnitte aus Fig. 3 | 65,201179 | 21,154949 |

Einsetzen der Geradengleichungen ineinander führt zu folgender Abhängigkeit der Regelgröße visc vom Isoindex iso des eingesetzten Di-n-Butens gemäß Gleichung (6):

$$visc = \{-4{,}1003453 \cdot \ln[x(\mathrm{L1})] + 65{,}201179\} \cdot \left[\frac{iso}{100}\right] + \{3{,}4837009 \cdot \ln[x(\mathrm{L1})] + 21{,}154949\} \qquad (6)$$

wobei x(L1) hier der Stoffmengenanteil des Liganden L1 in der Mischung ist. iso ist die Regelgröße der Oligomerisierung die sich aus dem Isoindex des Dibutens berechnet nach Gleichung (7):

$$iso = \mathrm{Isoindex} \cdot 100 \qquad (7)$$

Diese Gleichung kann von einem Steuerungscomputer zur Regelung des Prozesses eingesetzt werden.

Beispiel 2

Da auch andere Steuerungsliganden als L1 technisch einsetzbar sind, wird in diesem Beispiel die Abhängigkeit bei Einsatz einer Mischung aus Bis-(4,6-di-tert-butyl-2-methylphenyl)ethylphosphit (L3) und L2, sowie einer Mischung aus L4 und L2 bestimmt. Hierzu wurden erneut Hydroformylierungsexperimente durchgeführt, deren wesentliche Ergebnisse Tabelle 6 entnommen werden können:

Tabelle 6: Berechnungen zu Beispiel 2

| Nr | Isoindex DnB | P/Rh Ligand L1 | P/Rh Ligand L2 | P/Rh Ligand L3 | P/Rh Ligand L4 | Anteil Steuerligand [mol-%] | visc [mPas] |
|---|---|---|---|---|---|---|---|
| 10 | 1,038 | 0 | 6,5 | 13,2 | 0 | 67 (L3) | 86,1 |
| 11 | 1,038 | 0 | 10,0 | 10,0 | 0 | 50 (L3) | 86,6 |

(fortgesetzt)

| Nr | Isoindex DnB | P/Rh Ligand L1 | P/Rh Ligand L2 | P/Rh Ligand L3 | P/Rh Ligand L4 | Anteil Steuerligand [mol-%] | visc [mPas] |
|---|---|---|---|---|---|---|---|
| 12 | 1,038 | 0 | 13,3 | 6,5 | 0 | 33 (L3) | 87,3 |
| 13 | 1,038 | 0 | 16,1 | 4,0 | 0 | 20 (L3) | 88,1 |
| 14 | 1,038 | 0 | 4,9 | 0 | 14,9 | 75 (L4) | 87,3 |
| 15 | 1,038 | 0 | 9,9 | 0 | 10,0 | 50 (L4) | 88,6 |
| 16 | 1,038 | 0 | 14,8 | 0 | 4,9 | 25 (L4) | 89,4 |
| 17 | 1,038 | 0 | 18,0 | 0 | 2,1 | 10 (L4) | 89,8 |

Analoge Auswertung dieser Daten ergibt, dass der Anteil an L3 in der Mischung mit L2 beispielsweise um etwa 11 Prozentpunkte angehoben werden muss um eine Verringerung der berechneten Viskosität (respektive der Regelgröße visc) um 0,5 mPas zu erzielen. Der Anteil von L4 in der Mischung mir L2 muss 13 Prozentpunkte angehoben werden, um eine Verringerung um 0,5 mPas zu bewirken. Die Werte gelten entsprechend, wenn die Viskosität erhöht werden soll.

**Beispiel 3**

In einer weiteren Ausführungsform der Erfindung kann die Regelung der Hydroformylierung auch über den absoluten Reaktionsdruck erfolgen. Hierzu wurde eine Reihe von Hydroformylierungsexperimenten bei verschiedenen Reaktionsdrücken durchgeführt. Die Temperatur und Reaktionszeit waren analog zu Beispiel 1 und 2. Die wesentlichen Ergebnisse sind in Tabelle 7 zusammengefasst:

Tabelle 7: Berechnungen zu Beispiel 3

| Nr. | Isoindex DnB | Reaktionsdruck [bara] | visc [mPas] |
|---|---|---|---|
| 18 | 1,038 | 50 | 79,4 |
| 19 | 1,038 | 100 | 85,8 |
| 20 | 1,038 | 150 | 89,2 |
| 21 | 1,038 | 200 | 93,2 |
| 22 | 1,038 | 235 | 95,4 |

Die Abhängigkeit der Regelgröße visc vom Reaktionsdruck ist in Figur 5 dargestellt.

Figur 5: Aus der Aldehydverteilung von Hydroformylierungsausträgen berechnete Viskosität eines DINP als Regelgröße visc, aufgetragen über den absoluten Reaktionsdruck der Hydroformylierungsreaktion, die in Gegenwart des Liganden L1 durchgeführt wurde.

Aus der Steigung der abgebildeten Regressionsgeraden lässt sich die Druckabhängigkeit in hinreichender Näherung ableiten. So führt eine Druckänderung um 6 bar zu einer Veränderung der Steuergröße visc um 0,5 mPas.

**Beispiel 4**

Dieses Beispiel soll die Steuerung eines Hydroformylierungsprozesses mit Hilfe der Regelgröße visc verdeutlichen. Hierzu wurde die Hydroformylierung eines Di-n-buten-Einsatzstoffes durchgeführt. Die Reaktionstemperatur wurde auf 140 °C eingestellt und der Reaktionsdruck auf 50 bar. Die Verweilzeit im Reaktor betrug etwa 180 Minuten. Durch Änderung der Massenanteile von Basis- und Steuerligand in der zugeführten Ligandenmischung konnte die Selektivität der Reaktion und damit die aus dem Aldehydaustrag berechnete DINP-Viskosität beeinflusst werden. Darüber hinaus wird gezeigt, wie sich die Selektivität der Hydroformylierung bei Schwankungen des Isoindex des eingesetzten Di-n-butens verhält und wie mit Hilfe der Regelgröße visc ein Aldehydprodukt erzeugt werden kann, dessen Zusammensetzung innerhalb definierter Grenzen konstant bleibt. In Tabelle 8 sind wichtige Betriebspunkte der Versuchskampagne zusammengestellt, welche die erfindungsgemäßen Effekte verdeutlichen:

Tabelle 8: Versuchskampagne aus Beispiel 4

| Nr. | Systemänderung oder Regelungsschritt | Isoindex DnB | Anteil Steuerligand [mol-%] | Ausbeute Aldehyd und Alkohol [%] | visc berechnet aus Versuchsprodukt [mPas] | visc aus Steuerungsmodell [mPas] |
|---|---|---|---|---|---|---|
| 1 | | 1,038 | 75 | 81,6 | 85,1 | |
| | visc < 88 mPas → Verringerung Anteil L1 auf 2% → nur noch L2 nachdosieren | | | | | 88,3 ± 1,5 |
| 2 | | 1,038 | 10 | 87,7 | 88,2 | |
| | visc im Sollbereich → aktuelles Ligandenverhältnis (10% L1) halten → Mischung dosieren 10 % L1 und 90 % L2 | | | | | 87,1 ± 1,5 |
| | Zulaufveränderungen in der Oligomerisierung → Erhöhung des Isoindex auf 1,151 | | | | | |
| 3 | | 1,151 | 10 | 90,2 | 94,3 | |
| | visc > 94 mPas → Erhöhung L1 auf 25 % → Dosierung von 50 % L1 und 50 % L2 | | | | | 92,2 ± 1,5 |
| 4 | | 1,151 | 25 | 88,7 | 93,0 | |
| | visc im Sollbereich → aktuelles Ligandenverhältnis (25% L1) halten → Mischung dosieren 25 % L1 und 75 % L2 | | | | | 92,2 ± 1,5 |

Arbeitsvorschrift für die Beispiele 5 bis 7

Die Oligomerisierung wurde in einem weitgehend isotherm betriebenen Rohreaktor mit folgenden Maßen nachgestellt: Länge 4.0 m, Innendurchmesser 32.8 mm. Der Reaktoren war mit einem Doppelmantel zur Thermostatisierung ausgerüstet. Als Wärmeträger diente das Produkt Marlotherm der Firma Sasol. Die Reaktion wurde bei einem Absolutdruck von 30 bar in der Flüssigphase durchgeführt. Als Einsatzstoff wurde ein Kohlenwasserstoffgemisch verwendet, enthaltend folgende, sich zu 100 Gew.-% ergänzende Komponenten:

1-Buten 0-20 Gew.-%

2-Buten 55-75 Gew.-%

Isobuten < 1 Gew.-%

Isobutan < 2Gew.-%

n-Butan > 24 Gew.-%

Als Katalysator wurde ein Material verwendet, welches gemäß Beispiel 1 der WO2011/000697A1 hergestellt und nach Beispiel 4 derselben Veröffentlichung nachbehandelt worden war und welches bereits seit 2000 Stunden im Einsatz war. Auf diese Weise wurde ein durchschnittlicher Betriebszustand der Oligomerisierung nachgestellt.

Hinter der Reaktionsstufe wurden die Oligomere von den Butanen und nicht umgesetzten Butenen abgetrennt und auf ihre Zusammensetzung analysiert. Dazu wurde zur Identifizierung der Octen-Skelettisomeren eine hydrierende GC-Analytik verwendet, bei der die oligomeren Olefine zunächst zu Alkanen hydriert werden. Die so erhaltenen Alkane werden dann gaschromatographisch getrennt und detektiert. Man kann zwischen drei relevanten C8-Isomeren differenzieren: n-Octan (gebildet aus n-Octenen), Methylheptan (gebildet aus Methylheptenen) und Dimethylhexan (gebildet aus Dimethylhexenen). Aus diesen Werten wurde der Isoindex und daraus wiederum die Regelgröße iso berechnet.

Ein Teil des Butane und nicht umgesetzte Butene enthaltenden Stromes wurde in den vorhergehenden Reaktor zurückgeführt. Der nicht rückgeführte Teil dieses Gemisches wurde entsorgt.

Um den betrachteten Gesamtprozess abzubilden, wurde in Abständen der Produktaustrag gesammelt (ca. 500g) und einer destillativen Trennung unterzogen um die gewünschte $C_8$-Fraktion von den verbliebenen leichter siedenden unumgesetzten Butenen und Butanen und den höher siedenden schwereren Oligomeren und Nebenprodukten zu trennen.

**Beispiel 5**

Für die Auslegung der Regelung ist eine einmalige experimentelle Bestimmung der Abhängigkeiten zwischen der Regelgröße iso und den Parametern Temperatur bzw. Rückführmenge nötig. Im Falle der Oligomerisierung wurde Bestimmung anhand eines statistischen Versuchsplanes durchgeführt, der mit Hilfe des Computerprogramms MiniTab aufgestellt worden war. Aus Basis dieses Versuchsplans wurden die notwendigen Parameteränderungen an der Versuchsanlage vorgenommen. Nach jeder Änderung wurde abgewartet, bis das Reaktionssystem sich wieder im stationären Zustand befand. Sodann wurde die Beprobung durchgeführt. Der Parameterraum für den Versuchsplan wurde von den technischen Gegebenheiten der Anlage begrenzt. So konnten Temperaturen nur zwischen 45 und 60 °C eingestellt werden, da bei tieferen Temperaturen die Reaktion zu langsam wird und es bei höheren Temperaturen zu einer schnellen Deaktivierung des Katalysators kommen kann. Die Menge an rückführbarem Buten/Butan-Strom kann von 0 bis 400 Gramm pro Stunde variiert werden. Die Zusammensetzung des Rohstoffstromes wurde willkürlich auf 0,6 bis 40 Massen-% 1-Buten festgelegt und die benötigten Zusammensetzungen gezielt angemischt.

Der Versuchsplan und die erhaltenen Ergebnisse sind in Tabelle 9 zusammengestellt.

Tabelle 9: Versuchsplan und Ergebnisse zu Beispiel 5

| StdRfolge | DIRfolge | Punkttyp | Blöcke | Temp. [°C] | Rücklauf [kg/h] | Konz. 1-Buten [Massen-%] | Umsatz [%] | S(C8) [%] | Anteil nO | Anteil MH | Anteil DMH | Isoindex |
|---|---|---|---|---|---|---|---|---|---|---|---|---|
| 15 | 1 | 0 | 1 | 52.5 | 0.20 | 20.3 | 73.6 | 81.5 | 0.150 | 0.654 | 0.196 | 1.046 |
| 10 | 2 | -1 | 1 | 60.0 | 0.20 | 20.3 | 83.4 | 79.0 | 0.150 | 0.645 | 0.204 | 1.054 |
| 1 | 3 | 1 | 1 | 48.0 | 0.08 | 8.6 | 61.4 | 81.5 | 0.146 | 0.648 | 0.206 | 1.059 |
| 20 | 4 | 0 | 1 | 52.5 | 0.20 | 20.3 | 73.6 | 81.5 | 0.150 | 0.654 | 0.196 | 1.046 |
| 3 | 5 | 1 | 1 | 48.0 | 0.32 | 8.6 | 67.8 | 84.3 | 0.145 | 0.656 | 0.199 | 1.054 |
| 14 | 6 | -1 | 1 | 52.5 | 0.20 | 40.0 | 74.4 | 81.3 | 0.151 | 0.661 | 0.187 | 1.036 |
| 6 | 7 | 1 | 1 | 57.0 | 0.08 | 32.0 | 78.5 | 78.0 | 0.154 | 0.652 | 0.194 | 1.040 |
| 13 | 8 | -1 | 1 | 52.5 | 0.20 | 0.6 | 73.6 | 81.5 | 0.143 | 0.628 | 0.229 | 1.085 |
| 8 | 9 | 1 | 1 | 57.0 | 0.32 | 32.0 | 81.5 | 81.3 | 0.150 | 0.657 | 0.192 | 1.042 |
| 18 | 10 | 0 | 1 | 52.5 | 0.20 | 20.3 | 73.6 | 81.5 | 0.150 | 0.654 | 0.196 | 1.046 |
| 12 | 11 | -1 | 1 | 52.5 | 0.40 | 20.3 | 76.6 | 83.4 | 0.148 | 0.659 | 0.194 | 1.046 |
| 2 | 12 | 1 | 1 | 57.0 | 0.08 | 8.6 | 77.9 | 78.1 | 0.148 | 0.628 | 0.223 | 1.075 |
| 17 | 13 | 0 | 1 | 52.5 | 0.20 | 20.3 | 73.6 | 81.5 | 0.150 | 0.654 | 0.196 | 1.046 |
| 9 | 14 | -1 | 1 | 45.0 | 0.20 | 20.3 | 59.4 | 84.2 | 0.146 | 0.663 | 0.191 | 1.045 |
| 4 | 15 | 1 | 1 | 57.0 | 0.32 | 8.6 | 81.3 | 81.3 | 0.146 | 0.642 | 0.211 | 1.065 |
| 16 | 16 | 0 | 1 | 52.5 | 0.20 | 20.3 | 73.6 | 81.5 | 0.150 | 0.654 | 0.196 | 1.046 |
| 19 | 17 | 0 | 1 | 52.5 | 0.20 | 20.3 | 73.6 | 81.5 | 0.150 | 0.654 | 0.196 | 1.046 |
| 7 | 18 | 1 | 1 | 48.0 | 0.32 | 32.0 | 68.7 | 84.1 | 0.148 | 0.665 | 0.187 | 1.040 |
| 5 | 19 | 1 | 1 | 48.0 | 0.08 | 32.0 | 49.9 | 83.7 | 0.147 | 0.667 | 0.186 | 1.039 |
| 11 | 20 | -1 | 1 | 52.5 | 0.00 | 20.3 | 68.0 | 77.8 | 0.152 | 0.648 | 0.199 | 1.047 |

StdRfolge = Standardreihenfolge; DIRfolge = Durchlaufreihenfolge; Temp. = Temperatur; Konz. = Konzentration; S(C8) = Selektivität zu Di-n-Buten

Die so erhaltenen Versuchsdaten wurden nach den Methoden der statistischen Versuchsplanung (engl. Design of Experiment) ausgewertet. Eine umfassende Einführung in die Methoden des Design of Experiment bietet z.B.

Z. R. Lazic, Design of Experiments in Chemical Engineering, Wiley-VCH, 2004.)

Hierfür wurde ebenfalls das Computerprogramm MiniTab verwendet, mit dessen Hilfe die eine sogenannte Wirkfläche über die unterschiedlichen Parameter berechnet wurde. Das Programm gibt Informationen gemäß Tabelle 10 und 11 aus:

Tabelle 10: Ergebnisse für den Isoindex

| **Term** | **Koeffizient** |
|---|---|
| Konstante | 113.814 |
| Temperatur | -0.360474 |
| Rücklauf | -4.36236 |
| Butenkonz. | -0.00618174 |
| Temperatur*Temperatur | 0.00518875 |
| Butenkonz*Butenkonz. | 0.00364209 |
| Temperatur*Butenkonz. | -0.00545420 |
| Rücklauf*Butenkonz. | 0.174800 |

Tabelle 11: Ergebnisse für den Umsatz

| **Term** | **Koeffizient** |
|---|---|
| Konstante | -181.465 |
| Temperatur | 7.52134 |
| Rücklauf | 237.263 |
| Butenkonz. | -1.70002 |
| Temperatur*Temperatur | -0.0510611 |
| Temperatur*Rücklauf | -4.39695 |
| Temperatur*Butenkonz. | 0.0272597 |
| Rücklauf*Butenkonz. | 1.08140 |

Mit Hilfe der Terme und Koeffizienten kann eine Beschreibung der Wirkfläche anhand der folgenden beiden Gleichungen (3) und (4) formuliert werden:

$$iso = 113{,}814 - 0{,}360474 \cdot T - 4{,}36236 \cdot R - 0{,}0618174 \cdot w(\text{1-B}) + 0{,}00518875 \cdot T^2 + 0{,}0364209 \cdot w^2(\text{1-B}) - 0{,}00545420 \cdot T \cdot w(\text{1-B}) + 0{,}174800 \cdot R^2 \quad (3)$$

$$conv = -181{,}465 + 7{,}52134 \cdot T + 237{,}263 \cdot R - 1{,}70002 \cdot w(\text{1-B}) - 0{,}0510611 \cdot T^2 - 4{,}39695 \cdot T \cdot R + 0{,}0272597 \cdot T \cdot w(\text{1-B}) + 1{,}08140 \cdot R \cdot w(\text{1-B}) \quad (4)$$

wobei iso und conv die in Figur 1 angegebenen Regelgrößen sind. T steht für die Temperatur (in Grad Celsius) des Wärmeträgers mit dem der Oligomerisierungsreaktor temperiert wird, R gibt die Rückführmenge an unumgesetzten Butenen (auf Basis der Experimenten angegeben in kg/h) an und w(1-B) den Massenanteil an 1-Buten in dem eingesetzten $C_4$-Kohlenwasserstoffstrom.

**Beispiel 6**

Dieses Beispiel soll eine Regelung der Oligomerisierung anhand der Größen iso und conv beschreiben. Die Versuche wurden in der gleichen Anlage durchgeführt, wie die in Beispiel 5. Da die zur Verfügung stehende Versuchsanlage nur mit manuellen Reglern ausgestattet war, wurde die Regelung hier dergestalt implementiert, dass zunächst die Gleichungssysteme für die Regelung an einem Steuerungsrechner programmiert wurden. In diesem Rechner wurden auch die Analysenergebnisse und sonstigen Messdaten teils automatisch, teils manuell (Eingabe durch einen Mitarbeiter) erfasst. Dem Rechner wurden bestimmte Maßgaben auferlegt, zum Beispiel, dass der Umsatz auf mindestens 80% gesteigert werden soll oder dass der Isoindex des Produktes zwischen 1,045 und 1,050 liegen soll. Die Steuerungssoftware hat basierend auf diesen Maßgaben eine Regelanweisung erzeugt (z.B. Temperatur auf mindestens 57 °C anheben), die dann von einem Mitarbeiter abgelesen und an den manuellen Reglern der Anlage programmiert wurde.

Die Ergebnisse zeigt Tabelle 12.

Tabelle 12: Ergebnisse für Beispiel 6

| Nr. | Systemänderung oder Regelungsschritt | Gehalt 1-Buten im Zulauf [Massen-%] | Temp. [°C] | Rücklauf unumgesetzte Butene [kg/h] | Umsatz Butene [%] | Isoindex aus Produkt berechnet | iso bzw. conv aus Formel 3 bzw. 4 |
|---|---|---|---|---|---|---|---|
| 1 | | 24,7 | 50 | 0,2 | 69,6 | 1,042 | 104,1<br>67,5 |
| | Steigerung des Umsatzes gewünscht→ Anheben der Temperatur→ Zielgröße conv > 80 | | soll >= 57 | | | | 104,5<br>80,4 |
| 2 | | 24,7 | 60 | 0,2 | 83,6 | 1,049 | 104,8<br>84,4 |
| | Senkung des Isoindex, bei Umsatz > 80% gewünscht →Absenken der Temperatur, Anheben des Rücklaufs → Zielgrößen 80<conv<84, iso<= 104,5 | | soll ~55 | soll >= 0,3 | | | 104,3<br>79,5 |
| 3 | | 24,7 | 55 | 0,4 | 80,1 | 1,045 | 104,3<br>81,4 |
| | Beimischung eines 1-Buten armen C4-Stromes → Absinken der 1-Buten-Konzentration | | | | | | |
| 4 | | 10,2 | 55 | 0,4 | 79,8 | 1,058 | 105,9<br>78,3 |
| | Senkung des Isoindex gewünscht →Absenken der Temperatur, Anpassen des Rücklaufs →Zielgröße iso <= 105,0 | | soll <= 47 | soll >= 0,4 | | | 105,0<br>71,6 |
| 5 | | 10,2 | 45 | 0,4 | 68,9 | 1,050 | 104,9<br>68,9 |

**Beispiel 7**

Das folgende Beispiel soll zeigen, das es besonders vorteilhaft ist, wenn die betrachteten Teilprozesse Oligomerisierung und Hydroformylierung nicht getrennt von einander betrachtet werden, sondern als eine Prozesseinheit die gemeinschaftlich mit Hilfe weiniger Größen wie iso und visc gesteuert werden kann.

Hierzu wurde die Oligomerisierung wie oben beschrieben durchgeführt und das Produkt Din-buten durch Destillation von höher und leichter siedenden Verunreinigungen befreit und für die Hydroformylierung bereitgestellt. Das bereitgestellte Dibuten wurde zur Bestimmung des Isoindexes wie oben beschrieben analysiert. Sodann wurde das Dibuten wie oben beschrieben hydroformyliert und die erhaltenen Produktaldehyde geschromatographisch untersucht um die Aldehydverteilung zu ermitteln aus der die Regelgröße visc berechnet wurde. Die Mess- und Analysendaten wurden auf einem Steuerungsrechner gesammelt und mit Hilfe der oben abgeleiteten Wechselwirkungsfunktionen ausgewertet. Auf der Basis bestimmter Rahmenparameter (Parametergrenzen der Oligomerisierung und Hydroformylierung, sowie willkürlich vorgegebene $C_4$-Einsatzstoffgemische und Ober- und Untergrenzen für die berechnete DINP-Viskosität) wurden von dem Rechner Steueranweisungen für die Hydroformylierung und Oligomerisierung erzeugt, die von einem Mitarbeiter an den Versuchsanlagen eingestellt wurden. Nach Einstellen der stationären Zustände wurden die Anlagen beprobt. Anhand der Ergebnisse wurde der Erfolg der Regelung kontrolliert und der nächste Steuerungsschritt ausgeführt. Die Ergebnisse zeigt Tabelle 13.

Tabelle 13: Versuchsergebnisse zu Beispiel 7

| Nr. | Systemänderung oder Vorgaben | Steuerungsanweise-ung Oligomeris-ierung | Gehalt 1-Buten im Zulauf [Massen-%] | Temp. [°C] | Rücklauf unumgesetzte Butene [kg/h] | Umsatz Butene [%] | Isoindex aus Produkt | iso bzw. conv aus Formel 3 bzw. 4 | Steuerungsanweise-ung Hydrofor-mylierung | Anteil Steuerlig-and L1 [mol-%] | visc berechnet aus Versuchsprodukt [mPas] | visc aus Steuerungsmodell [mPas] |
|---|---|---|---|---|---|---|---|---|---|---|---|---|
| | Rahmenbedingungen | | 0,6 - 40 | 45 - 60 | 0 - 0,4 | | | | | | 90-92 | |
| 1 | | | 10,2 | 45 | 0,4 | 68,9 | 1,050 | | | 10 | 89,2 | |
| | | visc >= 90<br>conv >= 80 | | Soll<br>>=59 | Soll<br><= 0,2 | | | 107,1<br>81,3 | | | | |
| 2 | | | 10,2 | 60 | 0,1 | 82,3 | 1,075 | | | 10 | 90,6 | |
| | 1-Buten 10,2→0,6% | | | | | | | | | | | |
| 3 | | | 0,6 | 60 | 0,1 | 83,5 | 1,136 | | | 10 | 94,0 | |
| | | visc <= 92<br>conv >= 80 | | Soll<br>60 | Soll<br>0,2-0,3 | | | 109,4 | | | | → 90,2 |
| 4 | | | 0,6 | 60 | 0,2 | 84,1 | 1,113 | | | 10 | 93,2 | |
| | | | | | | | | | visc <= 92 | Soll 25 | | Soll <= 92 |
| 5 | | | 0,6 | 60 | 0,2 | 84,1 | 1,113 | | | 25 | 92,3 | |
| | | visc <= 90<br>conv ~ 80 | | Soll<br>55 | Soll<br>0,4 | | | 107,8 | | | | → 88,4 |
| 6 | | | 0,6 | 55 | 0,4 | 79,9 | 1,077 | | | 25 | 90,4 | |
| | 1-Buten 0,6→ 10,2% | | | | | | | | | | | |
| 7 | | | 10,2 | 55 | 0,4 | 79,8 | 1,058 | | | 25 | 89,3 | |
| | | | | | | | | | visc >= 90 | Soll<br>0,1 | | <=92 |
| 8 | | | 10,2 | 55 | 0,4 | 79,8 | 1,058 | | | 0,1 | 91,2 | |

(fortgesetzt)

| Nr. | Systemänderung oder Vorgaben | Steuerungsanweise-ung Oligomeris-ierung | Gehalt 1-Buten im Zulauf [Massen-%] | Temp. [°C] | Rücklauf unumgesetzte Butene [kg/h] | Umsatz Butene [%] | Isoindex aus Produkt | iso bzw. conv aus Formel 3 bzw. 4 | Steuerungsanweise-ung Hydrofor-mylierung | Anteil Steuerlig-and L1 [mol-%] | visc berechnet aus Versuch-sprodukt [mPas] | visc aus Steuerungsmod ell [mPas] |
|---|---|---|---|---|---|---|---|---|---|---|---|---|
| | 1-Buten 10,2→ 24,7% | | | | | | | | | | | |
| 9 | | | 24,7 | 55 | 0,4 | 80,1 | 1,045 | | | 0,1 | 90,3 | |

**Bezugszeichenliste**

| | |
|---|---|
| C4 | Einsatzgemisch |
| [1] | Oligomerisierung |
| C8 | Dibuten |
| [2] | Hydroformylierung |
| CO | Kohlenmonoxid |
| H2 | Wasserstoff |
| INAL | Aldehydgemisch |
| [3] | Hydrierung |
| INA | Alkoholgemisch (INA) |
| [4] | Veresterung |
| PSA | Phthalsäureanhydrid |
| DINP | Estergemisch (Diisononylphthalat) |
| comp | Analyse der Zusammensetzung |
| R1 | Regler der Oligomerisierung |
| calc1 | Rechner zur Bestimmung des Isoindex |
| iso | Isoindex des Dibutens |
| T | Temperatur der Oligomerisierung |
| conv | Umsatz der Oligomerisierung |
| R2 | Regler der Hydroformylierung |
| calc2 | Rechner zur Bestimmung der Viskosität |
| visc | genäherte Viskosität des DINP |
| p | Druck der Hydroformylierung |
| cat | Katalysatorzusammensetzung der Hydroformylierung |

## Patentansprüche

**1.** Verfahren zur kontinuierlichen Herstellung eines Alkoholgemisches, bei welchem ein Olefine enthaltendes Einsatzgemisch, dessen Zusammensetzung sich über die Zeit verändert, unter Erhalt eines Oligomerisats einer Oligomerisierung unterworfen und zumindest ein Teil der im Oligomerisat enthaltenden Olefinoligomere in einer Hydroformylierung in Gegenwart eines homogenen Katalysatorsystems mit Kohlenmonoxid und Wasserstoff in Aldehyde oxiert werden, von denen zumindest ein Teil durch anschließende Hydrierung in das Alkoholgemisch umgesetzt werden,
**dadurch gekennzeichnet,**
**dass** die Zusammensetzung des Oligomerisats und die Zusammensetzung der Aldehyde bestimmt werden,
**dass** die Temperatur und/oder der Umsatz der Oligomerisierung in Abhängigkeit von der gegenwärtigen Zusammensetzung des Oligomerisats gesteuert werden,
und **dass** die Zusammensetzung des Katalysatorsystems und/oder der Druck der Hydroformylierung in Abhängigkeit von der gegenwärtigen Zusammensetzung der Aldehyde gesteuert werden,
wobei zumindest die Steuerung der Hydroformylierung in einer Weise erfolgt, dass eine aus der Zusammensetzung der Aldehyde errechnete, erste skalare Regelgröße konstant gehalten wird,
wobei es sich bei der ersten skalaren Regelgröße um eine Näherung der Viskosität eines Estergemisches handelt, welches durch Veresterung des Alkoholgemisches oder durch Umesterung mit Hilfe des Alkoholgemisches erhältlich ist,
wobei die Steuerung der Oligomerisierung in einer Weise erfolgt, dass eine aus der Zusammensetzung der Olefinoligomere errechnete, zweite skalare Regelgröße konstant gehalten wird,
und wobei es sich bei der zweiten skalaren Regelgröße um eine Näherung des Isoindex des Oligomerisats handelt.

**2.** Verfahren nach Anspruch 1,
**dadurch gekennzeichnet,**
**dass** die Zusammensetzung des Oligomerisats und die Zusammensetzung der Aldehyde kontinuierlich bestimmt werden, und dass die Oligomerisierung und/oder die Hydroformylierung kontinuierlich gesteuert werden.

**3.** Verfahren nach Anspruch 2,

**dadurch gekennzeichnet,**
**dass** auch die Steuerung der Oligomerisierung in einer Weise erfolgt, dass die aus der Zusammensetzung der Aldehyde errechnete, erste skalare Regelgröße konstant gehalten wird.

**4.** Verfahren nach einem der vorhergehenden Ansprüche,
**dadurch gekennzeichnet,**
**dass** das Einsatzgemisch Olefine mit vier Kohlenstoffatomen enthält, welche im Zuge der Oligomerisierung zu Olefinoligomeren mit acht, zwölf und sechzehn Kohlenstoffatomen oligomerisiert werden, und dass die Olefinoligomere mit acht Kohlenstoffatomen aus dem Oligomerisat abgetrennt und zu Aldehyden mit neun Kohlenstoffatomen hydroformyliert werden.

**5.** Verfahren nach Anspruch4,
**dadurch gekennzeichnet,**
**dass** sich das Einsatzgemisch zusammensetzt aus einer Kombination der folgenden Substanzmassenströme, die sich innerhalb der angegebenen Substanzmassenstrombereiche mit einer jeweiligen, im angegebenen Veränderungsgeschwindigkeitsbereich liegenden Veränderungsgeschwindigkeit verändern:

| Substanz | Substanzmassenstrom | Veränderungsgeschwindigkeit |
|---|---|---|
| Isobuten: | 0 kg/s bis 1 kg/s | - 0.05 $g/s^2$ bis 0.05 $g/s^2$ |
| 1-Buten: | 0 kg/s bis 6 kg/s | - 0.30 $g/s^2$ bis 0.30 $g/s^2$ |
| 2-Buten (cis + trans): | 1 kg/s bis 13 kg/s | - 0.30 $g/s^2$ bis 0.30 $g/s^2$ |
| Isobutan: | 0 kg/s bis 3 kg/s | - 0.15 $g/s^2$ bis 0.15 $g/s^2$ |
| n-Butan: | 1 kg/s bis 7 kg/s | - 0.30 $g/s^2$ bis 0.30 $g/s^2$ |
| Sonstige Stoffe: | 0 kg/s bis 1 kg/s | - 0.05 $g/s^2$ bis 0.05 $g/s^2$ |

**6.** Verfahren nach einem der vorhergehenden Ansprüche,
**dadurch gekennzeichnet,**
**dass** die Oligomerisierung in Gegenwart eines heterogenen Nickel-Katalysators erfolgt.

**7.** Verfahren nach einem der vorhergehenden Ansprüche, wobei die Oligomerisierung in Kreislauffahrweise betrieben wird, dergestalt, dass ein Anteil des aus der Oligomerisierung abgezogenen Oligomerisats in die Oligomerisierung zurückgeführt wird,
**dadurch gekennzeichnet,**
**dass** die Steuerung des Umsatzes der Oligomerisierung durch Veränderung des Anteils des zurückgeführten Oligomerisats erfolgt.

**8.** Verfahren nach einem der vorhergehenden Ansprüche,
**dadurch gekennzeichnet,**
**dass** die Hydroformylierung in Gegenwart eines homogenen Katalysatorsystems erfolgt, welches Rhodium sowie potentiell zumindest zwei unterschiedliche Monophosphit-Liganden umfasst, wobei die Steuerung der Zusammensetzung des Katalysatorsystems der Hydroformylierung durch Zudosierung eines der beiden Monophosphit-Liganden in die Hydroformylierung erfolgt.

**9.** Verfahren nach Anspruch 8,
**dadurch gekennzeichnet,**
**dass** die Zudosierung des einen Monophosphit-Liganden in einer Weise erfolgt, dass das molare Verhältnis der Summe aller Monophosphit-Liganden zu Rhodium unter Berücksichtigung von Ligandenverlusten konstant bleibt.

**10.** Verfahren nach Anspruch 8 oder 9,
**dadurch gekennzeichnet,**
**dass** das homogene Katalysatorsystem genau zwei unterschiedliche Monophosphit-Liganden umfasst, wobei es sich bei dem ersten Monophosphit-Liganden um eine Verbindung gemäß Strukturformel II handelt, und wobei es sich bei dem zweiten Monophosphit-Liganden um eine Verbindung gemäß Strukturformel Ia, Ib, oder III handelt:

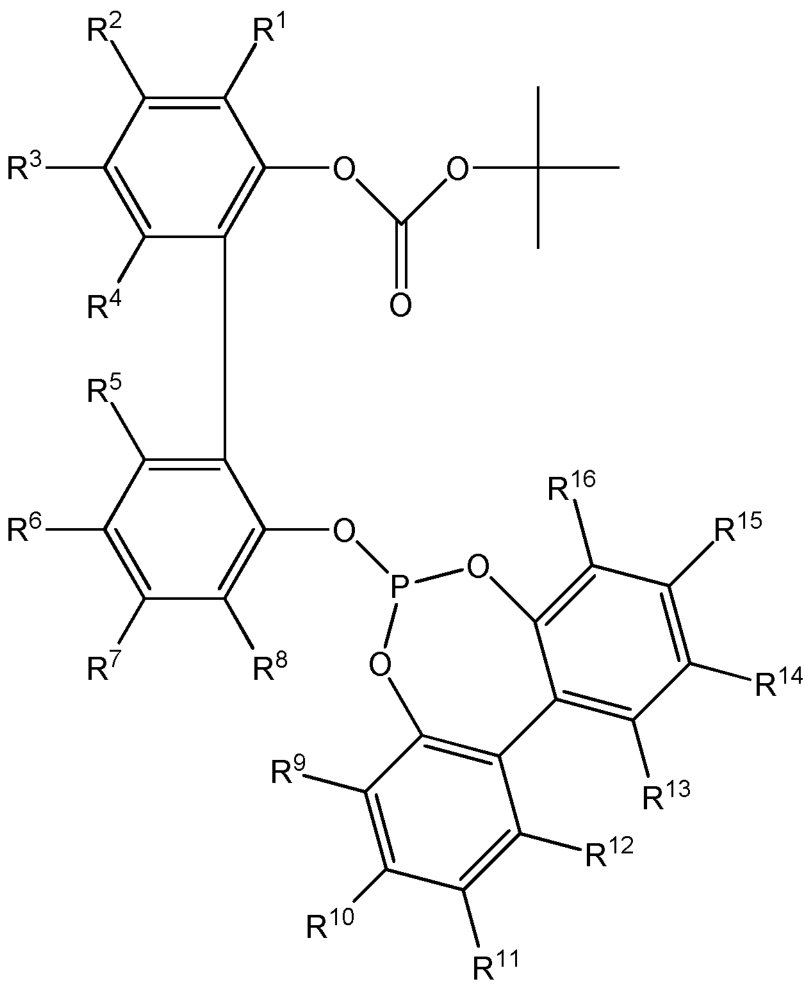

**Ia**

wobei in Ia

$R^1$, $R^2$, $R^3$, $R^4$, $R^5$, $R^6$, $R^7$, $R^8$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, Halogen, COO-($C_1$-$C_{12}$)-Alkyl, CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl,-CO-OH, -OH, -$SO_3H$ -$SO_3Na$ -$NO_2$, -CN, -$NH_2$, -N[($C_1$-$C_{12}$)-Alkyl]$_2$;

wobei $R^9$, $R^{10}$, $R^{11}$, $R^{12}$, $R^{13}$, $R^{14}$, $R^{15}$, $R^{16}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CONH-($C_1$-$C_{12}$)-Alkyl, -($C_6$-$C_{20}$)-Aryl-CON[($C_1$-$C_{12}$)-Alkyl]$_2$, -CO-($C_1$-$C_{12}$)-Alkyl, -CO-($C_6$-$C_{20}$)-Aryl, -CO-OH, - OH, -$SO_3H$; -$SO_3Na$ -$NO_2$, -CN, -$NH_2$, -N[($C_1$-$C_{12}$)-Alkyl]$_2$;

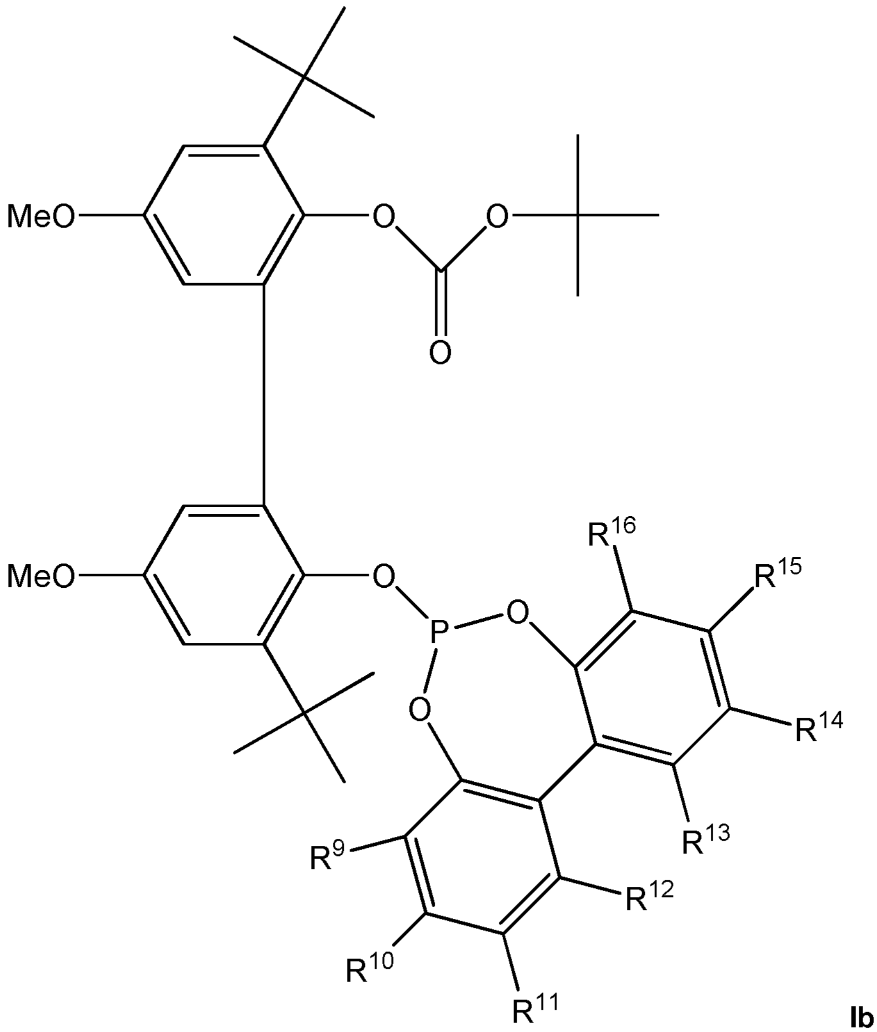

**Ib**

wobei in Ib
$R^9$, $R^{10}$, $R^{11}$, $R^{12}$, $R^{13}$, $R^{14}$, $R^{15}$, $R^{16}$ jeweils unabhängig voneinander ausgewählt sind aus:

-H, $-(C_1-C_{12})$-Alkyl, $-O-(C_1-C_{12})$-Alkyl, $-O-(C_6-C_{20})$-Aryl, $-(C_6-C_{20})$-Aryl, -Halogen, $-COO-(C_1-C_{12})$-Alkyl, $-CONH-(C_1-C_{12})$-Alkyl, $-(C_6-C_{20})$-Aryl-CON[$(C_1-C_{12})$-Alkyl]$_2$, $-CO-(C_1-C_{12})$-Alkyl, $-CO-(C_6-C_{20})$-Aryl, -COOH, -OH, $-SO_3H$ $-SO_3Na$ $-NO_2$, -CN, $-NH_2$, -N[$(C_1-C_{12})$-Alkyl]$_2$;

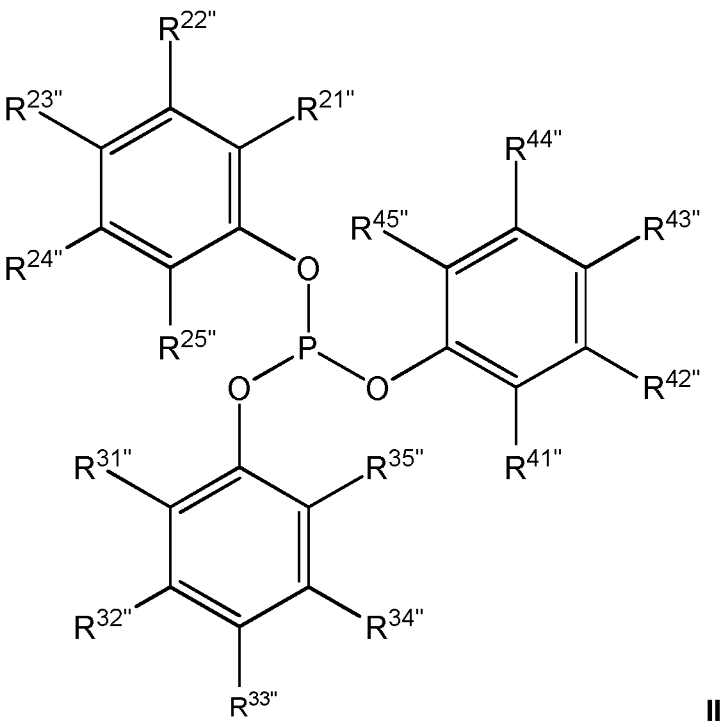

II

wobei in II

$R^{21''}$, $R^{22''}$, $R^{23''}$, $R^{24''}$, $R^{25''}$, $R^{31''}$, $R^{32''}$, $R^{33''}$, $R^{34''}$, $R^{35''}$, $R^{41''}$, $R^{42''}$, $R^{43''}$, $R^{44''}$, $R^{45''}$ jeweils unabhängig voneinander ausgewählt sind aus:

-H, $-(C_1-C_{12})$-Alkyl, $-O-(C_1-C_{12})$-Alkyl, $-O-(C_6-C_{20})$-Aryl, $-(C_6-C_{20})$-Aryl, -Halogen, $-COO-(C_1-C_{12})$-Alkyl, $-CONH-(C_1-C_{12})$-Alkyl, $-(C_6-C_{20})$-Aryl-CON$[(C_1-C_{12})$-Alkyl$]_2$, $-CO-(C_1-C_{12})$-Alkyl, $-CO-(C_6-C_{20})$-Aryl, -COOH, -OH, $-SO_3H$, $-SO_3Na$, $-NO_2$, -CN, $-NH_2$, $-N[(C_1-C_{12})$-Alkyl$]_2$;

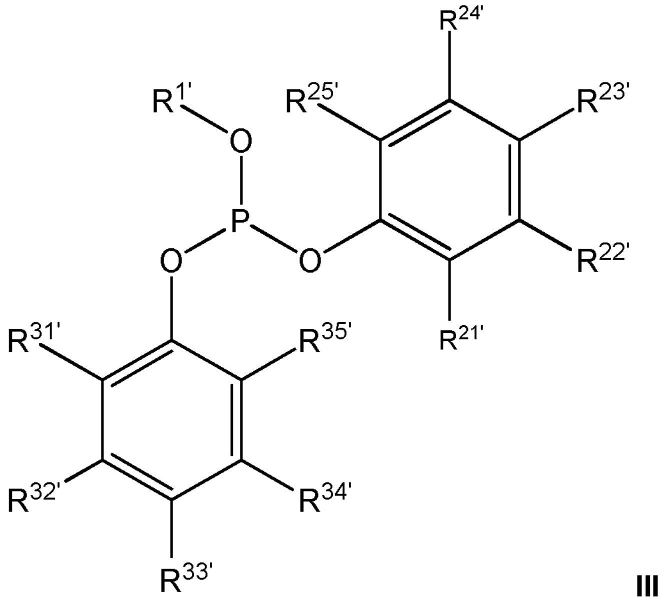

III

wobei in III

$R^{1'}$ ausgewählt ist aus:

$-(C_1-C_{12})$-Alkyl, $-(C_3-C_{12})$-Cycloalkyl, und

$R^{21'}$, $R^{22'}$, $R^{23'}$, $R^{24'}$, $R^{25'}$, $R^{31'}$, $R^{32'}$, $R^{33'}$, $R^{34'}$, $R^{35'}$ jeweils unabhängig voneinander ausgewählt sind aus:

-H, -($C_1$-$C_{12}$)-Alkyl, -O-($C_1$-$C_{12}$)-Alkyl, -O-($C_6$-$C_{20}$)-Aryl, -($C_6$-$C_{20}$)-Aryl, -Halogen, -COO-($C_1$-$C_{12}$)-Alkyl, -CO-($C_1$-$C_{12}$)-Alkyl, -CO-($C_6$-$C_{20}$)-Aryl, -COOH, -OH.

## Claims

1. Process for continuously preparing an alcohol mixture, in which an input mixture which comprises olefins and has a composition that changes over time is subjected to an oligomerization to obtain an oligomerizate and at least a portion of the olefin oligomers present in the oligomerizate are hydroformylated with carbon monoxide and hydrogen in a hydroformylation in the presence of a homogeneous catalyst system to give aldehydes, at least some of which are converted to the alcohol mixture by subsequent hydrogenation,
**characterized in that**
the composition of the oligomerizate and the composition of the aldehydes are determined,
the temperature and/or the conversion of the oligomerization are controlled as a function of the current composition of the oligomerizate,
and the composition of the catalyst system and/or the pressure in the hydroformylation are controlled as a function of the current composition of the aldehydes,
wherein at least the hydroformylation is controlled in such a way that a first scalar controlled variable calculated from the composition of the aldehydes is kept constant,
wherein the first scalar controlled variable is an approximation of the viscosity of an ester mixture obtainable by esterification of the alcohol mixture or by transesterification with the aid of the alcohol mixture,
wherein the oligomerization is controlled in such a way that a second scalar controlled variable calculated from the composition of the olefin oligomers is kept constant,
and wherein the second scalar controlled variable is an approximation of the iso index of the oligomerizate.

2. Process according to Claim 1,
**characterized in that**
the composition of the oligomerizate and the composition of the aldehydes are determined continuously, and the oligomerization and/or the hydroformylation are controlled continuously.

3. Process according to Claim 2,
**characterized in that**
the oligomerization is also controlled in such a way that the first scalar controlled variable calculated from the composition of the aldehydes is kept constant.

4. Process according to any of the preceding claims,
**characterized in that**
the input mixture comprises olefins having four carbon atoms which are oligomerized in the course of the oligomerization to give olefin oligomers having eight, twelve and sixteen carbon atoms, and the olefin oligomers having eight carbon atoms are removed from the oligomerizate and hydroformylated to aldehydes having nine carbon atoms.

5. Process according to Claim 4,
**characterized in that**
the input mixture is composed of a combination of the following substance mass flow rates which vary within the specified substance mass flow rate ranges with a respective rate of variation within the specified range of rates of variation:

| Substance | Substance mass flow rate | Rate of variation |
|---|---|---|
| Isobutene: | 0 kg/s to 1 kg/s | - 0.05 $g/s^2$ to 0.05 $g/s^2$ |
| 1-Butene: | 0 kg/s to 6 kg/s | - 0.30 $g/s^2$ to 0.30 $g/s^2$ |
| 2-Butene (cis + trans): | 1 kg/s to 13 kg/s | - 0.30 $g/s^2$ to 0.30 $g/s^2$ |
| Isobutane: | 0 kg/s to 3 kg/s | - 0.15 $g/s^2$ to 0.15 $g/s^2$ |
| n-Butane: | 1 kg/s to 7 kg/s | - 0.30 $g/s^2$ to 0.30 $g/s^2$ |
| Other materials: | 0 kg/s to 1 kg/s | - 0.05 $g/s^2$ to 0.05 $g/s^2$ |

6. Process according to any of the preceding claims, **characterized in that** the oligomerization is effected in the presence of a heterogeneous nickel catalyst.

7. Process according to any of the preceding claims, wherein the oligomerization is operated in circulation mode, in such a way that a proportion of the oligomerizate drawn off from the oligomerization is recycled into the oligomerization, **characterized in that** the conversion in the oligomerization is controlled by varying the proportion of the oligomerizate recycled.

8. Process according to any of the preceding claims, **characterized in that** the hydroformylation is effected in the presence of a homogeneous catalyst system comprising rhodium and potentially at least two different monophosphite ligands, the composition of the catalyst system in the hydroformylation being controlled by metered addition of one of the two monophosphite ligands into the hydroformylation.

9. Process according to Claim 8, **characterized in that** the one monophosphite ligand is metered in in such a way that the molar ratio of the sum total of all the monophosphite ligands to rhodium remains constant, taking account of ligand losses.

10. Process according to Claim 8 or 9, **characterized in that** the homogeneous catalyst system comprises exactly two different monophosphite ligands, the first monophosphite ligand being a compound of structural formula II, and the second monophosphite ligand being a compound of structural formula Ia, Ib or III:

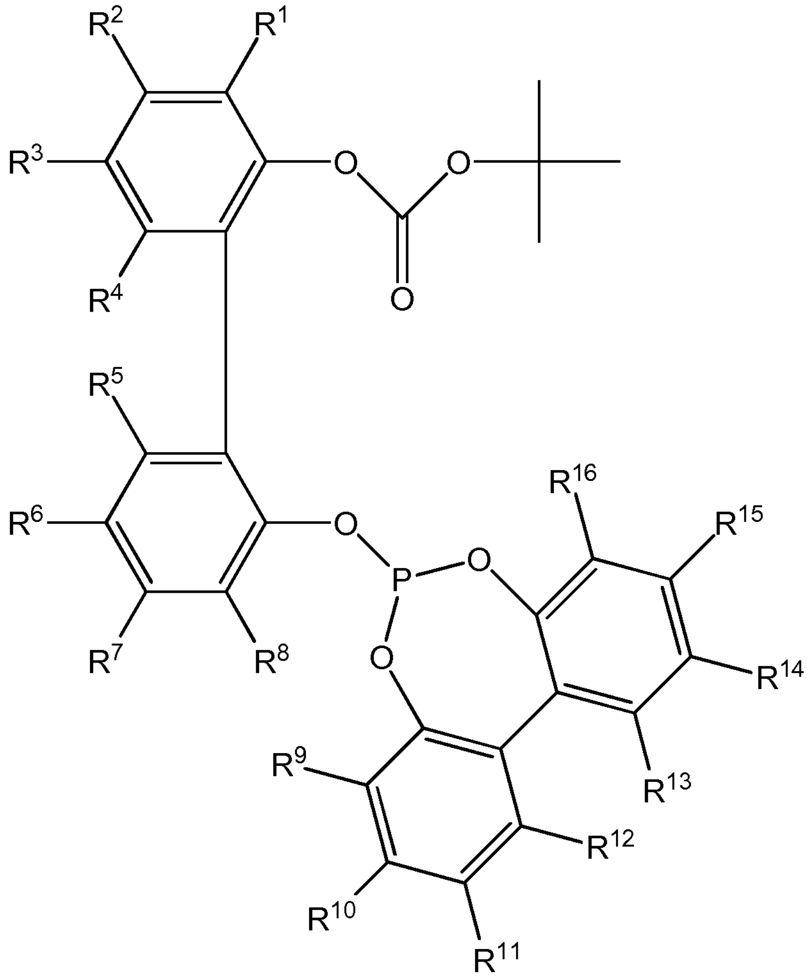

**Ia**

where, in Ia,

$R^1$, $R^2$, $R^3$, $R^4$, $R^5$, $R^6$, $R^7$, $R^8$ are each independently selected from:

-H, -($C_1$-$C_{12}$)-alkyl, -O-($C_1$-$C_{12}$) -alkyl, -O- ($C_6$-$C_{20}$) -aryl, -(C6-$C_{20}$)-aryl, halogen, COO-($C_1$-$C_{12}$)-alkyl, CONH-($C_1$-$C_{12}$)-alkyl,- ($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3H$ -$SO_3Na$ -$NO_2$, -CN, -$NH_2$, -N[($C_1$-$C_{12}$)-alkyl]$_2$;

where $R^9$, $R^{10}$, $R^{11}$, $R^{12}$, $R^{13}$, $R^{14}$, $R^{15}$, $R^{16}$ are each independently selected from:

-H, -($C_1$-$C_{12}$)-alkyl, -O-($C_1$-$C_{12}$)-alkyl, -O-($C_6$-$C_{20}$)-aryl, -(C6-$C_{20}$)-aryl, -halogen, -COO-($C_1$-$C_{12}$)-alkyl, -CONH-($C_1$-$C_{12}$)-alkyl,- ($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3H$ -$SO_3Na$ -$NO_2$, -CN, -$NH_2$, -N[($C_1$-$C_{12}$)-alkyl]$_2$;

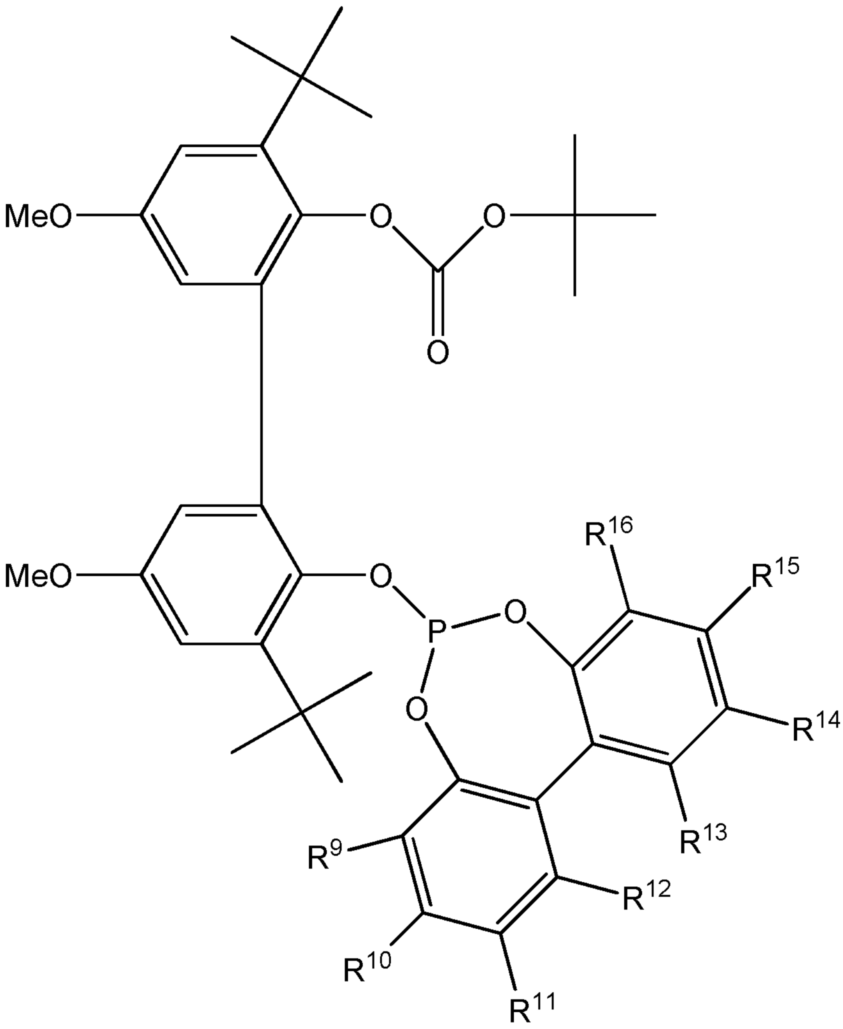

**Ib**

where, in Ib,
$R^9$, $R^{10}$, $R^{11}$, $R^{12}$, $R^{13}$, $R^{14}$, $R^{15}$, $R^{16}$ are each independently selected from:

- H, -($C_1$-$C_{12}$)-alkyl, -O-($C_1$-$C_{12}$)-alkyl, -O-($C_6$-$C_{20}$)-aryl, -(C6-$C_{20}$)-aryl, -halogen, -COO-($C_1$-$C_{12}$)-alkyl, -CONH-($C_1$-$C_{12}$)-alkyl,- ($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3H$ -$SO_3Na$ -$NO_2$, -CN, -$NH_2$, -N[($C_1$-$C_{12}$)-alkyl]$_2$;

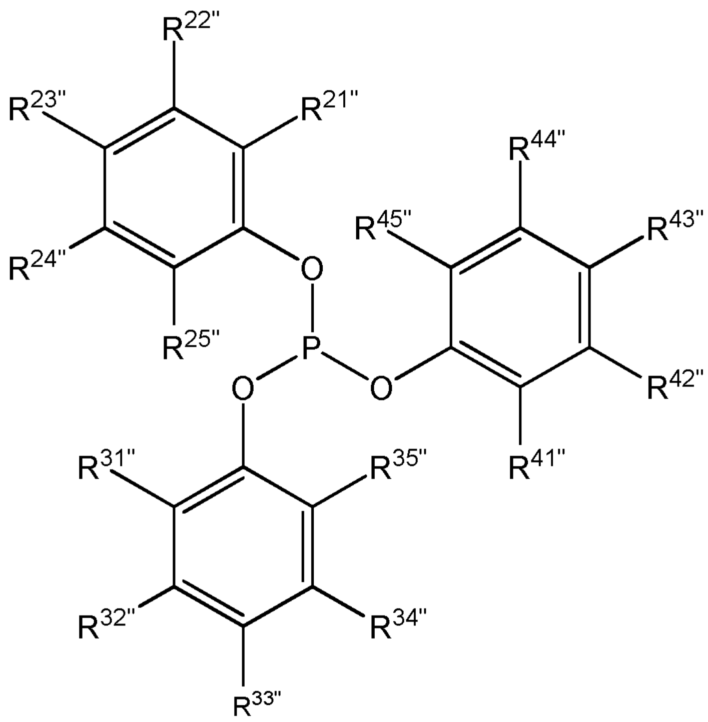

II

where, in II,

$R^{21"}$, $R^{22"}$, $R^{23"}$, $R^{24"}$, $R^{25"}$, $R^{31"}$, $R^{32"}$, $R^{33"}$, $R^{34"}$, $R^{35"}$, $R^{41"}$, $R^{42"}$, $R^{43"}$, $R^{44"}$, $R^{45"}$ are each independently selected from:

-H, -($C_1$-$C_{12}$)-alkyl, -O-($C_1$-$C_{12}$)-alkyl, -O- ($C_6$-$C_{20}$) -aryl, -(C6-$C_{20}$)-aryl, -halogen, -COO-($C_1$-$C_{12}$)-alkyl, -CONH-($C_1$-$C_{12}$)-alkyl,- ($C_6$-$C_{20}$)-aryl-CON[($C_1$-$C_{12}$)-alkyl]$_2$, -CO-($C_1$-$C_{12}$)-alkyl, -CO-($C_6$-$C_{20}$)-aryl, -COOH, -OH, -$SO_3$H -$SO_3$Na -$NO_2$, -CN, -$NH_2$, -N[($C_1$-$C_{12}$)-alkyl]$_2$;

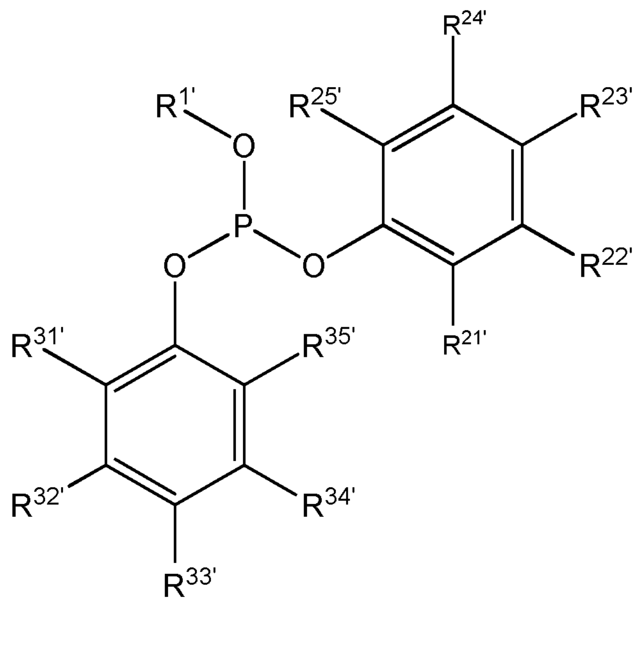

III

where, in III,

$R^{1'}$ is selected from:

-($C_1$-$C_{12}$)-alkyl, -($C_3$-$C_{12}$)-cycloalkyl, and

$R^{21'}$, $R^{22'}$, $R^{23'}$, $R^{24'}$, $R^{25'}$, $R^{31'}$, $R^{32'}$, $R^{33'}$, $R^{34'}$, $R^{35'}$ are each independently selected from:

-H, -($C_1$-$C_{12}$)-alkyl, -O-($C_1$-$C_{12}$)-alkyl, -O- ($C_6$-$C_{20}$) -aryl, -(C6-$C_{20}$)-aryl, -halogen, -COO-($C_1$-$C_{12}$)-alkyl, -CO-($C_1$-$C_{12}$)-alkyl, -CO-($C_6$-$C_{20}$)-aryl, -COOH, -OH.

## Revendications

1. Procédé de fabrication continue d'un mélange d'alcools, dans lequel un mélange d'alimentation contenant des oléfines, dont la composition change avec le temps, est soumis à une oligomérisation pour obtenir un oligomérisat, et au moins une partie des oligomères d'oléfine contenus dans l'oligomérisat est oxydée en aldéhydes dans une hydroformylation en présence d'un système catalytique homogène avec du monoxyde de carbone et de l'hydrogène ; au moins une partie des aldéhydes est convertie en le mélange d'alcools par une hydrogénation consécutive, **caractérisé en ce que**
la composition de l'oligomérisat et la composition des aldéhydes sont déterminées,
la température et/ou le taux de conversion de l'oligomérisation soient contrôlés en fonction de la composition instantanée de l'oligomérisat,
et la composition du système catalytique et/ou la pression de l'hydroformylation soient contrôlées en fonction de la composition instantanée des aldéhydes,
où au moins le contrôle de l'hydroformylation se fait de telle sorte qu'une première grandeur de réglage scalaire calculée à partir de la composition des aldéhydes soit maintenue constante,
où la première grandeur de réglage scalaire est une approximation de la viscosité d'un mélange d'esters qu'on obtient par estérification du mélange d'alcools ou par transestérification à l'aide du mélange d'alcools,
où le contrôle de l'oligomérisation se fait de telle sorte qu'une deuxième grandeur de réglage scalaire calculée à partir de la composition des oligomères d'oléfine soit maintenue constante,
et où la deuxième grandeur de réglage scalaire est une approximation de l'indice iso de l'oligomérisat.

2. Procédé selon la revendication 1, **caractérisé en ce que** la composition de l'oligomérisat et la composition des aldéhydes sont déterminées en continu, et **en ce que** l'oligomérisation et/ou l'hydroformylation sont contrôlées en continu.

3. Procédé selon la revendication 2, **caractérisé en ce que** le contrôle de l'oligomérisation se fait de telle sorte que la première grandeur de réglage scalaire calculée à partir de la composition des aldéhydes soit maintenue constante.

4. Procédé selon l'une des revendications précédentes,
**caractérisé en ce que** le mélange d'alimentation contient des oléfines avec quatre atomes de carbone, qui sont oligomérisés au cours de l'oligomérisation en oligomères d'oléfine avec huit, douze et seize atomes de carbone, et **en ce que** les oligomères d'oléfine avec huit atomes de carbone sont séparés de l'oligomérisat et sont hydroformylés en aldéhydes avec neuf atomes de carbone.

5. Procédé selon la revendication 4, **caractérisé en ce que** le mélange d'alimentation se compose d'une combinaison des flux massiques des substances suivantes, qui varient à l'intérieur des plages de flux massiques de substances indiquées avec une vitesse de changement respective située dans la plage de variation respective indiqués :

| Substance | Débit massique de substance | Vitesse de variation |
|---|---|---|
| isobutène | 0 à 1 kg/s | -0,05 à 0,05 $g/s^2$ |
| but-1-ène | 0 à 6 kg/s | -0,30 à 0,30 $g/s^2$ |
| but-2-ène (cis + trans) | 1 à 13 kg/s | -0,30 à 0,30 $g/s^2$ |
| isobutane | 0 à 3 kg/s | -0,15 à 0,15 $g/s^2$ |
| n-butane | 1 à 7 kg/s | -0,30 à 0,30 $g/s^2$ |
| autre | 0 à 1 kg/s | -0,05 à 0,05 $g/s^2$ |

6. Procédé selon l'une des revendications précédentes,
**caractérisé en ce que** l'oligomérisation se fait en présence d'un catalyseur hétérogène au nickel.

7. Procédé selon l'une des revendications précédentes, où l'oligomérisation est effectuée en circuit fermé, de sorte qu'une partie de l'oligomérisat extrait de l'oligomérisation est réintroduite dans l'oligomérisation,
**caractérisé en ce que** le contrôle du taux de conversion de l'oligomérisation se fait en modifiant la part de l'oligomérisat réintroduite.

8. Procédé selon l'une des revendications précédentes,
**caractérisé en ce que** l'hydroformylation se fait en présence d'un système catalytique homogène qui contient du rhodium ainsi que, potentiellement, au moins deux ligands monophosphites distincts, le contrôle de la composition du système catalytique d'hydroformylation se faisant par addition d'un des deux ligands monophosphites dans l'hydroformylation.

9. Procédé selon la revendication 8, **caractérisé en ce que** l'ajout du ligand monophosphite se fait de telle sorte que le rapport molaire de la somme de tous les ligands monophosphites au rhodium reste constant en prenant en compte les pertes de ligands.

10. Procédé selon la revendication 8 ou 9, **caractérisé en ce que** le système catalytique homogène comprend exactement deux différents ligands monophosphites, où le premier ligand monophosphite est un composé selon la formule structurelle II, et où le deuxième ligand monophosphite est un composé selon la formule structurelle Ia, Ib ou III :

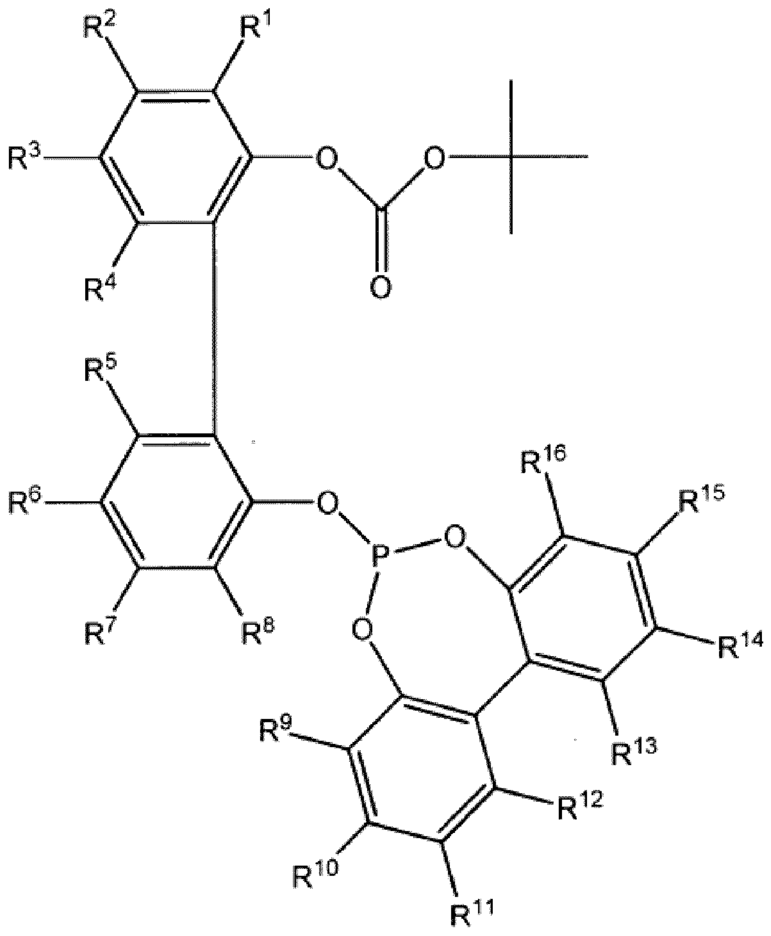

**Ia**

où, dans Ia :

$R^1$, $R^2$, $R^3$, $R^4$, $R^5$, $R^6$, $R^7$, $R^8$ sont choisis respectivement, indépendamment les uns des autres, parmi :

-H, -$C_{1-12}$-alkyle, -O-$C_{1-12}$-alkyle, -O-$C_{6-20}$-aryle, -$C_{6-20}$-aryle, halogène, -COO-$C_{1-12}$-alkyle, -CONH-

$C_{1-12}$-alkyle, $C_{6-20}$-aryl-CON ($C_{1-12}$-alkyl)$_2$, -CO-$C_{1-12}$-alkyle, -CO-$C_{6-20}$-aryle, -COOH, -OH, -$SO_3H$ -$SO_3Na$ -$NO_2$, -CN, -$NH_2$, -N($C_{1-12}$-alkyl)$_2$ ;

où $R^9$, $R^{10}$, $R^{11}$, $R^{12}$, $R^{13}$, $R^{14}$, $R^{15}$, $R^{16}$ sont choisis respectivement, indépendamment les uns des autres, parmi :

-H, -$C_{1-12}$-alkyle, -O-$C_{1-12}$-alkyle, -O-$C_{6-20}$-aryle, -$C_{6-20}$-aryle, halogène, -COO-$C_{1-12}$-alkyle, -CONH-$C_{1-12}$-alkyle, $C_{6-20}$-aryl-CON ($C_{1-12}$-alkyl)$_2$, -CO-$C_{1-12}$-alkyle, -CO-$C_{6-20}$-aryle, -COOH, -OH, -$SO_3H$ ; -$SO_3Na$, -$NO_2$, - CN, -$NH_2$, -N($C_{1-12}$-alkyl)$_2$ ;

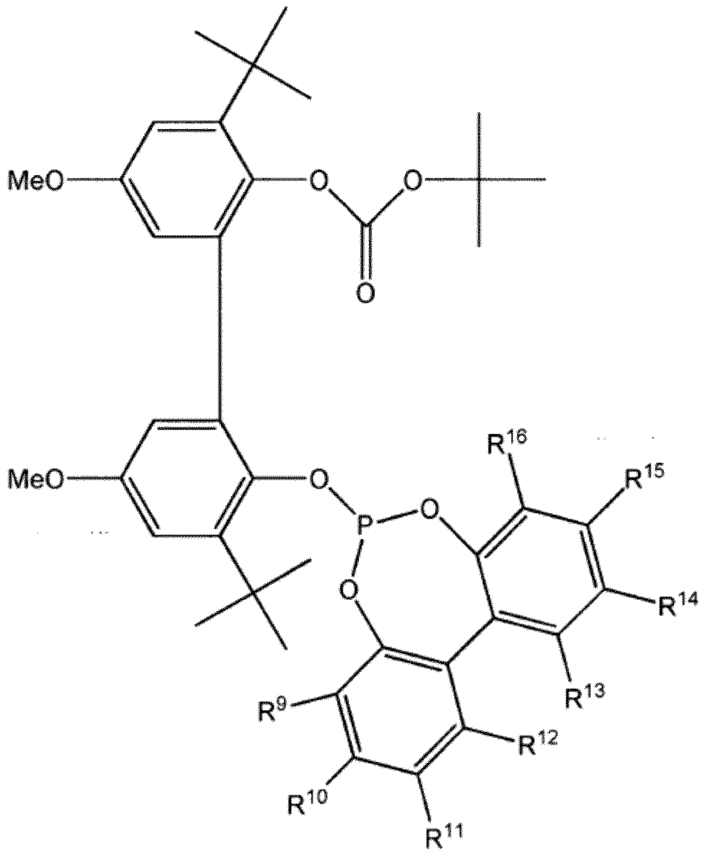

Ib

où, dans Ib :

$R^9$, $R^{10}$, $R^{11}$, $R^{12}$, $R^{13}$, $R^{14}$, $R^{15}$, $R^{16}$ sont choisis respectivement, indépendamment les uns des autres, parmi :

-H, -$C_{1-12}$-alkyle, -O-$C_{1-12}$-alkyle, -O-$C_{6-20}$-aryle, -$C_{6-20}$-aryle, halogène, -COO-$C_{1-12}$-alkyle, -CONH-$C_{1-12}$-alkyle, $C_{6-20}$-aryl-CON ($C_{1-12}$-alkyl)$_2$, -CO-$C_{1-12}$-alkyle, -CO-$C_{6-20}$-aryle, -COOH, -OH, -$SO_3H$ -$SO_3Na$ -$NO_2$, -CN, -$NH_2$, -N($C_{1-12}$-alkyl)$_2$ ;

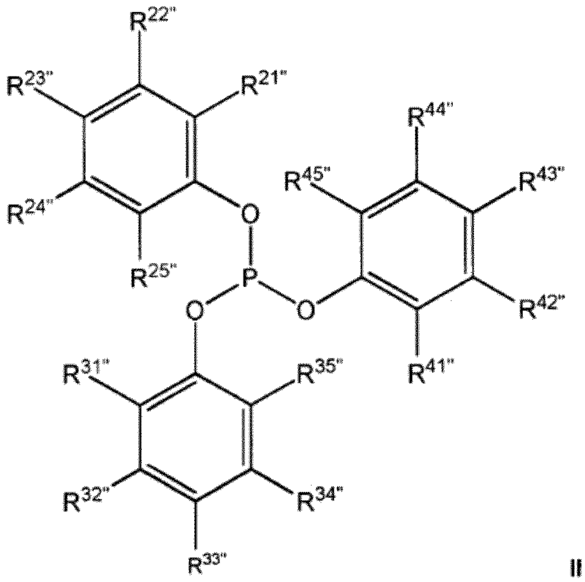

II

où dans II :

$R^{21''}$, $R^{22''}$, $R^{23''}$, $R^{24''}$, $R^{25''}$, $R^{31''}$, $R^{32''}$, $R^{33''}$, $R^{34''}$, $R^{35''}$, $R^{41''}$, $R^{42''}$, $R^{43''}$, $R^{44''}$, $R^{45''}$ sont choisis respectivement, indépendamment les uns des autres, parmi : -H, $-C_{1-12}$-alkyle, $-O-C_{1-12}$-alkyle, $-O-C_{6-20}$-aryle, $-C_{6-20}$-aryle, halogène, $-COO-C_{1-12}$-alkyle, $-CONH-C_{1-12}$-alkyle, $C_{6-20}$-aryl-CON $(C_{1-12}$-alkyl$)_2$, $-CO-C_{1-12}$-alkyle, $-CO-C_{6-20}$-aryle, -COOH, -OH, $-SO_3H$ $-SO_3Na$ $-NO_2$, -CN, $-NH_2$, $-N(C_{1-12}$-alkyl$)_2$ ;

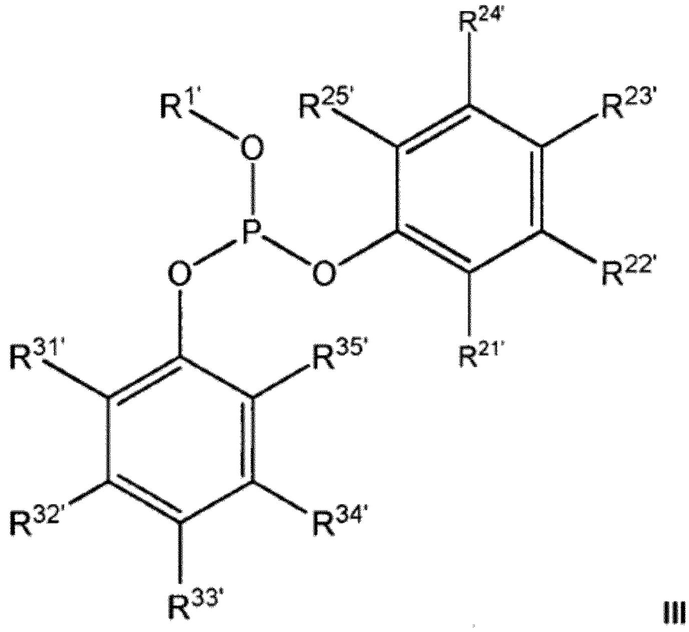

III

où dans III,

$R^1$ est choisi parmi $-C_{1-12}$-alkyle, $-C_{3-12}$-cycloalkyle, et
$R^{21'}$, $R^{22'}$, $R^{23'}$, $R^{24'}$, $R^{25'}$, $R^{31'}$, $R^{32'}$, $R^{33'}$, $R^{34'}$, $R^{35'}$ sont choisis respectivement, indépendamment les uns des autres, parmi :

-H, $-C_{1-12}$-alkyle, $-O-C_{1-12}$-alkyle, $-O-C_{6-20}$-aryle, $-C_{6-20}$-aryle, halogène, $-COO-C_{1-12}$-alkyle, $-CO-C_{1-12}$-alkyle, $-CO-C_{6-20}$-aryle, -COOH, -OH.

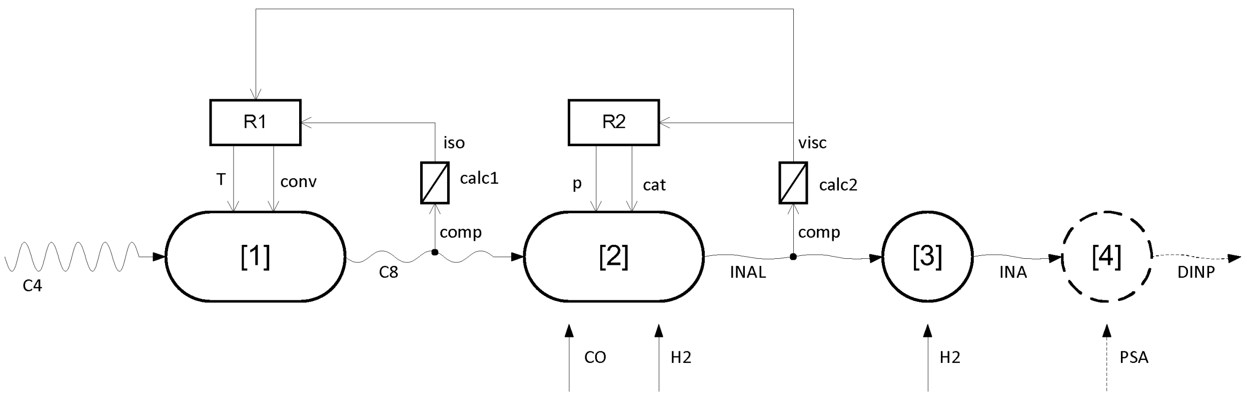
R1
R2
iso
visc
T
conv
calc1
p
cat
calc2
comp
comp
[1]
[2]
[3]
[4]
C4
C8
INAL
INA
DINP
CO
H2
H2
PSA

Fig. 1

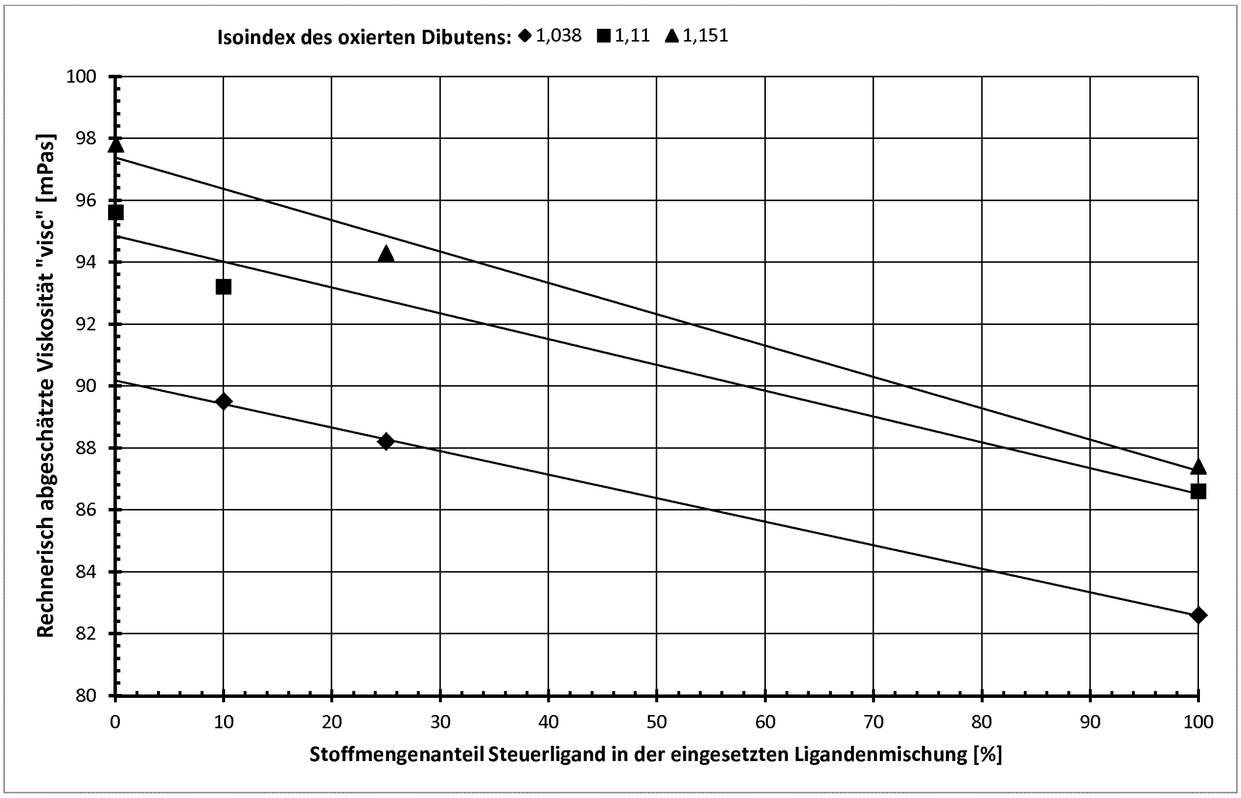
Isoindex des oxierten Dibutens: ◆ 1,038 ■ 1,11 ▲ 1,151
100
98
96
94
92
90
88
86
84
82
80
Rechnerisch abgeschätzte Viskosität "visc" [mPas]
0
10
20
30
40
50
60
70
80
90
100
Stoffmengenanteil Steuerligand in der eingesetzten Ligandenmischung [%]

Fig. 2

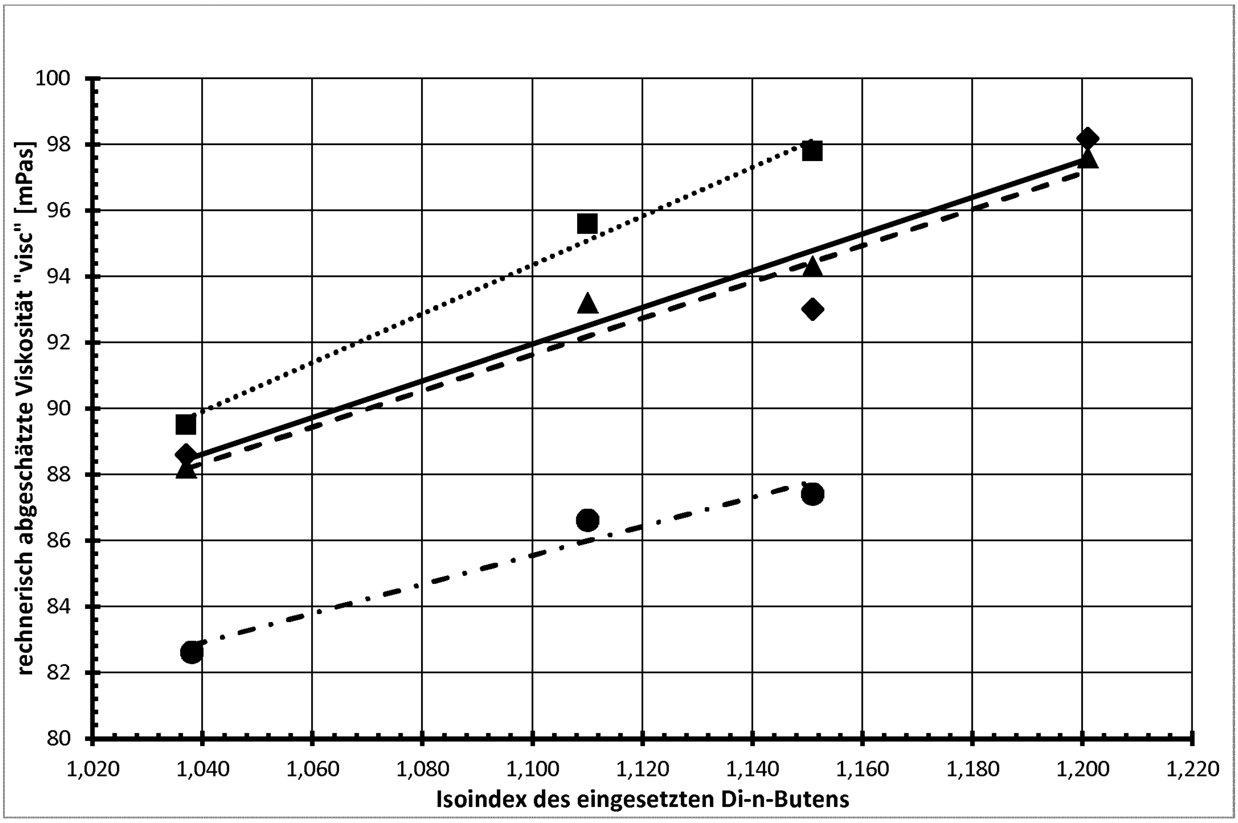


● 100 % L1 ■ 100 % L2 ▲10 % L1 und 90 % L2 ♦ 25 % L1 und 75% L2

Fig. 3

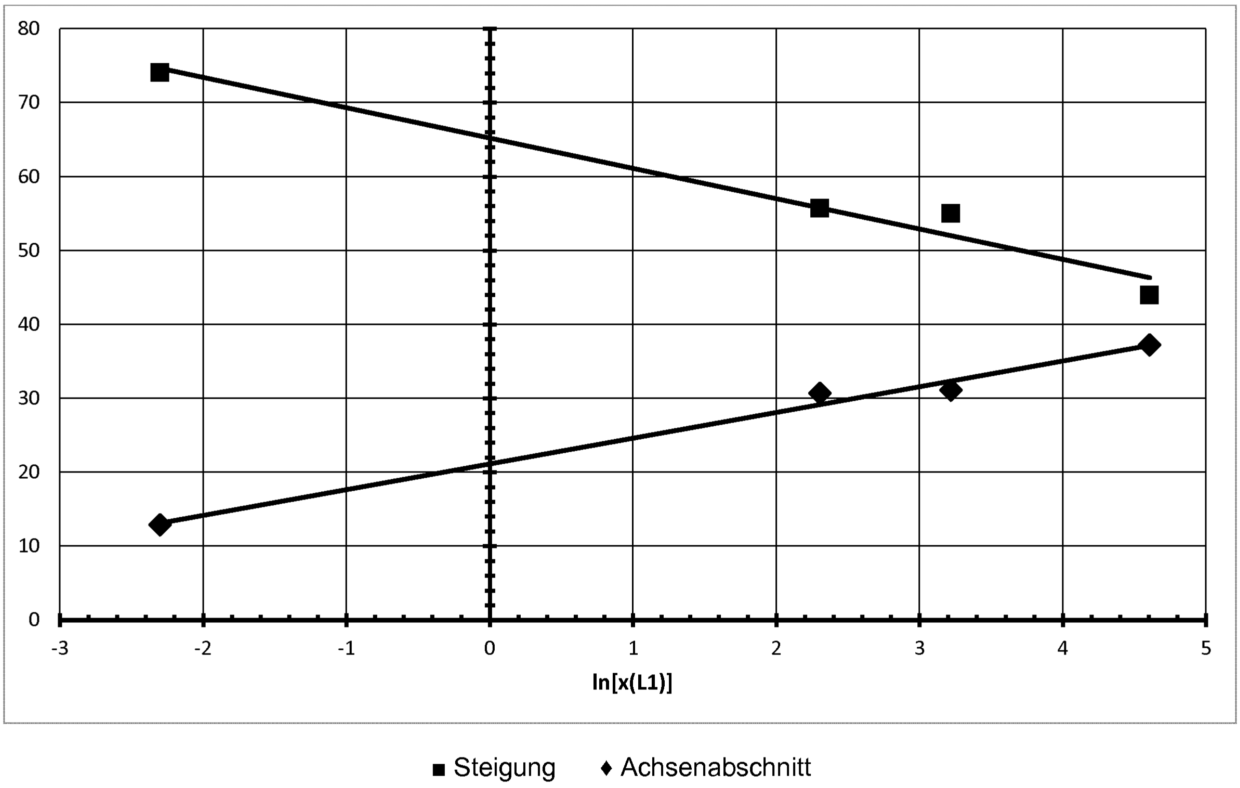
80
70
60
50
40
30
20
10
0
-3
-2
-1
0
1
2
3
4
5
ln[x(L1)]
■ Steigung
♦ Achsenabschnitt

Fig. 4

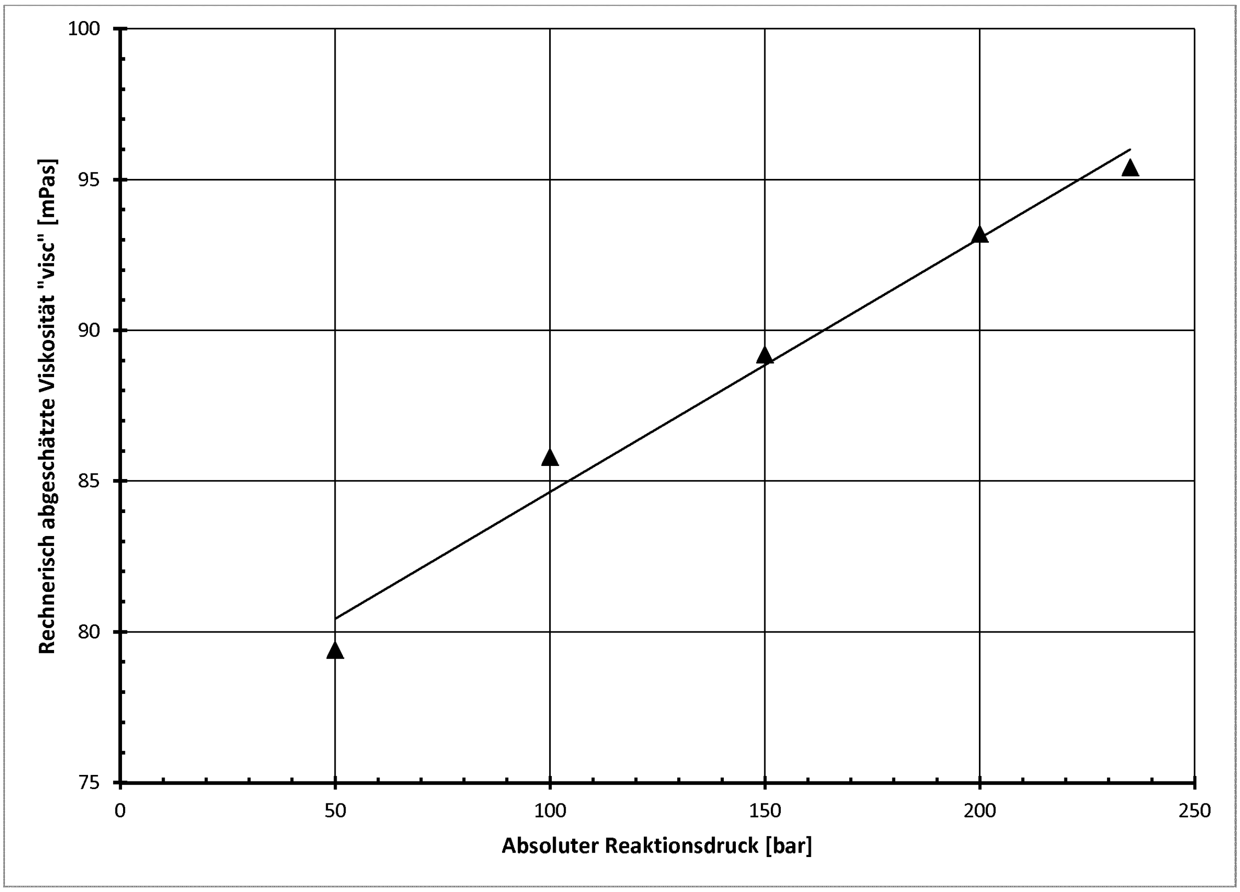
100
95
90
85
80
75
Rechnerisch abgeschätzte Viskosität "visc" [mPas]
0
50
100
150
200
250
Absoluter Reaktionsdruck [bar]

Fig. 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102008007080 A1
- EP 2220017 B1
- EP 1674441 B1
- DE 102008007081 A1
- EP 1029839 A1
- WO 9925668 A
- DE 10015002 A1
- WO 9925668 A1
- DE 102013212481
- US 20120253080 A
- EP 1099678 B1
- EP 1430014 B1
- WO 2011000697 A1


### In der Beschreibung aufgeführte Nicht-Patentliteratur


- **B. CORNILS ; W. A. HERRMANN.** Applied Homogeneous Catalysis with Organometallic Compounds. VCH, 1996, vol. 1 & 2
- **R. FRANKE ; D. SELENT ; A. BÖRNER.** Applied Hydroformylation. *Chem. Rev.,* 2012, vol. 112, 5675-5732
- **B. L. HAYMORE ; A. VAN HASSELT ; R. BECK.** *Annals of the New York Acad. Sci.,* 1983, vol. 415, 159-175
- **B. SCHOLZ.** The HÜLS OCTOL Process: Heterogeneously catalyzed dimerization of n-butenes and other olefins. *DGMK-Tagung,* April 1989, 21, , 22
- **R.H. FRIEDLANDER ; D.J. WARD ; F. OBENAUS ; F. NIERLICH ; J. NEUMEISTER.** Make plasticizer olefins via n-butene dimerization. *Hydrocarbon Processing,* Februar 1986, 31-33
- **F. NIERLICH.** Oligomerize for better gasoline. *Hydrocarbon Processing,* Februar 1992, 45-46
- **P. W. N. M. VAN LEEUWEN.** Rhodium Catalyzed Hydroformylation. Kluwer, 2000
- **H. TRICAS ; O. DIEBOLT ; P. W .N .M .VAN LEEUWEN.** *Journal of Catalysis,* 2013, vol. 298, 198-205
- Alcohols Aliphatic. **J. FALBE ; H. BAHRMANN ; W. LIPPS ; D. MAYER ; G. D. FREY.** Ullmann's Encyclopedia of Industrial Chemistry. 2013
- Hydrogenation and Dehydrogenation. **D. SANFILIPPO ; P. N. RYLANDER.** Ullmann's Encyclopedia of Industrial Chemistry. 2009
- **ALAN S. WILSON.** *Plasticizers, The Institute of Materials,* 1995, ISBN 0 901716 76 6, 135-136
- **BRIAN L. WADEY ; LUCIEN THIL ; MO A. KHUDDUS ; HANS REICH.** The Nonyl Phthalate Ester and It's Use in flexible PVC. *Journal of Vinyl Technology,* 1990, vol. 12, 208-211
- **W. L. F. ARMAREGO ; CHRISTINA CHAI.** Purification of Laboratory Chemicals. Butterworth Heinemann (Elsevier), 2009
- **ROBIN K. HARRIS ; EDWIN D. BECKER ; SONIA M. CABRAL DE MENEZES ; ROBIN GOODFELLOW ; PIERRE GRANGER.** *Pure Appl. Chem.,* 2001, vol. 73, 1795-1818
- **ROBIN K. HARRIS ; EDWIN D. BECKER ; SONIA M. CABRAL DE MENEZES ; PIERRE GRANGER ; ROY E. HOFFMAN ; KURT W. ZILM.** *Pure Appl. Chem.,* 2008, vol. 80, 59-84
- **Z. R. LAZIC.** Design of Experiments in Chemical Engineering. Wiley-VCH, 2004